# EUROPEAN PATENT SPECIFICATION

(11) **EP 2 350 131 B1**
(45) Date of publication and mention of the grant of the patent: **07.06.2017**
(21) Application number: 09825562.3
(22) Date of filing: 09.11.2009
(51) Int. Cl.: C12N 15/113, A61P 35/00, C07K 16/28, C07K 16/18, A61K 39/395

(54) **COMPOSITIONS AND METHODS FOR THE INHIBITION OF CRIPTO/GRP78 COMPLEX FORMATION AND SIGNALING**
ZUSAMMENSETZUNGEN UND METHODEN ZUR INHIBIERUNG DER CRIPTO/GRP78-KOMPLEXBILDUNG UND SIGNALVERMITTLUNG
COMPOSITIONS ET MÉTHODES POUR L'INHIBITION DE LA FORMATION ET DE LA SIGNALISATION DU COMPLEXE CRIPTO/GRP78

(30) Priority: 07.11.2008 US 112579 P
(43) Date of publication of application: 03.08.2011
(73) Proprietor: Research Development Foundation, Carson City, NV 89703 (US)
(72) Inventor: GRAY, Peter, C., San Diego, CA 92120 (US); SHANI, Gidi, San Diego, CA 92122 (US); KELBER, Jonathan, A., San Diego, CA 92122 (US); VALE, Wylie, La Jolla, CA 92037 (US)
(74) Representative: Vossius & Partner Patentanwälte Rechtsanwälte mbB
(86) International application number: PCT/US2009/063748
(87) International publication number: WO 2010/054328

(56) References cited:
- WO-A1-2008/105560
- ADKINS H B ET AL: "ANTIBODY BLOCKADE OF THE CRIPTO CFC DOMAIN SUPPRESSES TUMOR CELL GROWTH IN VIVO", JOURNAL OF CLINICAL INVESTIGATION, vol. 112, no. 4, 1 August 2003 (2003-08-01), pages 575-587, XP009059619, AMERICAN SOCIETY FOR CLINICAL INVESTIGATION, US ISSN: 0021-9738, DOI: 10.1172/JCI200317788
- S TSUTSUMI ET AL: "Celecoxib upregulates endoplasmic reticulum chaperones that inhibit celecoxib-induced apoptosis in human gastric cells", ONCOGENE, vol. 25, no. 7, 16 February 2006 (2006-02-16), pages 1018-1029, XP055045777, ISSN: 0950-9232, DOI: 10.1038/sj.onc.1209139
- A. C. RANGANATHAN: "Functional Coupling of p38-Induced Up-regulation of BiP and Activation of RNA-Dependent Protein Kinase-Like Endoplasmic Reticulum Kinase to Drug Resistance of Dormant Carcinoma Cells", CANCER RESEARCH, vol. 66, no. 3, 1 February 2006 (2006-02-01), pages 1702-1711, XP055045583, ISSN: 0008-5472, DOI: 10.1158/0008-5472.CAN-05-3092
- K ZU ET AL: "Enhanced selenium effect on growth arrest by BiP/GRP78 knockdown in p53-null human prostate cancer cells", ONCOGENE, 1 January 2005 (2005-01-01), XP055045598, ISSN: 0950-9232, DOI: 10.1038/sj.onc.1209071
- DATABASE WPI Week 200566 Thomson Scientific, London, GB; AN 2005-640288 XP002688119, -& CN 1 616 476 A (HANGZHOU NEW RAYJAY BIOMED CORP) 18 May 2005 (2005-05-18)
- RAUSCHERT N ET AL: "A new tumor specific variant of GRP78 as target for antibody-based therapy", LABORATORY INVESTIGATION, vol. 88, 1 April 2008 (2008-04-01), pages 375-386, XP002520043, NATURE PUBLISHING GROUP, THE UNITED STATES AND CANADIAN ACADEMY OF PALHOLOGY, INC ISSN: 0023-6837, DOI: 10.1038/LABINVEST.2008.2
- SHANI, G. ET AL.: 'GRP78 and Cripto form a complex at the cell surface and co llaborate to inhibit transforming growth factor beta signaling and enhance cel I growth.' MOLECULAR AND CELLULAR BIOLOGY. vol. 28, no. 2, 08 November 2007, pages 666 - 677, XP008147926
- DAVIDSON, D. J. ET AL.: 'Kringle 5 of human plasminogen induces apoptosis of endothelial and tumor cells through surface-expressed glucose-regulated prot ein 78.' CANCER RESEARCH. vol. 65, no. 11, 01 June 2005, pages 4663 - 4672, XP002382037
- PHILIPPOVA, M. ET AL.: 'Identification of proteins associating with glycosylp hosphatidylinositol-anchored T-cadherin on the surface of vascular endotheli al cells: role for Grp78/BiP in T-cadherin-dependent cell survival.' MOLECULAR AND CELLULAR BIOLOGY. vol. 28, no. 12, June 2008, pages 4004 - 4017, XP008147978
- KELBER, J. A. ET AL.: 'Blockade of Cripto binding to cell surface GRP78 inhib its oncogenic Cripto signaling via MAPK/PI3K and Smad2/3 pathways.' ONCOGENE vol. 28, 04 May 2009, pages 2324 - 2336, XP055045850
- R. K. Reddy ET AL: "Endoplasmic Reticulum Chaperone Protein GRP78 Protects Cells from Apoptosis Induced by Topoisomerase Inhibitors: ROLE OF ATP BINDING SITE IN SUPPRESSION OF CASPASE-7 ACTIVATION", Journal of Biological Chemistry, vol. 278, no. 23, 28 March 2003 (2003-03-28), pages 20915-20924, XP055140920, ISSN: 0021-9258, DOI: 10.1074/jbc.M212328200
- Y. Zhang ET AL: "Cell Surface Relocalization of the Endoplasmic Reticulum Chaperone and Unfolded Protein Response Regulator GRP78/BiP", Journal of Biological Chemistry, vol. 285, no. 20, 5 March 2010 (2010-03-05), pages 15065-15075, XP055140936, ISSN: 0021-9258, DOI: 10.1074/jbc.M109.087445
- MARIO GONZALEZ-GRONOW ET AL: "Prostate cancer cell proliferation in vitro is modulated by antibodies against glucose-regluated protein 78 isolated from patient serum", CANCER RESEARCH, AMERICAN ASSOCIATION FOR CANCER RESEARCH, US, vol. 66, no. 23, 1 December 2006 (2006-12-01), pages 11424-11431, XP008145010, ISSN: 0008-5472, DOI: 10.1158/0008-5472.CAN-06-1721
- ARAP M A ET AL: "Cell surface expression of the stress response chaperone GRP78 enables tumor targeting by circulating ligands", CANCER CELL, CELL PRESS, US, vol. 6, no. 3, 1 September 2004 (2004-09-01), pages 275-284, XP002366633, ISSN: 1535-6108, DOI: 10.1016/J.CCR.2004.08.018
- JAKOBSEN CHARLOTTE G ET AL: "Phage display derived human monoclonal antibodies isolated by binding to the surface of live primary breast cancer cells recognize GRP78", CANCER RESEARCH, AMERICAN ASSOCIATION FOR CANCER RESEARCH, US, vol. 67, no. 19, 1 October 2007 (2007-10-01), pages 9507-9517, XP002576192, ISSN: 0008-5472, DOI: 10.1158/0008-5472.CAN-06-4686
- A Aplin ET AL: "Cell adhesion molecules, signal transduction and cell growth", Current Opinion in Cell Biology, vol. 11, no. 6, 1 December 1999 (1999-12-01), pages 737-744, XP055140939, ISSN: 0955-0674, DOI: 10.1016/S0955-0674(99)00045-9

### SEQUENCE LISTING

<110> GRAY, PETER C. SHANI, GIDI KELBER, JONATHAN A. VALE, WYLIE
<120> COMPOSITIONS AND METHODS FOR THE INHIBITION OF CRIPTO / GRP78 COMPLEX FORMATION AND SIGNALING
<130> CLFR:334WO
<140> UNKNOWN
   <141> 2009-11-09
<150> 61/112,579
   <151> 2008-11-07
<160> 5
<170> PatentIn version 3.3
<210> 1
   <211> 17
   <212> PRT
   <213> Artificial
<220>
   <223> Synthetic peptide
<400> 1
   Cys Pro Pro Ser Phe Tyr Gly Arg Asn Cys Glu His Asp Val Arg Lys
   1               5                   10                  15
   Glu
<210> 2
   <211> 84
   <212> DNA
   <213> Artificial
<220>
   <223> Synthetic primer
<400> 2
   ctgtctagac aaaaaaccat acattcaagt tgattctctt gaaatcaact tgaatgtatg     60
   gtcggggatc tgtggtctca taca                                            84
<210> 3
   <211> 25
   <212> DNA
   <213> Artificial
<220>
   <223> Synthetic primer
<400> 3
   ctcgaaatta accctcacta aaggg 25
<210> 4
   <211> 84
   <212> DNA
   <213> Artificial
<220>
   <223> Synthetic primer
<400> 4
   ctgtctagac aaaaacaatg actctgaatt aaagtctctt gaactttaat tcagagtcat 60
   tgcggggatc tgtggtctca taca 84
<210> 5
   <211> 84
   <212> DNA
   <213> Artificial
<220>
   <223> Synthetic primer
<400> 5
   ctgtctagac aaaaaaccat acattcaagt tgattctctt gaaatcaact tgaatgtatg 60
   gtcggggatc tgtggtctca taca 84

## Description

### BACKGROUND OF THE INVENTION

This invention was made with government support under R01CA107420 awarded by the National Cancer Institute. The government has certain rights in the invention.

### 1. Field of the Invention

The present invention relates generally to the fields of molecular biology and medicine. More particularly, it concerns treatments for hyperproliferative diseases involving the disruption of Cripto/GSP78 signaling.

### 2. Description of Related Art

Cripto (Cripto-1, TDGF1) is a small, GPI-anchored signaling protein with essential physiological roles during embryogenesis. It is also expressed at high levels in human tumors and has been linked to several aspects of tumor initiation and progression including increased cellular proliferation, migration, invasion, tumor angiogenesis and epithelial to mesenchymal transition (EMT) (Strizzi *et al*., 2005).

Multiple mechanisms of action have been attributed to Cripto that are thought to underlie its oncogenic function (Strizzi *et al*., 2005). For example, it modulates signaling of TGF-β superfamily members by forming complexes with some of these ligands and their respective signaling receptors. In this context, Cripto has an obligatory role in facilitating signaling by certain ligands such as Nodal (Schier, 2003; Shen and Schier, 2000) while inhibiting signaling by activins (Adkins *et al*., 2003; Gray *et al*., 2003) and TGF-β1 (Reddy *et al*., 2003). Since activins and TGF-βs have tumor suppressor function (Pardali and Moustakas, 2007), inhibition of their signaling by Cripto provides a mechanism that may at least partly explain the ability of Cripto to promote tumor growth (Adkins *et al*., 2003; Gray *et al*., 2003; Gray *et al*., 2006). Conversely, Cripto-dependent Nodal signaling may contribute to late stages of tumor growth and metastasis under conditions in which cells have become refractory to growth inhibitory effects of TGF-β ligands (Pardali and Moustakas, 2007; Topczewska *et al*., 2006).

Cripto can also be released from the cell in a soluble form and act in a manner resembling that of secreted growth factors (Strizzi *et al*.2005). In this regard, it was reported that Cripto and the Xenopus Cripto ortholog FRL-1 cause phosphorylation of erbB-4 (Bianco *et al.,* 1999) and FGFR-1 (Kinoshita *et al*., 1995), respectively. Cripto does not bind these proteins directly, however, and a putative Cripto receptor mediating these phosphorylation events is yet to be found (Bianco *et al*., 1999; Kinoshita *et al*., 1995). In this regard, although Cripto possesses an EGF-like domain and resembles EGF receptor ligands, it does not directly bind to any of the members of the EGF receptor family (Bianco *et al*., 1999). Furthermore, while Cripto binds the extracellular GPI-anchored proteoglycan Glypican-1 to cause activation of MAPK and PI3K pathways via c-Src, a transmembrane protein mediating this action has not yet been identified (Bianco *et al*., 2003).

Therefore, while Cripto has multiple signaling mechanisms that may contribute to tumor growth, its known cell surface binding partners do not appear to fully explain its reported oncogenic functions. In view of the significant social and economic impact of hyperproliferative diseases, there exists a need for improved therapies for hyperproliferative diseases, and the dileneation of the mechanism by which Cripto causes these effects could yeild improved therapies and screening methods.

### SUMMARY OF THE INVENTION

The present invention overcomes limitations in the prior art by determining that Cripto can bind glucose regulated protein 78 (GRP78) and result downstream signaling which promotes the growth of hyperproliferative cells. Accordingly, the present invention provides inhibitors of the Cripto/GRP78 interaction which may be used to treat a disease such as a cancer.

More specifically, the present invention relates to the embodiments characterized in the claims. Thus, it relates to the following items:
1. A selective GRP78/Cripto targeting compound for use in treating a hyperproliferative disease, wherein said use comprises inhibiting Cripto signaling in a cancerous, pre-cancerous, or malignant cell to reduce the cell's proliferation by contacting the cell with an amount of said selective GRP78/Cripto targeting compound,
   wherein said targeting compound is effective to inhibit the formation of complexes between Cripto and GRP78, thereby reducing the cell's proliferation,
   wherein said targeting compound is an anti-GRP78 antibody that binds an N-20 epitope of the GRP78, wherein said N-20 epitope consists of 20 amino acid residues within the first 50 residues downstream from the GRP78 signal peptide.
2. The selective GRP78/Cripto targeting compound for the use according to item 1, wherein the antibody is a human or humanized monoclonal antibody, is a bispecific antibody, or is conjugated to a reporter molecule.
3. The selective GRP78/Cripto targeting compound for the use according to item 2, wherein the reporter molecule is a radioligand or a fluorescent label.
4. The selective GRP78/Cripto targeting compound for the use according to any one of items 1 to 3, wherein antibody is an anti-GRP78 scFv, F(ab) or F(ab)2.
5. The selective GRP78/Cripto targeting compound for the use according to item 1, wherein the hyperproliferative disease is cancer.
6. The selective GRP78/Cripto targeting compound for the use according to any one of items 1 to 5, wherein the cancer is selected from the group consisting of breast cancer, colon cancer, stomach cancer, pancreatic cancer, lung cancer, ovarian cancer, endometrial cancer, testicular cancer, bladder cancer, prostate cancer, head and neck cancer, cervical cancer, gall bladder cancer, or adrenocortical carcinoma.
7. A humanized monoclonal anti-GRP78 antibody, wherein the antibody binds an N-20 epitope in GRP78, wherein said N-20 epitope consists of 20 amino acid residues within the first 50 residues downstream from the GRP78 signal peptide and wherein said binding inhibits the formation of Cripto/GRP78 complexes.
8. The antibody of item 7, wherein the antibody is comprised in a pharmaceutical composition.

In other aspects, there are disclosed methods for screening for modulators of Cripto/GRP78 complex formation.

The invention is at least partially based on the surprising discovery that cell surface GRP78 is a Cripto binding partner and is necessary for Cripto signaling in human tumor cells, human embryonic stem cells and normal human mammary epithelial cells. Thus, the cell surface Cripto/GRP78 complex represents a novel and desirable target in stem cells and tumor cells which may be targeted therapeutically. The inventors have further shown below that the cell surface Cripto/GRP78 interaction is required for Cripto co-receptor function and tumor growth factor activity. The results demonstrate that knockdown or immunoneutralization of cell surface GRP78 blocks Cripto modulation of activin, Nodal and TGF-β signaling and prevents Cripto activation c-Src/MAPK/PI3K pathways. The data thus support the idea that GRP78 is a Cripto receptor/co-factor that is essential for Cripto signaling. Importantly, the inventors have provided the first demonstration that GRP78 is present at the surface of human ES cells where it co-localizes with Cripto and mediates the opposing effects of Cripto on activin and Nodal signaling. Cripto binding to cell surface GRP78 was also required for the ability of Cripto to increase cellular proliferation and decrease E-Cadherin expression and cellular adhesion, indicating these proteins may work together to promote tumor growth and metastasis. The inventors found that activin-A and Nodal are mitogenic in the presence of Cripto/GRP78 complexes whereas activin-A had cytostatic effects and Nodal had no effect on proliferation in their absence. Without wishing to be bound by any theory, this result support the idea that the cell surface Cripto/GRP78 complexes regulate cellular proliferation by coordinating crosstalk between MAPK/PI3K and Smad2/3 pathways.

There is also disclosed a method for inhibiting Cripto signaling in a cell comprising the step of inhibiting the formation of complexes between Cripto and GRP78. The method may comprise contacting at least the surface of said cell with a selective GRP78-targeting compound, wherein said inhibiting comprises inhibiting the formation of Cripto/GRP78 complexes at about the surface of the cell. The formation of said complexes may be inhibited by an anti-GRP78 antibody, and the anti-GRP78 antibody may bind an N-20 epitope of the GRP78. In certain embodiments, the anti-GRP78 antibody is a humanized monoclonal antibody. The antibody may be conjugated to a reporter molecule, such as a radioligand or a fluorescent label. The formation of said complexes may be inhibited by an anti-GRP78 F(ab) or F(ab)₂, or a GRP78-targeting shRNA, siRNA, or siNA (e.g., GRP78-targeting shRNA comprising SEQ ID NO:5). The administration may be systemic, local, regional, parenteral, intravenous, intraperitoneal, via inhalation, or intratumoral.

As disclosed herein, the method is further defined as a method of decreasing cell proliferation comprising contacting a cell with an effective amount of a GRP-78-targeting compound that preferentially binds GRP-78 and inhibits the ability of the GRP-78 to bind Cripto and cause Nodal signaling. The method may comprise contacting at least the surface of said cell with a Cripto-targeting compound, wherein the Cripto-targeting compound selectively binds to an epitope in a CFC domain or a GRP78-binding domain of Cripto and inhibits the formation of complexes between Cripto and GRP78. The Cripto-targeting compound may be an antibody which binds to an epitope in the GRP78-binding domain or CFC domain of Cripto. The method may comprise contacting at least the surface of said cell with a shRNA, wherein the shRNA comprises SEQ ID NO:4. The targeting compound may be a GRP78 mutant which does not bind or essentially does not bind Cripto. The GRP78 mutant may be a GRP78 mutant lacking amino acids 19-68 of natural GRP78, such as Δ19-68 GRP78. In other embodiments, GRP78 may be mutated via one or more substitution or insertion mutation(s) in the 19-68 amino acid region of GRP78 to produce a GRP78 mutant which does not bind or essentially does not bind Cripto. Said cell may be derived from an organ selected from the group consisting of breast, colon, stomach, pancreas, lung, ovary, endometrial, testis, bladder, prostate, head, neck, cervix, gastric, gall bladder and adrenal cortex. The cell may be cancerous, pre-cancerous, or a malignant cell, and wherein the method is further defined as a method of treating a hyperproliferative disease, such as a cancer. The cancer may be selected from the group consisting of breast cancer, colon cancer, stomach cancer, pancreatic cancer, lung cancer, ovarian cancer, endometrial cancer, testicular cancer, bladder cancer, prostate cancer, head and neck cancer, cervical cancer , gall bladder cancer, or adrenocortical carcinoma.

As disclosed herein, the method is further defined as a method for decreasing cell proliferation. The method may comprise a method of decreasing Nodal signaling, activin/TGF-β signaling or c-Src/MAPK/PI3K signaling by Cripto in the cell. The method may comprise the administration of a second cancer therapy to the subject, such as a chemotherapy (*e.g*., taxol, cisplatin, or carboplatin), a radiotherapy, a gene therapy, an immunotherapy or a surgery.

The method may be further defined as a method of promoting the differentiation of a stem cell into a neuronal cell, wherein the cell is a stem cell, and wherein inhibiting the formation of complexes between Cripto and GRP78 promotes differentiation of the cell into a neuronal cell. The cell may be a human embryonic stem cell (*e.g*., H9 or BG02) or an induced pluripotent stem cell (iPSC).

There is also disclosed a method of screening for an inhibitor of Cripto/GRP78 complex formation comprising: obtaining a candidate modulator, contacting the candidate modulator with a Cripto and a GRP78, and measuring the formation of Cripto/GRP78 complexes, wherein decrease in the formation of Cripto/GRP78 complexes or a decrease in Cripto/GRP78 complex signaling in the presence of the candidate modulator indicates that the candidate modulator is an inhibitor of Cripto/GRP78 complex formation. The Cripto and the GRP78 may be expressed by a cell. In certain embodiments, the Cripto and the GRP78 are transgenically over-expressed by the cell. The cell may be a cancerous or pre-cancerous cell. The method may comprise measuring Cripto/GRP78 signaling, wherein the Cripto/GRP78 signaling comprises activin/TGF-β signaling, c-Src/MAPK/PI3K signaling or PI3K/Akt/GSK3β signaling. The method may comprise measuring binding between the Cripto and the GRP78, wherein a decrease in Cripto/GRP78 binding in the presence of the candidate modulator indicates the candidate modulator inhibits Cripto/GRP78 complex formation. Said measuring binding may comprise a cell surface biotinylation/Co-IP assay *(e.g.,* as described in Shani et al 2008), a ¹²⁵I-Cripto binding assay (*e.g*., in intact cells), an assay comprising measuring Cripto binding to immobilized GRP78 (*e.g*., in a multi-well plate), or an ELISA assay to measure soluble Cripto binding. In various embodiments, fluorescence assay comprising tagging the Cripto and GRP78 with a fluorophore or a quencher and measuring fluorescence, *e.g*., using FACS, may be used with the present invention.

Yet another aspect of the present invention relates to a humanized monoclonal anti-GRP78 antibody, wherein the antibody binds an N-20 epitope in GRP78,
wherein said N-20 epitope consists of 20 amino acid residues within the first 50 residues downstream from the GRP78 signal peptide, and wherein said binding inhibits the formation of Cripto/GRP78 complexes. The antibody may be comprised in a pharmaceutical composition, and the pharmaceutical composition may be formulated for parenteral, intravenous, or intratumoral administration.

The terms "inhibiting," "reducing," or "prevention," or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed in this specification can be implemented with respect to any method or composition of the invention, and vice versa. Furthermore, compositions of the invention can be used to achieve methods of the invention.

Throughout this application, the term "about" is used to indicate that a value includes the inherent variation of error for the device, the method being employed to determine the value, or the variation that exists among the study subjects.

The use of the term "or" in the claims is used to mean "and/or " unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

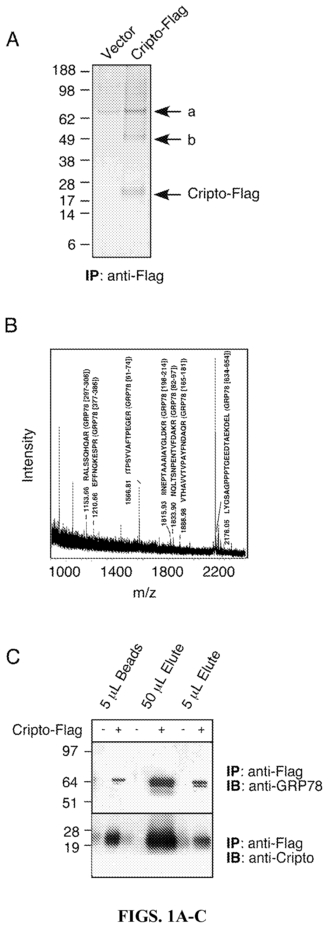

**FIGS. 1A-C****. Identification of novel Cripto binding proteins.** 293T cells were transfected with empty vector or Cripto-Flag, subjected to immunoprecipitation on anti-Flag beads and then eluted with Flag peptide. Cripto-associated proteins were separated by SDS-PAGE and then either silver stained (A) or blotted to nitrocellulose and probed with anti-GRP78 or anti-Cripto antibodies (C) as described under Materials and Methods. Bands designated as *a* and *b* in (A) were excised and subjected to mass spectrometric analysis as described under Materials and Methods. Mass fingerprint data corresponding to band *a* identified as GRP78 are shown in (B).

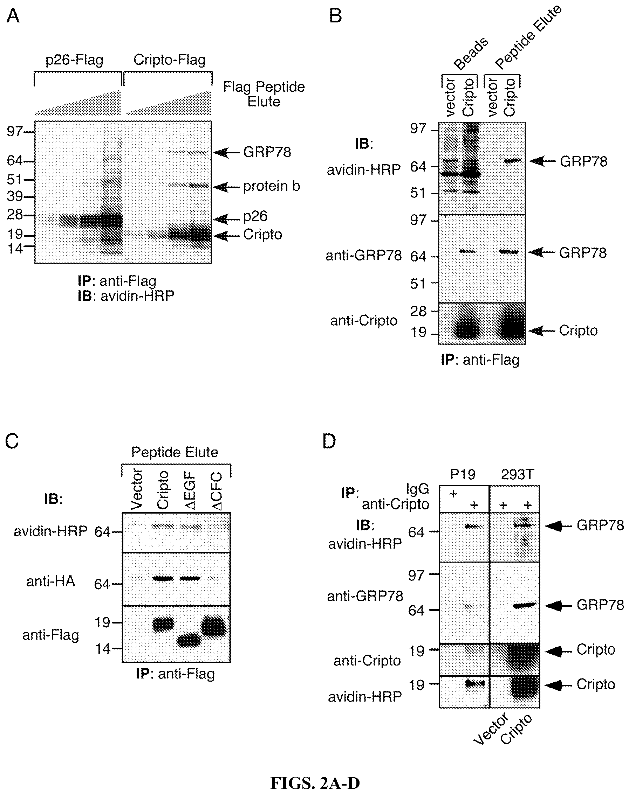

**FIGS. 2A-D****. Cripto binds GRP78 at the cell surface.** 293T cells transfected with the indicated constructs (A-D) and P19 cells (D) were labeled with cell impermeable NHS-LC-biotin. Cell lysates were subjected to immunoprecipitation using the indicated antibodies and then eluted with Flag peptide (Peptide Elute) or by heating the beads in sample buffer. Samples were resolved via SDS-PAGE and blotted with avidin-HRP or the indicated antibodies as described under Materials and Methods. In some cases (B and C), 293T cells overexpressing GRP78 were subjected to cell surface biotinylation and then resulting cell lysates were incubated with vector or Cripto-Flag beads.

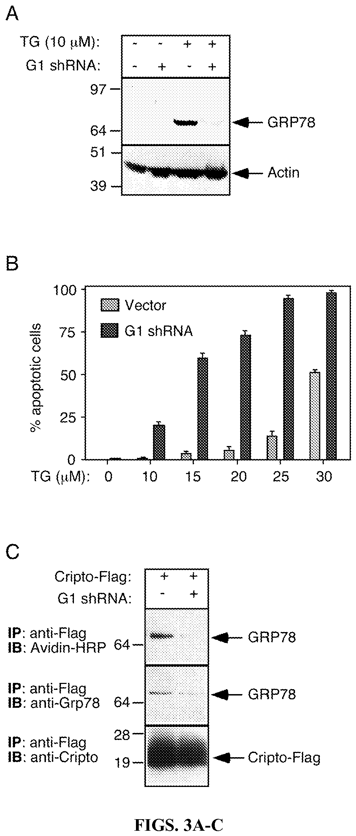

**FIGS. 3A-C****. Targeted reduction of GRP78 expression using RNA interference.** HeLa cells were infected with lentivirus containing either GRP78 shRNA (G1) or empty vector and either left untreated or treated with thapsigargin as indicated. (A) Cell lysates were analyzed by Western blot using anti-GRP78 or anti-actin antibodies as described in Materials and Methods. (B) Bars represent the number of apoptotic cells per 100 GFP positive cells as described in Materials and Methods. (C) Cells infected with vector or G1 shRNA virus were re-infected with virus containing Cripto-Flag. Lysates from these cells were subjected to immunoprecipitation using anti-Flag beads and then eluted with Flag peptide. Eluted proteins were subjected to Western blot analysis using avidin-HRP, anti-GRP78 and anti-Cripto as described under Materials and Methods.

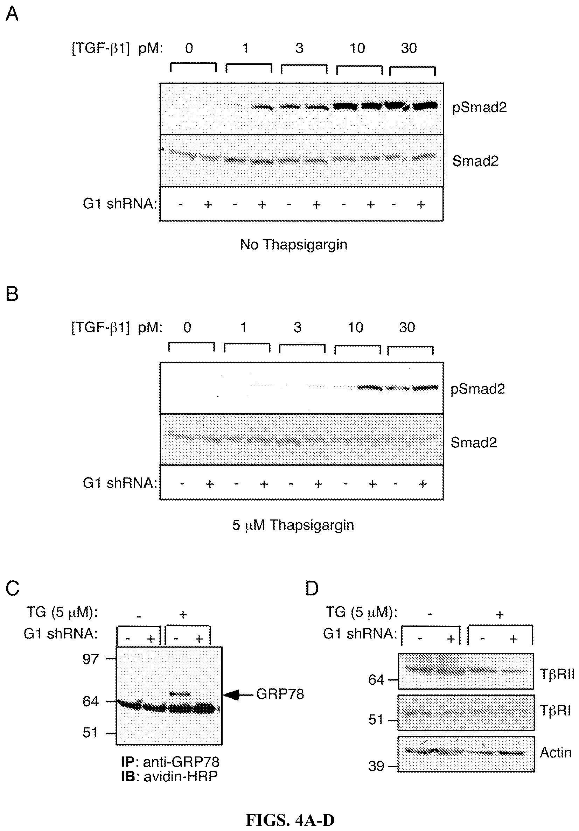

**FIGS. 4A-D****. Inhibition of endogenous GRP78 expression enhances TGF-β induced Smad2 phosphorylation.** HeLa cells were infected with lentivirus containing either shRNA (G1) targeted against GRP78 or empty vector and then left untreated or treated with 5µM thapsigargin as indicated. Following overnight thapsigargin treatment, cells were treated with the indicated doses of TGF-β1 (A and B) and then phospho-Smad2 and total Smad2 levels were determined by Western blot as described under Materials and Methods. Alternatively, the same cells treated as indicated were either labeled with cell impermeable biotin with resulting lysates subjected to immunoprecipitation with anti-GRP78 antibodies (anti-KDEL) followed by Western blotting using avidin-HRP (C) or lysates were subjected directly to Western blotting using anti-TβRI, anti-TβRII or anti-actin antibodies (D) as described in Materials and Methods.

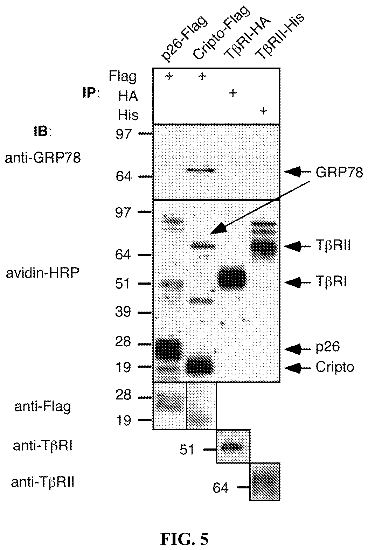

**FIG. 5****. GRP78 does not bind directly to TGF-β type I and type II receptors.** 293T cells were transfected with p26-Flag, Cripto-Flag, TβRI-HA or TβRII-His and then subjected to immunoprecipitation using anti-Flag, anti-HA or anti-His antibodies as indicated. Precipitated proteins were analyzed via Western blotting using avidin-HRP or the indicated antibodies as described under Materials and Methods.

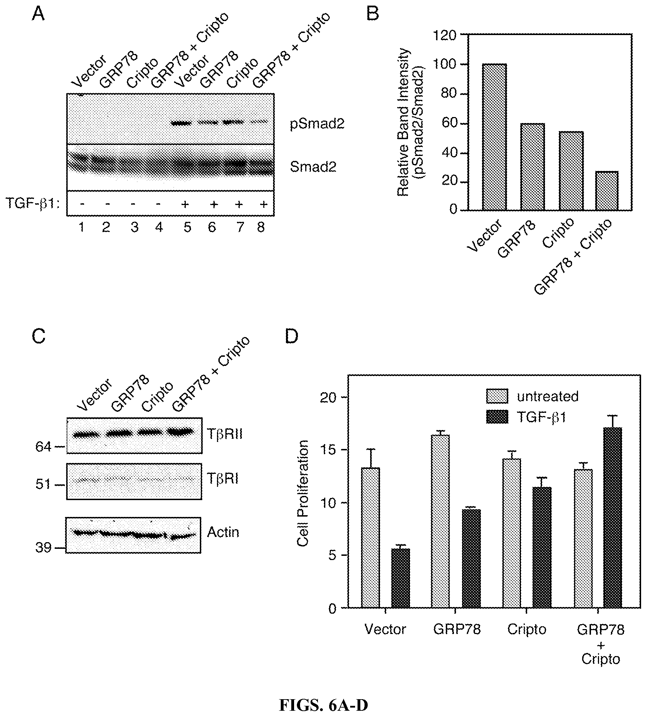

**FIGS. 6A-D****. Cripto and GRP78 cooperate to inhibit TGF-β signaling.** (A) PC3 cells infected with empty vector, GRP78, Cripto or both were either left untreated or treated with TGF-β1 (10 pM) as indicated. Phospho-Smad2 (pSmad2) and total Smad2 (Smad2) levels were determined by Western blot as described under Materials and Methods. (B) Phospho-Smad2 bands from (A) were quantitated using densitometry and normalized relative to corresponding Smad2 bands as described under Materials and Methods. (C) PC3 cell lysates were subjected to Western blotting using anti-TβRII, anti-TβRI and anti-actin as indicated and as described under Materials and Methods. (D) Cells were plated on 96 well plates and then cell proliferation was measured 8 days later as described under Materials and Methods.

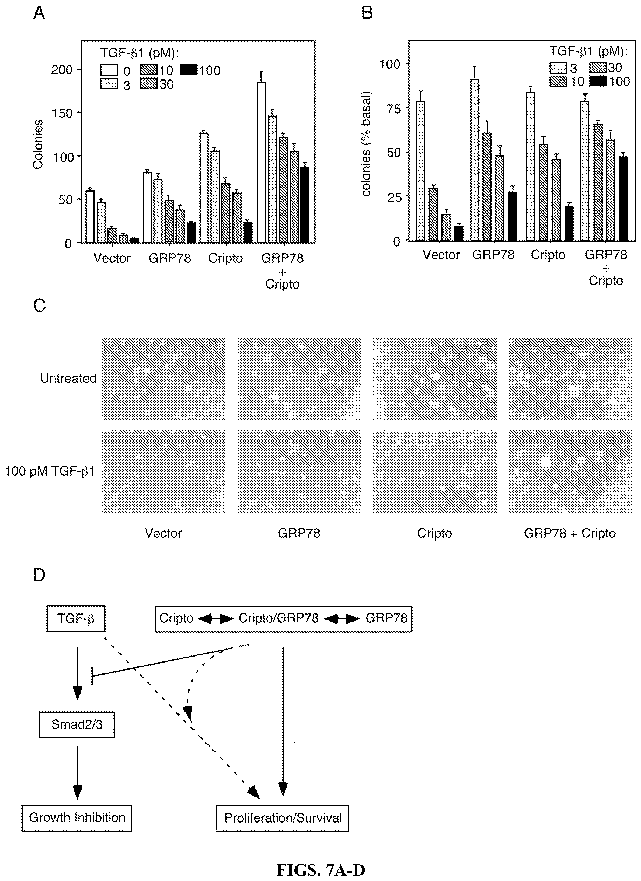

**FIGS. 7A-D****. GRP78 and Cripto collaborate to inhibit the antiproliferative effects of TGF-β on anchorage independent growth of prostate carcinoma cells.** PC3 cells infected with vector, GRP78, Cripto or both were grown for 15 days under anchorage independent conditions in soft agar in the presence of either vehicle or escalating doses of TGF-β1 as described under Materials and Methods. Data are presented as the number of colonies counted within a single field in the absence or presence of the indicated doses of TGF-β1 (A) or as the number of colonies counted in the presence of the indicated TGF-β1 concentrations divided by the number of colonies counted in the absence of TGF-β1 treatment (% basal) (B). (C) Photographs taken from the indicated fields either in the presence of 100 pM TGF-β1 or in its absence. (D) Model illustrating oncogenic function of Cripto/GRP78 complex. TGF-β potently inhibits proliferation of many cell types by signaling via the Smad2/3 pathway (left). Cripto and GRP78 interact to form a complex and act cooperatively to attenuate TGF-β-dependent Smad signaling and growth inhibition. In addition, they independently increase cell proliferation/survival. In the presence of Cripto and GRP78 TGF-β also can increase cellular proliferation (dashed arrow).

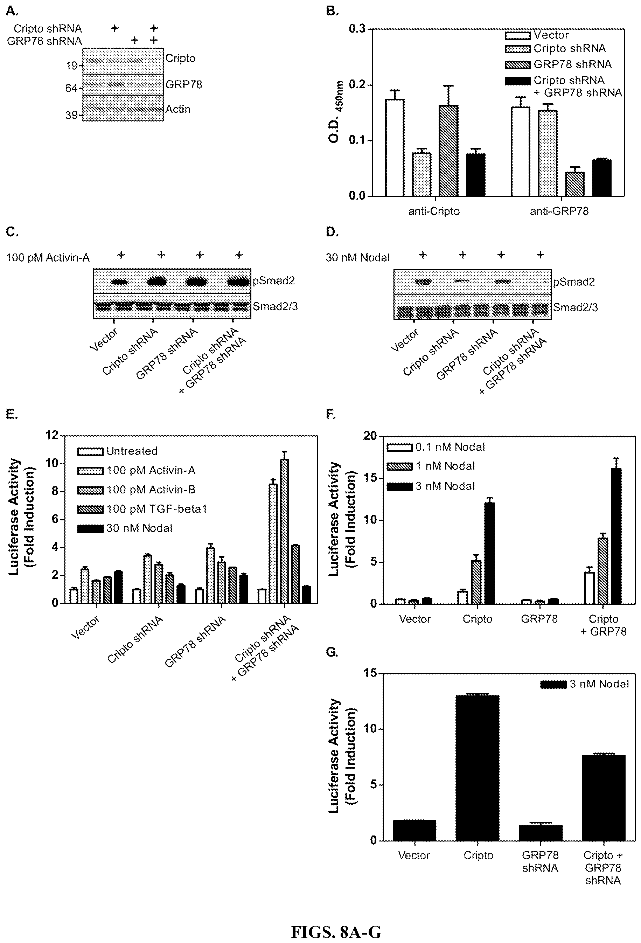

**FIGS. 8A-G****. Cripto and GRP78 cooperatively regulate activin, Nodal and TGF-β signaling.** NCCIT cells stably expressing Cripto and/or GRP78 shRNAs were analyzed by Western blot (A) or intact cell surface ELISA (B) using the indicated antibodies. The same cells were treated with activin-A (C) or Nodal (D) and resulting levels of phospho-Smad2 (pSmad2) and Smad2 were measured by Western blot using pSmad2 and Smad2 antibodies. NCCIT cells (E) and 293T cells (F, G) overexpressing the indicated proteins and/or shRNAs were transfected with a Smad2-responsive luciferase reporter and treated with the indicated doses of TGF-β ligands. Resulting luciferase activities were normalized and are presented as fold induction over untreated samples.

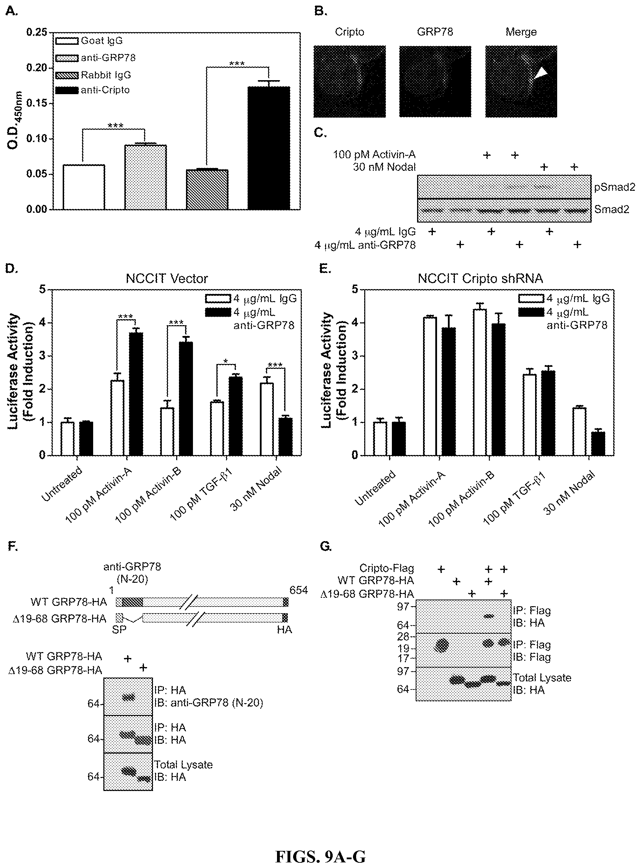

**FIGS. 9A-G****. Cell surface GRP78 mediates Cripto signaling in human ES cells.** H9 human ES cells were subjected to intact cell ELISA (A) or immunofluorescence (B) using the indicated antibodies. In (B), anti-Cripto staining is green and anti-GRP78 staining is red. (C) H9 ES cells were treated with activin-A or Nodal as indicated and resulting levels of phospho-Smad2 (pSmad2) and Smad2 were measured by Western blot using pSmad2 and Smad2 antibodies. NCCIT cells stably infected with empty vector (D) or Cripto shRNA (E) were transfected with a Smad2-responsive luciferase reporter and treated with the indicated doses of TGF-β ligands in the absence or presence of N-20 antibody as indicated. Resulting luciferase activities were normalized and are presented as fold induction over untreated samples. (F) Diagram illustrating wild type GRP78 and the Δ19-68 GRP78 construct lacking the N-20 epitope. (F, G) Lysates from 293T cells transfected with the indicated constructs were subjected to immunoprecipitation and Western blotting using anti-HA, anti-Flag and anti-GRP78 antibodies as indicated. *p<0.01; ***p<0.001.

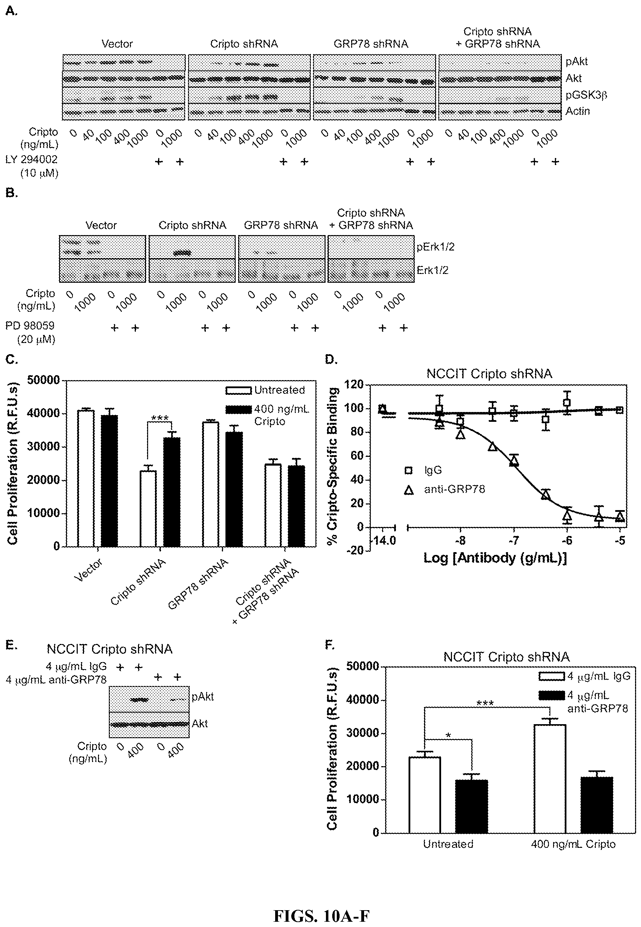

**FIGS. 10A-F****. Cripto requires GRP78 receptor function to activate PI3K and MAPK pathways and promote proliferation of NCCIT cells.** NCCIT cells stably expressing the indicated shRNAs were serum starved and then treated with the indicated doses of soluble Cripto and either the PI3K inhibitor (LY2940002) (A) or the MEK1/2 inhibitor (PD98059) (B) as indicated. Cell lysates were subjected to Western blotting using anti-phospho-Akt (pAkt), Akt, phospho-GSK3β (pGSK3β) and actin antibodies (A) or phospho-ERK1/2 (pERK1/2) and ERK1/2 antibodies (B) as indicated. (C) The same NCCIT cells were treated with Cripto as indicated, grown for 8 days and then proliferation was measured using the CyQuant proliferation assay kit. (D) NCCIT cells infected with Cripto shRNA were subjected to ¹²⁵I-Cripto binding in the presence of a range of doses of N-20 antibody or IgG control. Cripto specific binding represents the amount of ¹²⁵I-Cripto binding that is blocked by an excess of unlabeled soluble Cripto. NCCIT cells infected with Cripto shRNA were (E) serum-starved and then treated with the indicated dose of soluble Cripto after pretreatment with the indicated dose of IgG or anti-GRP78 (N-20) or (F) treated with soluble Cripto following pretreatment with IgG or anti-GRP78 (N-20) antibody as indicated. Cells were grown for an additional 8 days and proliferation was measured using the CyQuant proliferation assay kit. *p<0.01; ***p<0.001.

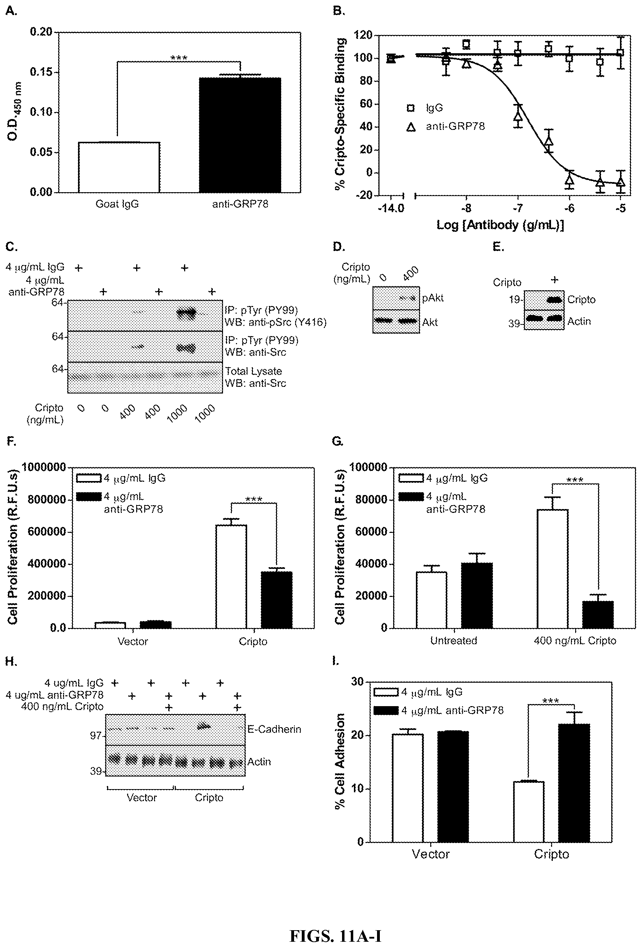

**FIGS. 11A-I****. Immunoneutralization of cell surface GRP78 blocks Cripto tumor growth factor activity in human mammary epithelial cells.** Human mammary epithelial MCF10A cells infected with empty vector were subjected to intact cell ELISA using IgG or N-20 antibody (A) or to ¹²⁵I-Cripto binding in the presence of a range of doses of N-20 antibody or control IgG antibody as indicated (B). (C) MCF10A cells infected with empty vector were serum starved and then treated with the indicated doses of soluble Cripto, N-20 antibody and/or IgG as indicated. Resulting cell lysates were subjected to immunoprecipitation with anti-phospho-Tyr (pTyr) antibody and Western blotting with anti-phospho-Src (pSrc, Y416) or anti-Src antibodies as indicated. (D) MCF10A cells infected with empty vector were serum starved, treated with the indicated dose of soluble Cripto and then cell lysates were analyzed by Western blot using phospho-Akt (pAkt) and Akt antibodies as indicated. (E) Cell lysates from empty vector-infected or Cripto-infected MCF10A cells were analyzed by Western blot using Cripto and actin antibodies as indicated. MCF10A cells infected with either empty vector or Cripto (F) or with empty vector (G) were treated with soluble Cripto, IgG and/or N-20 antibody as indicated. Cells were grown for 8 days and proliferation was measured using the CyQuant proliferation assay kit. (H) MCF10A cells infected with empty vector or Cripto were treated with soluble Cripto after pretreatment with IgG or N-20 antibody as indicated. Cell lysates were analyzed by Western blot using E-Cadherin and actin antibodies. (I) MCF10A cells infected with empty vector or Cripto were pre-treated with IgG or N-20 antibody, plated and allowed to adhere. Resulting cell adhesion was quantified using the CyQuant adhesion assay. ***p<0.001.

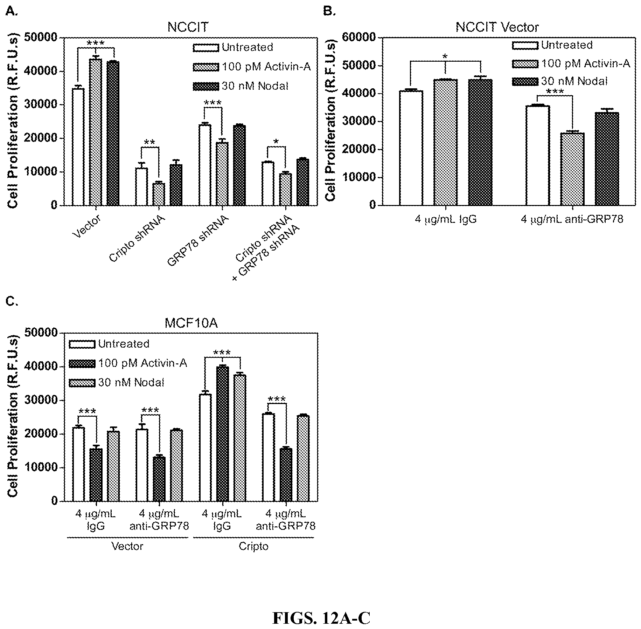

**FIG. 12A-C****. Cripto and GRP78 are required for pro-proliferative effects of activin-A and Nodal in NCCIT and MCF10A cells.** NCCIT cells infected with empty vector, Cripto and/or GRP78 shRNAs (A, B) and MCF10A cells infected with empty vector or Cripto (C) were left untreated or treated with activin-A or Nodal in the absence or presence of IgG or N-20 antibody as indicated. Cells were grown for an additional 8 days and proliferation was measured using the CyQuant proliferation assay kit. *** p<0.001; ** p<0.005; * p<0.01.

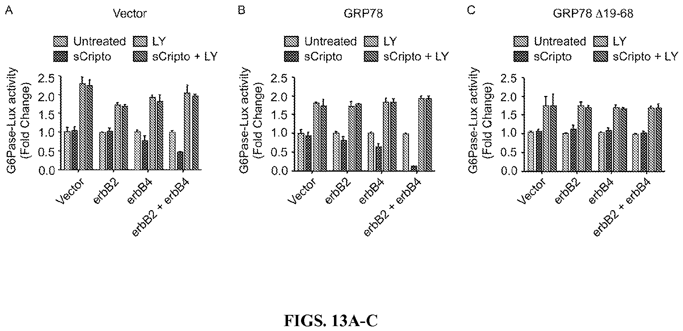

**FIGS. 13A-C****. GRP78 D19-68 inhibits Cripto signaling via erbB2, erbB4 and PI3K.** 293T cells were transfected as indicated with empty vector, ErbB2 and/or ErbB4 expression vectors together with glucose-6-phosphatase-luciferase (G6Pase-Lux) and b-galactosidase constructs. In addition, cells were transfected with (A) empty vector, (B) GRP78 or (C) GRP78 D19-68. Cells were left untreated or treated as indicated with Cripto (400 ng/ml) and/or LY294002 (LY). Resulting luciferase activities were normalized relative to b-galactosidase expression and are presented as fold change relative to untreated samples.

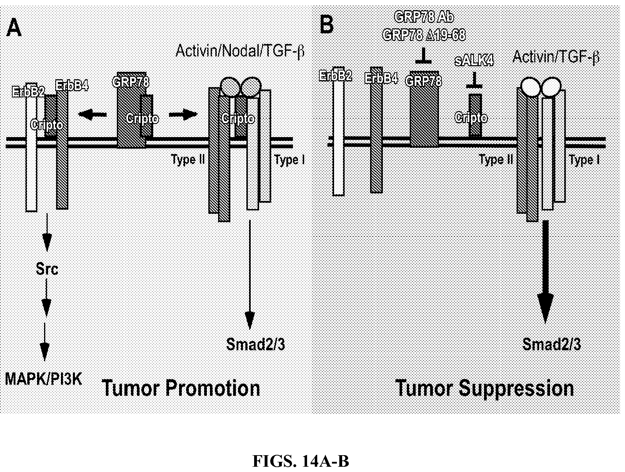

**FIGS. 14A-B****. Model illustrating proposed mechanisms of Cripto/GRP78 signaling and antagonism.** (A) The cell surface Cripto/GRP78 complex is necessary for oncogenic Cripto signaling via MAPK/PI3K and Smad2/3 pathways. Cripto binding to cell surface GRP78 leads to activation of cSrc/MAPK/PI3K pathways via ErbB2 and ErbB4. Cripto binding to cell surface GRP78 also facilitates Cripto effects on signaling by activin/Nodal/TGF-β ligands resulting in low levels of Smad2/3 activation. (B) Reagents that disrupt the oncogenic function of the cell surface Cripto/GRP78 complex include the N-20 GRP78 antibody, the GRP78 D19-68 mutant and sALK4-L75A-Fc.

### DESCRIPTION OF ILLUSTRATIVE EMBODIMENTS

The present disclosure overcomes limitations in the prior art by providing methods for inhibiting Cripto/GRP78 complex formation and screening methods for putative Cripto/GRP78 complex modulators. Cripto is a multifunctional cell surface protein with important roles in vertebrate embryogenesis and the progression of human tumors. While it has been shown to modulate multiple signaling pathways, its previously identified binding partners have not fully explained its molecular actions. The inventors conducted a screen aimed at identifying novel Cripto interacting proteins that led to the identification of Glucose Regulated Protein 78 (GRP78), an ER chaperone that is also expressed at the surface of tumor cells. As shown in the below examples, Cripto and GRP78 interact at the cell surface of multiple cell lines and that their interaction is independent of prior association within the ER. shRNA knockdown of endogenous GRP78 resulted in enhanced TGF-β signaling indicating that, like Cripto, GRP78 inhibits this pathway. When co-expressed, GRP78 and Cripto collaborate to antagonize TGF-β responses including Smad phosphorylation and growth inhibition of prostate cancer cells grown under anchorage dependent or independent conditions. The below examples further provide evidence that cells co-expressing GRP78 and Cripto grow much more rapidly in soft agar than cells expressing either protein individually.

Inhibition of GRP78/Cripto complex formation can disrupt Cripto signaling and decrease cell proliferation. Loss or immunoneutralization of cell surface GRP78 blocked Cripto-dependent Nodal signaling, antagonism of activin/TGF-β signaling and activation of ERK/MAPK and PI3K/Akt/GSK3β pathways. The inventors have shown that GRP78 is present at the surface of human ES cells where it co-localizes with Cripto and mediates the opposing effects of Cripto on activin and Nodal signaling. In addition, knockdown or immunoneutralization of cell surface GRP78 blocked the ability of Cripto to cause increased cell proliferation, decreased E-Cadherin expression and decreased cell adhesion. The inventors found that while activin-A was cytostatic in the absence of cell surface Cripto/GRP78 complexes, activin-A and Nodal both increased cellular proliferation in their presence. Together, the data indicate that cell surface GRP78 is required for Cripto signaling and supports the idea that GRP78 mediates Cripto function during normal embryonic development and tumorigenesis.

### I. CRIPTO

Cripto is a GPI-anchored signaling protein with important roles during development and cancer progression *(Strizzi et al*., 2005). In the developing mouse embryo, Cripto is required for proper establishment of the anterior-posterior axis and germ layer formation and cardiogenesis. Cripto has also been recognized as a marker of embryonic stem cells with important roles in pluripotency maintenance and differentiation (Adewumi *et al*., 2007; Strizzi *et al*., 2005). While Cripto expression is generally low or absent in normal adult tissues, it is found at high levels in many human solid tumors and its overexpression promotes anchorage independent growth, proliferation, survival, migration, invasion, angiogenesis and EMT (Strizzi *et al*., 2005). Cripto also promotes tumor growth *in vivo* since MMTV-Cripto and WAP-Cripto mice develop mammary tumors (Strizzi *et al*., 2005; Strizzi *et al*., 2004; Sun *et al.,* 2005; Wechselberger *et al*., 2005) and monoclonal antibodies targeting Cripto reduce the growth of tumor xenografts in nude mice (Adkins *et al*., 2003; Xing *et al*., 2004).

Similar to other growth factors, Cripto can be released from cells following cleavage of its GPI anchor and soluble forms of Cripto have been shown to activate mitogenic ras/raf/MAPK and PI3K/Akt pathways (Strizzi *et al*., 2005). Cripto also acts as an obligatory co-receptor for TGF-β superfamily members such as Nodal (Schier, 2003; Shen, 2007), GDF1 (Cheng *et al.,* 2003) and GDF3 (Chen *et al*., 2006). This co-receptor function is essential during embryogenesis (Schier, 2003; Strizzi *et al.,* 2005) and may regulate normal tissue growth and remodeling in the adult as indicated by the fact that Cripto and Nodal are co-expressed in the mammary gland during pregnancy and lactation *(Bianco et al.,* 2002a). Cripto co-receptor function may also promote tumor growth since Nodal signaling was recently shown to play a key role in promoting plasticity and tumorigenicity of human melanoma and breast cancer cells *(Postovit et al.,* 2008; *Topczewska et al.,* 2006). Cripto also inhibits signaling by activins (Adkins)(Gray *et al.,* 2003; Kelber *et al*., 2008) and TGF-β1 (Gray *et al*., 2006) and inhibits TGF-β1-dependent antiproliferative effects on human breast epithelial cells and prostate cancer cells (Gray *et al*., 2006; Shani *et al.,* 2008). Therefore, Cripto can promote tumorigenesis by activating growth/survival pathways and by inhibiting tumor suppressor pathways (Strizzi *et al*., 2005).

### II. GRP78

The inventors have identified GRP78 as a novel Cripto binding partner and shown that these two proteins form a cell surface complex that inhibits cytostatic TGF-β signaling and promotes tumor cell growth. GRP78 has been extensively characterized as an ER chaperone that assists in protein folding, maturation and assembly and also coordinates the unfolded protein response (UPR) (Bernales *et al*., 2006; Lee, 2005; Lee, 2001). GRP78 is induced under conditions of hypoxia and nutrient deprivation and is found at high levels in tumor cells (Lee, 2007). Evidence for a necessary role for GRP78 in tumor progression first emerged from the demonstration that inhibition of GRP78 induction in fibrosarcoma cells with antisense rendered them completely incapable of forming tumors in nude mice without affecting their growth *in vitro* (Jamora *et al*., 1996). Moreover, delivery of a suicide transgene driven by the GRP78 promoter into fibrosarcoma and breast tumor cells caused complete eradication of sizable tumors in mice (Chen *et al*., 2000; Dong *et al*., 2004; Gazit *et al*., 1999). Thus, the environment found within solid tumors causes induction of GRP78 and its expression facilitates tumor growth.

Although GRP78 resides predominantly within the ER, it can exist as a transmembrane protein (Reddy *et al*., 2003) and is localized to the plasma membrane in tumor cells as was initially demonstrated in human rhabdomyosarcoma cells following treatment with thapsigargin (Delpino and Castelli, 2002; Delpino *et al*., 1998). Subsequently, global profiling of the cell surface proteome has confirmed that GRP78 is surface exposed on tumor cells (Shin *et al*., 2003). Indeed, GRP78 has been shown to function together with MHC Class I at the cell surface as a co-receptor for viruses (Triantafilou *et al*., 2002) and to act as a receptor for plasminogen-derived Kringle 5 domain (Davidson *et al*., 2005) and activated α₂-macroglobulin (α₂M) (Misra *et al*., 2002; Misra *et al*., 2004). Of note, GRP78 receptor function was shown to cause activation of growth pathways leading to increased cellular proliferation and anti-apoptotic behavior (Misra *et al*., 2006; Misra *et al*., 2005; Misra *et al*., 2004). Such a receptor function of GRP78 draws cancer-related relevance from the observation that the presence of auto-antibodies to GRP78 has been linked to increased prostate cancer progression and decreased patient survival (Mintz *et al*., 2003). Moreover, a causal role for GRP78 in the progression of cancer was supported by the finding that suicide peptides targeting GRP78 at the plasma membrane were demonstrated to selectively kill tumor cells (Arap *et al*., 2004). Importantly, these findings validate cell surface GRP78 as a putative target for cancer therapy.

Previously, an antibody has been disclosed which binds to the N-terminal region of GRP78; WO 2008/105560 A1.

### III. CRIPTO AND GRP78 BIND AND CAUSE INTRACELLULAR SIGNALING

### PROMOTING CELL PROLIFERATION

Based on evidence that Cripto binds GRP78 at the surface of tumor cells, the inventors evaluated the possibility that GRP78 functions as a Cripto receptor. Consistent with this hypothesis and as shown in the below examples, Cripto binding to cell surface GRP78 is required for Cripto signaling in tumor cells, ES cells and mammary epithelial cells.

Cripto binding to GRP78 results in the activation of several downstream intracellular signaling pathways. For example, as shown in the below examples, Cripto binding to cell surface GRP78 is necessary for Cripto function as an obligatory Nodal coreceptor and antagonist of activin and TGF-β signaling. GRP78 receptor function mediates soluble Cripto tumor growth factor activity including activation of c-Src/MAPK/PI3K pathways, increased cellular proliferation, decreased E-Cadherin expression and decreased
cell adhesion. Activin-A and Nodal have pro-proliferative effects in the presence of Cripto/GRP78 complexes while activin-A is antiproliferative when the Cripto/GRP78 complex is disrupted. Without wishing to be bound by any theory, these findings indicate that GRP78 functions as a cell surface Cripto receptor/co-factor that is required for developmental and oncogenic effects of Cripto on activin/Nodal/TGF-β and MAPK/PI3K signaling pathways.

### A. GRP78 and Cripto form a complex at the cell surface that does not require prior association in the ER.

As demonstrated in the below examples, GRP78 forms a complex with Cripto at the cell surface, a discovery with functional as well as potential therapeutic implications. This interaction appeared to be specific and exclusive since GRP78 was one of only two cell surface proteins observed to co-purify with Cripto and since an irrelevant transmembrane protein similar in size to Cripto did not co-purify with GRP78. Likewise, both type I and type II TGF-β receptors were unable to immunoprecipitate GRP78 under the same conditions. The results also indicate that Cripto/GRP78 binding does not depend on GRP78 ER chaperone function since their interaction was observed in a cell free environment following the maturation and processing of these proteins within separate cell populations. This result also supports the existence of this complex at the cell surface since the GRP78 shown to bind Cripto under these conditions was derived from the plasma membrane. Furthermore, this result suggests that the information required for this specific binding interaction may be contained in full within the tertiary structures of these two proteins and it is anticipated that tumor cells may be targeted using molecules that specifically disrupt their interaction.

The inventors found that endogenous Cripto and endogenous GRP78 could be isolated as a complex originating from the surface of mouse embryonal carcinoma cells. Without wishing to be bound by any theory, this finding further supports an intrinsic role for their interaction as signaling co-factors at the plasma membrane and again argues against the possibility that GRP78 simply plays a role in the folding of overexpressed Cripto in the ER. The inventors have also explored the localization of Cripto and GRP78 in the same cells via immunocytochemistry and found that Cripto and GRP78 were predominantly co-localized in punctate structures, a portion of which were present at the cell surface. These findings support the conclusion that Cripto and GRP78 function together at the plasma membrane, but also indicate that they are associated during vesicular transport as is common for signaling proteins. In addition, the punctate nature of the cell surface staining is consistent with previous reports showing both of these proteins to associate with lipid rafts (Triantafilou and Triantafilou, 2003; Watanabe *et al*., 2007).

### B. GRP78 Functions as a Cell Surface Receptor

GRP78 has protective roles in the ER as a chaperone and coordinator of the UPR, but it can also be expressed at the cell surface where its functions are less well understood. Cell surface GRP78 has been identified as a tumor-specific antigen in primary human breast cancer samples and autoantibodies targeting GRP78 were found in the serum of prostate cancer patients and shown to serve as a biomarker of increased cancer aggressiveness *(Arap et al.,* 2004; *Mintz et al.,* 2003). Also, chimeric peptides composed of GRP78 binding motifs fused to an apoptosis-inducing sequence inhibited tumor growth in mouse models of prostate and breast cancer *(Arap et al.,* 2004; *Liu et al.,* 2007). The data demonstrate that GRP78 is not only a selective cell surface marker of tumor cells but also a receptor/co-factor that mediates Cripto effects on activin/Nodal/TGF-β and MAPK/PI3K signaling. While the discovery of a functional link between GRP78 and activin/Nodal/TGF-β signaling appears to be unique, previous studies have shown that cell surface GRP78 has receptor activity and can mediate growth/survival signaling. For example, α2-macroglobulin (α2-M) signals via cell surface GRP78 to cause activation of MAPK and PI3K pathways in 1-LN prostate carcinoma cells resulting in pro-proliferative and anti-apoptotic behavior (Misra *et al*., 2006; Misra *et al*., 2004). Antibodies from prostate cancer patient serum targeting GRP78 were similarly shown to activate MAPK/PI3K pathways and increase cellular proliferation. Interestingly, cell surface GRP78 was also shown to have an essential role in mediating GPI-anchored T-cadherin-dependent survival signal transduction via Akt in endothelial cells (Philippova *et al*., 2008). Cripto, T-Cadherin and GRP78 have each been localized to lipid rafts suggesting GRP78 receptor function may be restricted to these plasma membrane microdomains.

The below results indicate that GRP78 is required for the ability of Cripto to interact functionally with TGF-β ligands and their receptors. Without wishing to be bound by any theory, the inventors anticipate that GRP78 may serve as an anchor or scaffold at the plasma membrane that allows Cripto to adopt a conformation or orientation required for it to form complexes with TGF-β ligands and their receptors and alter their signaling properties. The CFC of Cripto mediates binding to GRP78 and also to the type I signaling receptors ALK4 and ALK7. Without wishing to be bound by any theory, the inventors anticipate that the binding interactions between Cripto and GRP78 or ALK4/7 will not be mutually exclusive but rather that GRP78 may facilitate complex formation between Cripto and ALK4/7 and ligands such as Nodal and activins. In addition to its role as an essential mediator of Cripto effects on TGF-β ligand signaling, the results also show that GRP78 couples soluble Cripto to activation of MAPK/PI3K pathways. This finding suggests that GRP78 either operates as a transmembrane receptor or couples to one or both. Although GRP78 is generally considered to be a soluble protein restricted to the ER lumen, it was shown that a substantial fraction of GRP78 exists as an integral membrane protein with two putative transmembrane α helices (Reddy *et al*., 2003). Reddy et al used limited trypsin digestion of ER microsomes to show that the N- and C-terminal regions of membrane-associated GRP78 are ER lumenal/extracellular while the middle third of the protein is cytoplasmic (Reddy *et al*., 2003). This unusual transmembrane topology is consistent with the fact that extracellular proteins have been shown to bind GRP78 near its N- or C-terminus (Davidson *et al*., 2005; Gonzalez-Gronow *et al*., 2006; Jakobsen *et al*., 2007; Philippova *et al*., 2008).

### C. Cripto Binds Near the N-terminus of GRP78

The below data indicates that Cripto binds to the extreme N-terminus of GRP78 since both the N-terminal N-20 antibody blocked binding and a GRP78 deletion mutant lacking the N-20 epitope was unable to bind Cripto. The N-20 antibody has also been shown to competitively block the cellular effects of Kringle 5 (Davidson *et al*., 2005) and T-cadherin (Philippova *et al*., 2008) indicating these proteins bind the N-terminus of GRP78. Furthermore, α2-M and pro-proliferative prostate cancer patient-derived autoantibodies bind to a site that was localized to the N-terminus of GRP78 adjacent to the N-20 epitope (Gonzalez-Gronow *et al*., 2006). The fact that each of these extracellular proteins binds the extreme N-terminus of GRP78 suggests that they may have similar modes of activating GRP78 receptor function and also that they may compete with each other for GRP78 binding.

### D. GRP78 binds the CFC domain of Cripto.

The specificity of the interaction between Cripto and GRP78 was further supported by the demonstration that the CFC domain of Cripto appeared to be both necessary and sufficient for GRP78 binding. This finding is also noteworthy since it suggests that Cripto may bind TGF-β via its EGF-like domain, as the inventors have previously shown (Gray *et al*., 2006), while simultaneously binding GRP78 via its CFC domain. The inventors speculate that larger order protein complexes containing Cripto, GRP78, TGF-β and TGF-β receptors may also form and result in reduced and/or altered TGF-β signaling. In addition, the observation that the CFC domain of Cripto binds GRP78 has further relevance with regard to possible effects of GRP78 on Cripto modulation of signaling by other TGF-β ligands such as Nodal and activins. For example, it has been previously shown that the CFC domain of Cripto specifically binds the activin/Nodal type I receptor ALK4 (Yeo and Whitman, 2001), and GRP78 may therefore compete with ALK4 for Cripto binding. Alternatively, GRP78 may participate in protein complexes containing ALK4, Cripto and activin/Nodal.

### E. Targeted knockdown of Cripto-associated GRP78 inhibits TGF-β signaling.

Multiple lines of evidence have indicate that Cripto and GRP78 each promote tumorigenesis and both proteins are selectively expressed at the cell surface of cancer cells. As described herein, it has now been discovered that these two proteins, both of which have been previously implicated in the promotion of tumor growth, form a complex at the cell surface. This discovery links these proteins physically and also mechanistically via inhibition of TGF-β signaling.

Initially, the role of GRP78 in TGF-β signaling was evaluated by developing an shRNA capable of reducing the levels of GRP78 associated with Cripto at the plasma membrane. This shRNA (SEQ ID NO:5) enhanced TGF-β-dependent Smad2 phosphorylation providing evidence that endogenous GRP78 can restrict TGF-β signaling. To the inventors' knowledge, this finding represents the first demonstration that GRP78 affects TGF-β signaling and, significantly, it constitutes a novel mechanism through which cell surface GRP78 may convey its tumorigenic message. This result also coincides with the previous demonstration that endogenous Cripto has a similar role in these cells (Gray *et al*., 2006) and is consistent with the hypothesis that GRP78 binds Cripto at the cell surface to antagonize growth-inhibitory TGF-β signaling.

### F. GRP78 and Cripto cooperate to attenuate cytostatic TGF-β signaling and enhance proliferation in human prostate carcinoma cells.

The demonstration that GRP78 and Cripto inhibit TGF-β-dependent Smad2 phosphorylation to a greater extent when expressed together than when expressed separately indicates that they function together to inhibit TGF-β signaling. Without wishing to be bound by any theory, since the level of Smad phosphorylation depends directly on the extent of receptor activation, this result suggests that Cripto and GRP78 exert their inhibitory effect by reducing the ability of TGF-β to activate its receptors. Such an interpretation is further supported by the fact that overexpression of Cripto and/or GRP78 in these cells does not alter TGF-β receptor levels. Furthermore, the inability to detect a direct interaction between GRP78 and either type I or type II TGF-β receptors suggests that GRP78 may exert its inhibitory effect on TGF-β signaling by binding Cripto or by directly binding TGF-β or both.

The inventors have further shown that Cripto and GRP78 function cooperatively to enhance cell growth and inhibit the cytostatic effects of TGF-β. When cells were grown under anchorage dependent conditions, Cripto and GRP78 each attenuated the growth inhibitory effects of TGF-β and, interestingly, their co-expression caused TGF-β to switch from being antiproliferative to being pro-proliferative in nature. Although the mechanism underlying this joint effect of Cripto and GRP78 on the proliferative response of cells to TGF-β remains to be determined, TGF-β was shown to increase proliferation/survival under conditions in which its cytostatic effects have been lost (Paradali and Moustakas, 2007). Likewise, the inventors found that Cripto and GRP78 had a cooperative ability to block the cytostatic effects of TGF-β under anchorage independent conditions. However, unlike what was observed in monolayers, TGF-β treatment did not enhance growth of cells co-expressing Cripto and GRP78 in soft agar. Another difference was that GRP78 and Cripto increased colony growth in soft agar in the absence of TGF-β treatment both when expressed separately and, more prominently, when co-expressed.

Therefore, the results indicate that GRP78 and Cripto influence cell growth and TGF-β responsiveness in a manner that varies depending on the specific growth conditions. Distinct signaling pathways may be activated in response to the environment in cells grown under anchorage dependent as opposed to anchorage independent conditions. For example, tumor cells utilize signaling pathways such as FAK and Src to facilitate anchorage independent growth and avoid anoikis (Mitra and Schlaepfer, 2006). Thus, although the specific mechanisms remain to be elucidated, the convergence of signals emanating from TGF-β with signaling pathways specifically associated with a particular growth setting can lead to nuances in growth effects. Despite the differences the inventors observed, however, the inventors have consistently found the effects of Cripto and GRP78 on TGF-β responsiveness to be greater when both proteins were expressed together than when expressed individually. Thus, cell surface Cripto-GRP78 complexes displayed a clear and consistent role in inhibiting cytostatic TGF-β responses under both anchorage dependent and independent growth conditions.

TGF-β is a major tumor suppressor and loss of its cytostatic function is associated with tumor initiation and progression (Pardali and Moustakas, 2007). This loss of growth inhibitory TGF-β signaling may result from reduction of receptor signaling, impaired Smad function or disruption of the transcriptional regulators or their targets that together constitute the cytostatic program (Siegel and Massague, 2003). Indeed, TGF-β signaling frequently exacerbates the growth and spread of tumors that are resistant to its antiproliferative effects (Pardali and Moustakas, 2007). Cripto and GRP78 have each been implicated separately in human cancer progression and each of these proteins is selectively expressed on the surface of tumor cells. Here the inventors have provided evidence that these two proteins physically interact at the cell surface. The inventors have further provided the demonstration that they cooperate to enhance tumor cell growth and reverse the tumor suppressor effects of TGF-β. Without wishing to be bound by any theory, these results support the idea that this complex leads to increased malignancy and that it confers a competitive proliferative advantage to tumor cells via inhibition of TGF-β signaling at the receptor level. In light of these findings, it is anticipated that the cell-surface Cripto-GRP78 complex represents a desirable target with significant therapeutic potential because of its intrinsic selective advantage of affecting only cancer cells but not their normal tissue counterparts.

### G. GRP78/Cripto/TGF-β ligands

The results reveal that GRP78 mediates Cripto co-receptor function and point to a novel and essential role for cell surface GRP78 during embryogenesis and stem cell regulation. Cripto plays critical roles as a co-receptor for Nodal and related TGF-β ligands during embryonic development and genetic studies in zebrafish and mice have shown that Cripto and related EGF-CFC proteins are required for mesoderm and endoderm formation, cardiogenesis, and the establishment of left/right asymmetry. Cripto has also been recognized as a marker of embryonic stem cells and plays important roles in stem cell maintenance and differentiation. ESCs generated from Cripto-null mice are unable to undergo cardiomyogenesis and spontaneously differentiate into neurons. As shown in the below examples, endogenous GRP78 is a necessary mediator of Cripto signaling including Cripto-dependent Nodal signaling. Furthermore, the inventors have provided a demonstration that GRP78 is present at the surface of human ES cells where it co-localized with Cripto. Antibody blockade of GRP78 on hES cells blocked Nodal signaling indicating GRP78 is necessary for Nodal signaling in these cells. These results support that targeting the interface between Cripto and GRP78 may aid in cell based therapies for neurodegenerative diseases that require preferential differentiation of hES cells into neurons.

Several studies also support a role for Cripto modulation of activin/Nodal/TGF-β signaling in promoting the tumor phenotype (Adkins *et al*., 2003; Gray *et al*., 2003; Gray *et al*., 2006; Salomon, 2006; Shani *et al*., 2008; Shen, 2003; Shukla *et al*., 2008; Topczewska *et al*., 2006). Cripto inhibits TGF-β signaling as well as the cytostatic effects of TGF-β on mammary epithelial cells (Gray *et al*., 2006) and primary keratinocytes (Shukla *et al*., 2008). Cripto also inhibited the antiproliferative effect of TGF-β on prostate carcinoma cells, an effect that was enhanced by GRP78 overexpression (Shani *et al*., 2008). Like TGF-β, activin-A inhibits proliferation of most epithelial cells and antagonism of activin/TGF-β signaling has been proposed as an oncogenic Cripto mechanism. By contrast, Nodal has been shown to promote melanoma and breast cancer plasticity and tumorigenicity (Hendrix *et al.,* 2007; Postovit *et al*., 2008; Salomon, 2006; Topczewska *et al*., 2006) and the results suggest that this may require signaling via Cripto and GRP78. In the present study the inventors have shown that targeting cell surface GRP78 on NCCIT cells using shRNA or the N-20 GRP78 blocking antibody abolished Cripto effects on signaling by activins, TGF-β and Nodal. This effect was likely mediated by the interaction between Cripto and GRP78 since knockdown of both proteins together had a much more pronounced effect than knockdown of either protein alone. Importantly, this represents the first demonstration that endogenous Cripto inhibits activin-A and activin-B signaling and confirms the previous demonstration that endogenous Cripto blocks signaling by TGF-β. These findings also support a novel role for GRP78 as a Cripto co-factor that is required for these potentially oncogenic effects of Cripto on activin/Nodal/TGF-β signaling.

### H. GRP78 Mediates Cripto Growth Factor Activity

In addition to its effects on activin/Nodal/TGF-β signaling, Cripto also activates the mitogen activated protein kinase (MAPK) and phosphatidylinositol-3-kinase (PI3K) pathways (De Santis *et al*., 1997; Ebert *et al*., 1999; Strizzi *et al*., 2005). These pathways are aberrantly activated in most human cancers and are widely recognized for their ability to promote multiple tumorigenic outcomes including increased tumor cell survival and proliferation (Dhillon *et al*., 2007; Shaw and Cantley, 2006). It was shown that treatment of HC-11 mammary epithelial cells with soluble Cripto results in tyrosine phosphorylation of the SH2-adaptor protein Shc, association of Shc with Grb2 and activation of the p42/44 Erk/MAPK pathway (Kannan *et al*., 1997). Soluble Cripto also caused phosphorylation of the p85 regulatory subunit of PI3K leading to phosphorylation and activation of Akt in SiHa cervical carcinoma cells (Ebert e*t al*., 1999). Cripto does not bind to members of the EGF receptor family, (Kannan *et al*., 1997) and activation of MAPK/PI3K pathways by soluble Cripto was reported to be ALK4-independent (Bianco *et al*., 2002b). c-Src is activated following treatment of cells with soluble Cripto and c-Src activation is necessary for Cripto-dependent activation of MAPK and PI3K pathways (Bianco *et al*., 2003). In addition, the GPI-anchored proteoglycan glypican-1 was reported to bind Cripto and facilitate Cripto-dependent c-Src activation (Bianco *et al*., 2003). However, a transmembrane Cripto receptor mediating c-Src activation and MAPK/PI3K signaling has not yet been identified. The below data demonstrate that cell surface GRP78 mediates Cripto tumor growth factor activity.

Cripto/GRP78 complexes also influence Akt/Erk signaling. Knockdown or antibody disruption of Cripto/GRP78 complexes in NCCIT cells blocked soluble Cripto-induced phosphorylation of Akt, GSK3b and p42/44.

### I. Cripto/GRP78 Complexes Switch Proliferative Effects of Activin-A and Nodal

Smad2/3 signaling in response to activin, Nodal and TGF-β ligands can have variable and even opposing effects on cellular proliferation, apoptosis and differentiation depending on the cell type and the cellular context. The tumor suppressor function of the Smad2/3 pathway has been well characterized and derives from its ability to inhibit cellular proliferation of multiple cell types and in some cases to cause terminal differentiation or apoptosis. It is now well-established that the cytostatic transcriptional program downstream of Smad2/3 signaling is critical for normal tissue homeostasis and tumor suppression and disruptions or alterations in this pathway have been observed in several types of human cancer including breast cancer. However, Activin/Nodal/TGF-β ligands can also exacerbate the tumor phenotype under conditions in which tumor cells have become refractory to the antiproliferative effects of the Smad2/3 pathway. Tumor cells generally secrete high levels of these ligands that act on tumor cells and other cell types within the tumor microenvironment including stromal fibroblasts, endothelial cells and immune cells. Activation of the Smad2/3 pathway in this context can cause increased proliferation, motility, invasion and epithelial to mesenchymal transition (EMT) of tumor cells as well as increased angiogenesis and decreased immune surveillance. Collectively, these effects can lead to increased tumor growth and metastasis and have led to therapeutic efforts aimed at blocking TGF-β signaling in human cancers.

The inventors have also shown that activin-A has opposing effects on cellular proliferation depending on the presence or absence of cell surface Cripto/GRP78 complexes. Activin-A had pro-proliferative effects on empty-vector infected NCCIT cells and Cripto-overexpressing MCF10A cells in which Cripto/GRP78 complexes were intact. By contrast, activin-A had antiproliferative effects when Cripto/GRP78 complexes in these cells were disrupted by knockdown or N-20 antibody blockade. Like Activin-A, Nodal increased proliferation in these cells when they expressed intact Cripto/GRP78 complexes. Unlike activin-A, however, Nodal had no effect on the proliferation of cells in which Cripto/GRP78 complexes were disrupted. This difference between activin-A and Nodal likely reflects the fact that Cripto is required for Nodal signaling but not for activin-A signaling. These data indicate that Cripto/GRP78 complexes on the cell surface can promote tumor growth by facilitating mitogenic effects of Nodal and causing activin-A to switch from being cytostatic to pro-proliferative in nature. Therefore, Cripto causes cellular responses to switch from being epithelial-cytostatic to being mesenchymal-pro-proliferativeand this resembles what is seen when tumor cells become resistant to cytostatic effects of Smad2/3. These results suggest a mechanism by which activin and Nodal to become pro-tumorigenic, and this effect appears to be GRP78 dependent. Interestingly, the inventors found that activin-A and Nodal are mitogenic in the presence of Cripto/GRP78 complexes whereas activin-A had cytostatic effects and Nodal had no effect on proliferation in the absence of these complexes. Therefore, cell surface Cripto/GRP78 complexes may promote crosstalk between MAPK/PI3K and Smad2/3 pathways that cause cytostatic Smad2/3 signaling to become pro-proliferative in nature. The cell surface Cripto/GRP78 complex thus functions as a cell signaling node to regulate multiple tumor and stem cell behaviors.

The biological relevance of the interaction between Cripto and GRP78 is supported by the fact that Cripto and GRP78 have overlapping distributions and functions. Cripto (Ding *et al*., 1998) and GRP78 (Luo *et al*., 2006) knockout mice are both early embryonic lethal. Cripto can also regulate embryonic stem cell behavior (Minchiotti, 2005) and the inventors have shown here that Cripto and GRP78 co-localize in stem cells and cooperatively regulate activin and Nodal signaling. In this regard, GRP78 was found to be required for proliferation and survival of embryonic inner cell mass cells that are the precursors of pluripotent stem cells (Luo *et al*., 2006). In addition, Cripto (Xu *et al*., 1998; Xu *et al.,* 1999) and GRP78 (Mao *et al*., 2006) are prominently expressed in the developing heart and they have each been implicated in cardiogenesis. Finally, like Cripto (Strizzi *et al*., 2005), GRP78 increases malignancy and provides a competitive growth advantage to tumor cells by increasing tumor cell survival, proliferation and angiogenesis (Dong *et al*., 2008; Lee, 2007). Importantly, Cripto and GRP78 are both selectively expressed at the plasma membrane of human tumor cells but not their normal tissue counterpartsm and they have each been independently validated as tumor-specific therapeutic targets *in vivo.* Therefore, the Cripto/GRP78 complex represents a novel and desirable target on with significant therapeutic potential.

### IV. INHIBITORS OF THE CRIPTO/GRP78 INTERACTION

The Cripto/GRP78 interaction may be selectively inhibited via a Cripto-targeting compound and/or a GRP78-targeting compound which inhibits Cripto/GRP78 complex formation and/or function. In certain embodiments, the Cripto-targeting or GRP78-targeting compound may be an antibody, a bi-functional antibody, an aptamer, an antibody fragment such as a f(ab) or f(ab)₂ region, an inhibitory peptide, a small molecule, an antisense molecule, or an siNA (*e.g*., a siRNA or a shRNA). These compounds may be produced by one of skill using screens which test for alterations in Cripto/GRP78 binding and/or downstream signaling by a candidate compound. In certain embodiments, a Cripto-targeting compound may specifically affect or bind the CFC domain of Cripto and inbhibit Cripto/GRP78 binding and/or signaling. In other embodiments, a GRP78-targeting compound may bind to or interact with an N-terminal region or extreme N-terminal region of GRP78, such as a a N-20 antibody epitope of GRP78.

### A. Antibodies

Certain aspects of the invention relate to one or more antibodies which selectively bind Cripto and/or GRP78. These antibodies may be used to treat a cancer (*e.g*., a melanoma, a liver cancer, a colorectal cancer, a pancreatic cancer, a lung cancer, NSCLC, a head or neck cancer). Further, these antibodies may be used to evaluate expression of Cripto and/or GRP78 in a tissue, such as a cancerous or prccanccrous tissue. In certain embodiments, a N-20 antibody or an antibody which binds an N-20 epitope of GRP78 may be used to target GRP78 and disrupt the formation of Cripto/GRP78 complexes.

As disclosed herein, it may be desirable to make antibodies against the identified targeting peptides or their receptors. The appropriate targeting peptide or receptor, or portions thereof, may be coupled, bonded, bound, conjugated, or chemically-linked to one or more agents via linkers, polylinkers, or derivatized amino acids. This may be performed such that a bispecific or multivalent composition or vaccine is produced. It is further envisioned that the methods used in the preparation of these compositions are familiar to those of skill in the art and should be suitable for administration to humans, *i.e*., pharmaceutically acceptable. Preferred agents are the carriers are keyhole limpet hemocyanin (KLH) or bovine serum albumin (BSA).

The term "antibody" is used to refer to any antibody-like molecule that has an antigen binding region, and includes antibody fragments such as Fab', Fab, F(ab')₂, single domain antibodies (DABs), Fv, scFv (single chain Fv), and the like. Techniques for preparing and using various antibody-based constructs and fragments are well known in the art. Means for preparing and characterizing antibodies are also well known in the art (See, e.g., Harlow and Lane, 1988; incorporated herein by reference).

As disclosed herein, circulating antibodies from one or more individuals with a disease state may be obtained and screened against phage display libraries. Targeting peptides that bind to the circulating antibodies may act as mimeotopes of a native antigen, such as a receptor protein located on an endothelial cell surface of a target tissue. For example, circulating antibodies in an individual with prostate cancer may bind to antigens specifically or selectively localized in prostate tumors. As discussed in more detail below, targeting peptides against such antibodies may be identified by phage display. Such targeting peptides may be used to identify the native antigen recognized by the antibodies, for example by using known techniques such as immunoaffinity purification, Western blotting, electrophoresis followed by band excision and protein/peptide sequencing and/or computerized homology searches. The skilled artisan will realize that antibodies against disease specific or selective antigens may be of use for various applications, such as detection, diagnosis and/or prognosis of a disease state, imaging of diseased tissues and/or targeted delivery of therapeutic agents.

In certain embodiments, the Cripto and/or GRP78 antibody is a monoclonal antibody. Monoclonal antibodies (MAbs) are recognized to have certain advantages, *e.g*., reproducibility and large-scale production, and their use is generally preferred. The invention thus provides monoclonal antibodies of the human, murine, monkey, rat, hamster, rabbit and even chicken origin. Due to the ease of preparation and ready availability of reagents, murine monoclonal antibodies will often be preferred.

"Humanized" antibodies are specifically contemplated in the present invention, as are chimeric antibodies from mouse, rat, or other species, bearing human constant and/or variable region domains, bispecific antibodies, recombinant and engineered antibodies and fragments thereof. Methods for the development of antibodies that are "custom-tailored" to the patient's disease are likewise known and such custom-tailored antibodies are also contemplated.

### 1. Methods for Antibody Production

Cripto- and/or GRP78-selective antibodies may be prepared using techniques well known in the art. For example, the methods for generating monoclonal antibodies (MAbs) generally begin along the same lines as those for preparing polyclonal antibodies. Briefly, a polyclonal antibody is prepared by immunizing an animal with a LEE or CEE composition in accordance with the present invention and collecting antisera from that immunized animal.

A wide range of animal species can be used for the production of antisera. Typically the animal used for production of antisera is a rabbit, a mouse, a rat, a hamster, a guinea pig or a goat. The choice of animal may be decided upon the ease of manipulation, costs or the desired amount of sera, as would be known to one of skill in the art.

In order to generate a more vigorous immune response and aid in the production of antisera, the immunogenicity of a particular immunogen composition can be enhanced by the use of non-specific stimulators of the immune response, known as adjuvants. Suitable adjuvants include all acceptable immunostimulatory compounds, such as cytokines, chemokines, cofactors, toxins, plasmodia, synthetic compositions or LEEs or CEEs encoding such adjuvants.

Adjuvants that may be used include IL-1, IL-2, IL-4, IL-7, IL-12, γ-interferon, GMCSP, BCG, aluminum hydroxide, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion is also contemplated. MHC antigens may even be used. Exemplary, often preferred adjuvants include complete Freund's adjuvant (a non-specific stimulator of the immune response containing killed *Mycobacterium tuberculosis)*, incomplete Freund's adjuvants and aluminum hydroxide adjuvant.

In addition to adjuvants, it may be desirable to coadminister biologic response modifiers (BRM), which have been shown to upregulate T cell immunity or downregulate suppressor cell activity. Such BRMs include, but are not limited to, Cimetidine (CIM; 1200 mg/d) (Smith/Kline, PA); low-dose Cyclophosphamide (CYP; 300 mg/m²) (Johnson/ Mead, NJ), cytokines such as γ-interferon, IL-2, or IL-12 or genes encoding proteins involved in immune helper functions, such as B-7.

The amount of immunogen composition used in the production of polyclonal antibodies varies upon the nature of the immunogen as well as the animal used for immunization. A variety of routes can be used to administer the immunogen including but not limited to subcutaneous, intramuscular, intradermal, intraepidermal, intravenous and intraperitoneal. The production of polyclonal antibodies may be monitored by sampling blood of the immunized animal at various points following immunization.

A second, booster dose (*e.g*., provided in an injection), may also be given. The process of boosting and titering is repeated until a suitable titer is achieved. When a desired level of immunogenicity is obtained, the immunized animal can be bled and the serum isolated and stored, and/or the animal can be used to generate MAbs.

For production of rabbit polyclonal antibodies, the animal can be bled through an ear vein or alternatively by cardiac puncture. The removed blood is allowed to coagulate and then centrifuged to separate serum components from whole cells and blood clots. The serum may be used as is for various applications or else the desired antibody fraction may be purified by well-known methods, such as affinity chromatography using another antibody, a peptide bound to a solid matrix, or by using, *e.g*., protein A or protein G chromato graphy.

MAbs may be readily prepared through use of well-known techniques, such as those exemplified in U.S. Patent 4,196,265. Typically, this technique involves immunizing a suitable animal with a selected immunogen composition, *e.g*., a purified or partially purified protein, polypeptide, peptide or domain, be it a wild-type or mutant composition. The immunizing composition is administered in a manner effective to stimulate antibody producing cells.

The methods for generating monoclonal antibodies (MAbs) generally begin along the same lines as those for preparing polyclonal antibodies. Rodents such as mice and rats are preferred animals, however, the use of rabbit, sheep or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

The animals are injected with antigen, generally as described above. The antigen may be mixed with adjuvant, such as Freund's complete or incomplete adjuvant. Booster administrations with the same antigen or DNA encoding the antigen would occur at approximately two-week intervals.

Following immunization, somatic cells with the potential for producing antibodies, specifically B lymphocytes (B cells), are selected for use in the MAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible.

Often, a panel of animals will have been immunized and the spleen of an animal with the highest antibody titer will be removed and the spleen lymphocytes obtained by homogenizing the spleen with a syringe. Typically, a spleen from an immunized mouse contains approximately 5 × 10⁷ to 2 × 10⁸ lymphocytes.

The antibody-producing B lymphocytes from the immunized animal may then be fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art (Goding, pp. 65-66, 1986; Campbell, pp. 75-83, 1984). cites). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al*., (1977). The use of electrically induced fusion methods is also appropriate (Goding pp. 71-74, 1986).

Fusion procedures usually produce viable hybrids at low frequencies, about 1 × 10⁻⁶ to 1 × 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e.g*., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide MAbs. The cell lines may be exploited for MAb production in two basic ways. First, a sample of the hybridoma can be injected (often into the peritoneal cavity) into a histocompatible animal of the type that was used to provide the somatic and myeloma cells for the original fusion (*e.g*., a syngeneic mouse). Optionally, the animals are primed with a hydrocarbon, especially oils such as pristane (tetramethylpentadecane) prior to injection. The injected animal develops tumors secreting the specific monoclonal antibody produced by the fused cell hybrid. The body fluids of the animal, such as serum or ascites fluid, can then be tapped to provide MAbs in high concentration. Second, the individual cell lines could be cultured *in vitro,* where the MAbs are naturally secreted into the culture medium from which they can be readily obtained in high concentrations.

MAbs produced by either means may be further purified, if desired, using filtration, centrifugation and various chromatographic methods such as HPLC or affinity chromatography. Fragments of the monoclonal antibodies of the invention can be obtained from the monoclonal antibodies so produced by methods which include digestion with enzymes, such as pepsin or papain, and/or by cleavage of disulfide bonds by chemical reduction. Alternatively, monoclonal antibody fragments encompassed by the present invention can be synthesized using an automated peptide synthesizer.

It is also contemplated that a molecular cloning approach may be used to generate monoclonals. In one embodiment, combinatorial immunoglobulin phagemid libraries are prepared from RNA isolated from the spleen of the immunized animal, and phagemids expressing appropriate antibodies are selected by panning using cells expressing the antigen and control cells. The advantages of this approach over conventional hybridoma techniques are that approximately 10⁴ times as many antibodies can be produced and screened in a single round, and that new specificities are generated by H and L chain combination which further increases the chance of finding appropriate antibodies. In another example, LEEs or CEEs can be used to produce antigens in vitro with a cell free system. These can be used as targets for scanning single chain antibody libraries. This would enable many different antibodies to be identified very quickly without the use of animals.

Alternatively, monoclonal antibody fragments encompassed by the present invention can be synthesized using an automated peptide synthesizer, or by expression of full-length gene or of gene fragments in *E. coli.*

Monoclonal fully human antibodies may be produced using transgenic animals, such as XenoMouse which includes germline-configured, megabase-sized YACs carrying portions of the human IgH and Igkappa loci, including the majority of the variable region repertoire, the genes for Cmicro, Cdelta and either Cgamma1, Cgamma2, or Cgamma4, as well as the cis elements required for their function (Green, 1999). The IgH and Igkappa transgenes were bred onto a genetic background deficient in production of murine immunoglobulin. The large and complex human variable region repertoire encoded on the Ig transgenes in XenoMouse strains support the development of large peripheral B cell compartments and the generation of a diverse primary immune repertoire similar to that from adult humans. Immunization of XenoMouse mice with human antigens routinely results in a robust secondary immune response, which can ultimately be captured as a large panel of antigen-specific fully human IgGkappa mAbs of sub-nanomolar affinities. Monoclonal antibodies from XenoMouse animals have been shown to have therapeutic potential both in vitro and *in vivo,* and appear to have the pharmacokinetics of normal human antibodies based on human clinical trials.

### 2. Antibody Conjugates

The present invention further provides antibodies that selectively bind Cripto or GRP78, generally of the monoclonal type, that are linked to at least one agent to form an antibody conjugate. In order to increase the efficacy of antibody molecules as diagnostic or therapeutic agents, the antibody may be covalently bound or complexed to at least one desired molecule or moiety. Such a molecule or moiety may be, but is not limited to, at least one effector or reporter molecule. Effector molecules comprise molecules having a desired activity, *e.g*., cytotoxic activity. Non-limiting examples of effector molecules which have been attached to antibodies include toxins, anti-tumor agents, therapeutic enzymes, radio-labeled nucleotides, antiviral agents, chelating agents, cytokines, growth factors, and oligo- or poly-nucleotides. By contrast, a reporter molecule is defined as any moiety which may be detected using an assay. Non-limiting examples of reporter molecules which have been conjugated to antibodies include enzymes, radiolabels, haptens, fluorescent labels, phosphorescent molecules, chemiluminescent molecules, chromophores, luminescent molecules, photoaffinity molecules, colored particles or ligands, such as biotin.

An Cripto and/or GRP78 antibody may be employed as the basis for an antibody conjugate. Sites for binding to biological active molecules in the antibody molecule, in addition to antigen binding sites, include sites that reside in the variable domain that can bind pathogens, B-cell superantigens, the T cell co-receptor CD4 and the HIV-1 envelope (Sasso *et al*., 1989; Shorki *et al*., 1991; Silvermann *et al*., 1995; Cleary *et al*., 1994; Lenert *et al*., 1990; Berberian *et al*., 1993; Kreier *et al*., 1991). In addition, the variable domain is involved in antibody self-binding (Kang *et al*., 1988), and contains epitopes (idiotopes) recognized by anti-antibodies (Kohler *et al*., 1989).

Certain examples of antibody conjugates are those conjugates in which the antibody is linked to a detectable label. "Detectable labels" are compounds and/or elements that can be detected due to their specific functional properties, and/or chemical characteristics, the use of which allows the antibody to which they are attached to be detected, and/or further quantified if desired. Another such example is the formation of a conjugate comprising an antibody linked to a cytotoxic or anti-cellular agent, and may be termed "immunotoxins."

Antibody conjugates are generally preferred for use as diagnostic agents. Antibody diagnostics generally fall within two classes, those for use in *in vitro* diagnostics, such as in a variety of immunoassays, and/or those for use *in vivo* diagnostic protocols, generally known as "antibody-directed imaging".

Many appropriate imaging agents are known in the art, as are methods for their attachment to antibodies (see, for *e.g*., U.S. Patents 5,021,236, 4,938,948, and 4,472,509). The imaging moieties used can be paramagnetic ions; radioactive isotopes; fluorochromes; NMR-detectable substances; X-ray imaging.

In the case of paramagnetic ions, one might mention by way of example ions such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and/or erbium (III), with gadolinium being particularly preferred. Ions useful in other contexts, such as X-ray imaging, include but are not limited to lanthanum (III), gold (III), lead (II), and especially bismuth (III).

Radioactive isotopes for therapeutic and/or diagnostic application include astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodine¹²⁵, iodine¹³¹, indium¹¹¹, ⁵⁹iron, ³²phosphorus, rhenium¹⁸⁶, rhenium¹⁸⁸, ⁷⁵selenium, ³⁵sulphur, technicium^{99m} and/or yttrium⁹⁰. ¹²⁵I may be preferred for use in certain embodiments, and technicium^{99m} and/or indium¹¹¹ are also often preferred due to their low energy and suitability for long range detection. Radioactively labeled monoclonal antibodies of the present invention may be produced according to well-known methods in the art. For instance, monoclonal antibodies can be iodinated by contact with sodium and/or potassium iodide and a chemical oxidizing agent such as sodium hypochlorite, or an enzymatic oxidizing agent, such as lactoperoxidase. Monoclonal antibodies according to the invention may be labeled with technetium^{99m} by ligand exchange process, for example, by reducing pertechnate with stannous solution, chelating the reduced technetium onto a Sephadex column and applying the antibody to this column. Alternatively, direct labeling techniques may be used, *e.g*., by incubating pertechnate, a reducing agent such as SNCl₂, a buffer solution such as sodium-potassium phthalate solution, and the antibody. Intermediary functional groups which are often used to bind radioisotopes which exist as metallic ions to antibody are diethylenetriaminepentaacetic acid (DTPA) or ethylene diaminetetracetic acid (EDTA).

Among the fluorescent labels contemplated for use as conjugates include Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5,6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, TAMRA, TET, Tetramethylrhodamine, and/or Texas Red.

Another type of antibody conjugates contemplated in the present invention are those intended primarily for use *in vitro,* where the antibody is linked to a secondary binding ligand and/or to an enzyme (an enzyme tag) that will generate a colored product upon contact with a chromogenic substrate. Examples of suitable enzymes include urease, alkaline phosphatase, (horseradish) hydrogen peroxidase or glucose oxidase. Preferred secondary binding ligands are biotin and/or avidin and streptavidin compounds. The use of such labels is well known to those of skill in the art and are described, for example, in U.S. Patents 3,817,837; 3,850,752; 3,939,350; 3,996,345; 4,277,437; 4,275,149 and 4,366,241.

Yet another known method of site-specific attachment of molecules to antibodies comprises the reaction of antibodies with hapten-based affinity labels. Essentially, hapten-based affinity labels react with amino acids in the antigen binding site, thereby destroying this site and blocking specific antigen reaction. However, this may not be advantageous since it results in loss of antigen binding by the antibody conjugate.

Molecules containing azido groups may also be used to form covalent bonds to proteins through reactive nitrene intermediates that are generated by low intensity ultraviolet light (Potter & Haley, 1983). In particular, 2- and 8-azido analogues of purine nucleotides have been used as site-directed photoprobes to identify nucleotide binding proteins in crude cell extracts (Owens & Haley, 1987; Atherton *et al.,* 1985). The 2- and 8-azido nucleotides have also been used to map nucleotide binding domains of purified proteins (Khatoon *et al*., 1989; King *et al*., 1989; and Dholakia *et al*., 1989) and may be used as antibody binding agents.

Several methods are known in the art for the attachment or conjugation of an antibody to its conjugate moiety. Some attachment methods involve the use of a metal chelate complex employing, for example, an organic chelating agent such a diethylenetriaminepentaacetic acid anhydride (DTPA); ethylenetriaminetetraacetic acid; N-chloro-p-toluenesulfonamide; and/or tetrachloro-3α-6α-diphenylglycouril-3 attached to the antibody (U.S. Patents 4,472,509 and 4,938,948).

Monoclonal antibodies may also be reacted with an enzyme in the presence of a coupling agent such as glutaraldehyde or periodate. Conjugates with fluorescein markers are prepared in the presence of these coupling agents or by reaction with an isothiocyanate. In U.S. Patent 4,938,948, imaging of breast tumors is achieved using monoclonal antibodies and the detectable imaging moieties are bound to the antibody using linkers such as methyl-p-hydroxybenzimidate or N-succinimidyl-3-(4-hydroxyphenyl)propionate.

In other embodiments, derivatization of immunoglobulins by selectively introducing sulfhydryl groups in the Fc region of an immunoglobulin, using reaction conditions that do not alter the antibody combining site are contemplated. Antibody conjugates produced according to this methodology are disclosed to exhibit improved longevity, specificity and sensitivity (U.S. Pat. No. 5,196,066).

Site-specific attachment of effector or reporter molecules, wherein the reporter or effector molecule is conjugated to a carbohydrate residue in the Fc region have also been disclosed in the literature (O'Shannessy *et al*., 1987). This approach has been reported to produce diagnostically and therapeutically promising antibodies which are currently in clinical evaluation.

### 3. Bi-functional Antibodies

In certain embodiments, a bi-functional antibody may be used to target Cripto and/or GRP78. For example, a bispecific Fc-dimer may be used to target and bind both Cripto and GRP78, and it is envisioned that this binding could inhibit subsequent signaling by the Cripto/GRP78 complex, such as Nodal signaling, activins/TGFβ signaling, ERK/MAPK signaling, and/or PI3K/Akt/GSK3β signaling. In certain embodiments, the bi-functional or bi-specific antibody may bind at least a portion of the EGF-like region of Cripto, the CFC domain of Cripto, amino acids 19-68 of GRP78, and/or the N-20 region of GRP78. Alternatively, a diabody, triabody or tetrabody containing multiple scFv molecules may be used to bind Cripto and/or GRP78 with, *e.g*., an increased affinity.

Linkers of varying length between V-domains in bispecific antibodies may be used to direct the formation of either diabodies (*e.g*., about 60 kDa), triabodies (*e.g*., about 90 kDa) or tetrabodies (*e.g*., about 120 kDa), as desired to optimize size, flexibility and valency, depending on the particular application desired, *e.g*., *in vivo* or *in vitro* imaging or therapy (Robinson *et al.* 2008; Todorovska *et al*.,2001)*.* Multi-functional antibodies can display increased binding valency of these scFv multimers, resulting in high avidity and decreased off-rates. In some embodiments, multi-functional antibodies may be advantageously used for tumour targeting, since certain molecules of about 60-100 kDa can display increased tumour penetration and fast clearance rates compared to the parent Ig (*e.g*., about 150 kDa).

In certain embodiments, multi-specific Fv modules are desiged to crosslink two or more different target antigens. These bi- and tri-specific multimers can be formed by association of different scFv molecules (Dutertre and Teillaud, 2006; Plückthun *et al.,* 1997; Kortt *et al*., 2001; Hudson *et al.*, 1999; Atwell *et al*., 1996).

### B. Cripto/GRP78-Targeting Peptides

A Cripto-targeting or GRP78-targeting protein or peptide may be used to inhibit the formation and/or function of Cripto/GRP78 complexes. For example, a library of peptides may be screened, e.g., using phage display in cells *in vitro,* to identify peptides or proteins which can bind Cripto and/or GRP78 to inhibit Cripto/GRP78 complex formation. Various methods may be used for this purpose including, *e.g.,* those described in Mintz PJ et al. Nat Biotechnol 2003 21(1) 57-63; Kim Y et al. Biochemistry 45(31) 9434-44; Jakobsen CG et al. Cancer Res 2007 67(19) 9507-17; and Gonzalez-Gronow M et al. Cancer Res 2006 66(23) 11424-31. As used herein, a protein or peptide generally refers, but is not limited to, a protein of greater than about 200 amino acids up to a full length sequence translated from a gene; a polypeptide of about 100 to 200 amino acids; and/or a peptide of from about 3 to about 100 amino acids. For convenience, the terms "protein," "polypeptide" and "peptide are used interchangeably herein.

As disclosed herein, a peptide comprising a N-20 epitope of GRP78 may be used to bind Cripto and inhibit Cripto/GRP78 complex formation. As shown herein, the N-20 epitope of GRP78 is critical for Cripto/GRP78 binding; thus, a peptide comprising a N-20 epitope of GRP78 could be used to competitively antagonize Cripto/GRP78 complex formation. The N-20 epitope consists of 20 amino acid residues within the first 50 residues downstream from the GRP78 signal peptide. The N-20 antibody can be purchased from Santa Cruz Biotechnology (CA, USA). Similarly, in other embodiments, a peptide comprising a CFC domain of Cripto may be used to bind GRP78 and inhibit Cripto/GRP78 complex formation. As shown in the below examples, the CFC domain of Cripto is critical for Cripto/GRP78 binding; thus, a peptide comprising a CFC domain of Cripto could be used to competitively antagonize Cripto/GRP78 complex formation.

Binding assays using ¹²⁵I-labeled soluble Cripto, such as those described in Kelber *et al*., may be used with the present invention. For example, Cripto binding to intact MCF10A or NCCIT cells can be competitively blocked by unlabeled Cripto and, importantly, by the N-20 GRP78 antibody. This assay or a modified version of it could be used to screen for peptides capable of disrupting Cripto/GRP78 binding.

As disclosed herein, the size of at least one protein or peptide may comprise, but is not limited to, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, about 110, about 120, about 130, about 140, about 150, about 160, about 170, about 180, about 190, about 200, about 210, about 220, about 230, about 240, about 250, about 275, about 300, about 325, about 350, about 375, about 400, about 425, about 450, about 475, about 500, about 525, about 550, about 575, about 600, about 625, about 650, about 675, about 700, about 725, about 750, about 775, about 800, about 825, about 850, about 875, about 900, about 925, about 950, about 975, about 1000, about 1100, about 1200, about 1300, about 1400, about 1500, about 1750, about 2000, about 2250, about 2500 or greater amino acid residues.

As used herein, an "amino acid residue" refers to any naturally occurring amino acid, any amino acid derivative or any amino acid mimic known in the art. As disclosed herein, the residues of the protein or peptide are sequential, without any non-amino acid interrupting the sequence of amino acid residues. As disclosed herein, the sequence may comprise one or more non-amino acid moieties. As disclosed herein, the sequence of residues of the protein or peptide may be interrupted by one or more non-amino acid moieties.

Accordingly, the term "protein or peptide" encompasses amino acid sequences comprising at least one of the 20 common amino acids found in naturally occurring proteins, or at least one modified or unusual amino acid, including but not limited to those shown on Table 2 below.

| **TABLE 2** | | | |
|---|---|---|---|
| **Modified and Unusual Amino Acids** | | | |
| Abbr. | Amino Acid | Abbr. | Amino Acid |
| Aad | 2-Aminoadipic acid | EtAsn | N-Ethylasparagine |
| Baad | 3- Aminoadipic acid | Hyl | Hydroxylysine |
| Bala | β-alanine, β-Amino-propionic acid | AHyl | allo-Hydroxylysine |
| Abu | 2-Aminobutyric acid | 3Hyp | 3-Hydroxyproline |
| 4Abu | 4- Aminobutyric acid, piperidinic acid | 4Hyp | 4-Hydroxyproline |
| Acp | 6-Aminocaproic acid | Ide | Isodesmosine |
| Ahe | 2-Aminoheptanoic acid | AIle | allo-Isoleucine |
| Aib | 2-Aminoisobutyric acid | MeGly | N-Methylglycine, sarcosine |
| Baib | 3-Aminoisobutyric acid | MeIle | N-Methylisoleucine |
| Apm | 2-Aminopimelic acid | MeLys | 6-N-Methyllysine |
| Dbu | 2,4-Diaminobutyric acid | MeVal | N-Methylvaline |
| Des | Desmosine | Nva | Norvaline |
| Dpm | 2,2'-Diaminopimelic acid | Nle | Norleucine |
| Dpr | 2,3-Diaminopropionic acid | Orn | Ornithine |
| EtGly | N-Ethylglycine | | |

Proteins or peptides may be made by any technique known to those of skill in the art, including the expression of proteins, polypeptides, or peptides through standard molecular biological techniques, the isolation of proteins or peptides from natural sources, or the chemical synthesis of proteins or peptides. The nucleotide and protein, polypeptide and peptide sequences corresponding to various genes have been previously disclosed, and may be found at computerized databases known to those of ordinary skill in the art. One such database is the National Center for Biotechnology Information's Genbank and GenPept databases (www.ncbi.nlm.nih.gov/). The coding regions for known genes may be amplified and/or expressed using the techniques disclosed herein or as would be know to those of ordinary skill in the art. Alternatively, various commercial preparations of proteins, polypeptides and peptides are known to those of skill in the art.

### 1. Peptide mimetics

For the preparation of polypeptides according to the invention the use of peptide mimetics is also disclosed. Mimetics are peptide-containing molecules that mimic elements of protein secondary structure. See, for example, Johnson *et al*., (1993), incorporated herein by reference. The underlying rationale behind the use of peptide mimetics is that the peptide backbone of proteins exists chiefly to orient amino acid side chains in such a way as to facilitate molecular interactions, such as those of antibody and antigen. A peptide mimetic is expected to permit molecular interactions similar to the natural molecule. These principles may be used to engineer second generation molecules having many of the natural properties of the targeting peptides disclosed herein, but with altered and even improved characteristics.

### 2. Fusion proteins

Also disclosed are fusion proteins. These molecules generally have all or a substantial portion of a targeting peptide, linked at the N- or C-terminus, to all or a portion of a second polypeptide or protein. For example, fusions may employ leader sequences from other species to permit the recombinant expression of a protein in a heterologous host. Another useful fusion includes the addition of an immunologically active domain, such as an antibody epitope, to facilitate purification of the fusion protein. Inclusion of a cleavage site at or near the fusion junction will facilitate removal of the extraneous polypeptide after purification. Other useful fusions include linking of functional domains, such as active sites from enzymes, glycosylation domains, cellular targeting signals or transmembrane regions. As disclosed herein, the fusion proteins as disclosed herein comprise a targeting peptide linked to a therapeutic protein or peptide. Examples of proteins or peptides that may be incorporated into a fusion protein include cytostatic proteins, cytocidal proteins, pro-apoptosis agents, anti-angiogenic agents, hormones, cytokines, growth factors, peptide drugs, antibodies, Fab fragments antibodies, antigens, receptor proteins, enzymes, lectins, MHC proteins, cell adhesion proteins and binding proteins. These examples are not meant to be limiting and it is contemplated that within the scope of the present disclosure virtually and protein or peptide could be incorporated into a fusion protein comprising a targeting peptide. Methods of generating fusion proteins are well known to those of skill in the art. Such proteins can be produced, for example, by chemical attachment using bifunctional cross-linking reagents, by *de novo* synthesis of the complete fusion protein, or by attachment of a DNA sequence encoding the targeting peptide to a DNA sequence encoding the second peptide or protein, followed by expression of the intact fusion protein.

### 3. Protein purification

As disclosed herein, a protein or peptide may be isolated or purified. As disclosed herein, these proteins may be used to generate antibodies for tagging with any of the illustrated barcodes (e.g. polymeric Raman label). Protein purification techniques are well known to those of skill in the art. These techniques involve, at one level, the homogenization and crude fractionation of the cells, tissue or organ to polypeptide and non-polypeptide fractions. The protein or polypeptide of interest may be further purified using chromatographic and electrophoretic techniques to achieve partial or complete purification (or purification to homogeneity). Analytical methods particularly suited to the preparation of a pure peptide are ion-exchange chromatography, gel exclusion chromatography, HPLC (high performance liquid chromatography) FPLC (AP Biotech), polyacrylamide gel electrophoresis, affinity chromatography, immunoaffinity chromatography and isoelectric focusing. An example of receptor protein purification by affinity chromatography is disclosed in U.S. Patent No. 5,206,347. One of the more efficient methods of purifying peptides is fast performance liquid chromatography (AKTA FPLC) or even A purified protein or peptide is intended to refer to a composition, isolatable from other components, wherein the protein or peptide is purified to any degree relative to its naturally-obtainable state. An isolated or purified protein or peptide, therefore, also refers to a protein or peptide free from the environment in which it may naturally occur. Generally, "purified" will refer to a protein or peptide composition that has been subjected to fractionation to remove various other components, and which composition substantially retains its expressed biological activity. Where the term "substantially purified" is used, this designation will refer to a composition in which the protein or peptide forms the major component of the composition, such as constituting about 50%, about 60%, about 70%, about 80%, about 90%, about 95%, or more of the proteins in the composition.

Various methods for quantifying the degree of purification of the protein or peptide are known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity therein, assessed by a "-fold purification number." The actual units used to represent the amount of activity will, of course, be dependent upon the particular assay technique chosen to follow the purification, and whether or not the expressed protein or peptide exhibits a detectable activity.

Various techniques suitable for use in protein purification are well known to those of skill in the art. These include, for example, precipitation with ammonium sulphate, PEG, antibodies and the like, or by heat denaturation, followed by: centrifugation; chromatography steps such as ion exchange, gel filtration, reverse phase, hydroxylapatite and affinity chromatography; isoelectric focusing; gel electrophoresis; and combinations of these and other techniques. As is generally known in the art, it is believed that the order of conducting the various purification steps may be changed, or that certain steps may be omitted, and still result in a suitable method for the preparation of a substantially purified protein or peptide.

There is no general requirement that the protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater "-fold" purification than the same technique utilizing a low pressure chromatography system. Methods exhibiting a lower degree of relative purification may have advantages in total recovery of protein product, or in maintaining the activity of an expressed protein.

Affinity chromatography is a chromatographic procedure that relies on the specific affinity between a substance to be isolated and a molecule to which it can specifically bind. This is a receptor-ligand type of interaction. The column material is synthesized by covalently coupling one of the binding partners to an insoluble matrix. The column material is then able to specifically adsorb the substance from the solution. Elution occurs by changing the conditions to those in which binding will not occur (*e.g*., altered pH, ionic strength, temperature, *etc*.). The matrix should be a substance that itself does not adsorb molecules to any significant extent and that has a broad range of chemical, physical and thermal stability. The ligand should be coupled in such a way as to not affect its binding properties. The ligand should also provide relatively tight binding. And it should be possible to elute the substance without destroying the sample or the ligand.

### 4. Synthetic Peptides

Because of their relatively small size, the targeting peptides as disclosed herein can be synthesized in solution or on a solid support in accordance with conventional techniques. Various automatic synthesizers are commercially available and can be used in accordance with known protocols. See, for example, Stewart and Young, 1984; Tam *et al*., 1983; Merrifield, 1986; and Barany and Merrifield, 1979.

Short peptide sequences, usually from about 6 up to about 35 to 50 amino acids, can be readily synthesized by such methods. Alternatively, recombinant DNA technology may be employed wherein a nucleotide sequence which encodes a peptide of the invention is inserted into an expression vector, transformed or transfected into an appropriate host cell, and cultivated under conditions suitable for expression.

### C. Short Interfering Nucleic Acids

There are disclosed short interfering nucleic acids (e.g., siRNA) that down-regulate the expression of Cripto and/or GRP78. These Cripto-targeting and GRP78-targeting siNA's may be administered to a subject in a pharmaceutical composition (e.g., parenterally, intravenously, or intratumorally) to treat a cancer. For example, as shown in the below examples, a shRNA may be effectively used to knockdown GRP78 (SEQ ID NO:5) or Cripto (SEQ ID NO:4) signaling and disrupt Cripto/GRP78 complex formation.

"siNA", as used herein, is defined as a short interfering nucleic acid. Examples of siNA include but are not limited to RNAi, double-stranded RNA, and siRNA. A siNA can inhibit the transcription of a gene in a cell. A siNA may be from 16 to 50 or more nucleotides long. In certain embodiments, the siNA may be 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides long. The siNA may comprise a nucleic acid and/or a nucleic acid analog. Typically, a siNA will inhibit the translation of a single gene within a cell; however, in certain embodiments, a siNA will inhibit the translation of more than one gene within a cell. While, in certain embodiments, a siNA may be used to disrupt a Cripto/GRP78 interaction, in other embodiments an antisense may be used to target Cripto and/or GRP78 to disrupt the Cripto/GRP78 interaction.

Within a siNA, a nucleic acids do not have to be of the same type (*e.g.,* a siNA may comprise a nucleotide and a nucleic acid analog). siNA form a double-stranded structure; the double-stranded structure may result from two separate nucleic acids that are partially or completely complementary. As disclosed herein, the siNA may comprise only a single nucleic acid or nucleic acid analog and form a double-stranded structure by complementing with itself (*e.g*., forming a hairpin loop). The double-stranded structure of the siNA may comprise 16 to 500 or more contiguous nucleobases. The siNA may comprise 17 to 35 contiguous nucleobases, more preferably 18 to 30 contiguous nucleobases, more preferably 19 to 25 nucleobases, more preferably 20 to 23 contiguous nucleobases, or 20 to 22 contiguous nucleobases, or 21 contiguous nucleobases that hybridize with a complementary nucleic acid (which may be another part of the same nucleic acid or a separate complementary nucleic acid) to form a double-stranded structure.

siNA (*e.g*., siRNA) are well known in the art. For example, siRNA and double-stranded RNA have been described in U.S. Patents 6,506,559 and 6,573,099, as well as in U.S. Applications 2003/0051263, 2003/0055020, 2004/0265839, 2002/0168707, 2003/0159161, 2004/0064842.

### 1. Nucleic Acids

There are also disclosed methods and compositions for the delivery of siNA *via* neutral liposomes. Because a siNA is composed of a nucleic acid, methods relating to nucleic acids (*e.g*., production of a nucleic acid, modification of a nucleic acid, *etc.*) may also be used with regard to a siNA.

The term "nucleic acid" is well known in the art. A "nucleic acid" as used herein will generally refer to a molecule (*i.e*., a strand) of DNA, RNA or a derivative or analog thereof, comprising a nucleobase. A nucleobase includes, for example, a naturally occurring purine or pyrimidine base found in DNA (*e.g*., an adenine "A," a guanine "G," a thymine "T" or a cytosine "C") or RNA (*e.g*., an A, a G, an uracil "U" or a C). The term "nucleic acid" encompass the terms "oligonucleotide" and "polynucleotide," each as a subgenus of the term "nucleic acid." The term "oligonucleotide" refers to a molecule of between 3 and about 100 nucleobases in length. The term "polynucleotide" refers to at least one molecule of greater than about 100 nucleobases in length.

These definitions refer to a single-stranded or double-stranded nucleic acid molecule. Double stranded nucleic acids are formed by fully complementary binding, although in some embodiments a double stranded nucleic acid may formed by partial or substantial complementary binding. Thus, a nucleic acid may encompass a double-stranded molecule that comprises one or more complementary strand(s) or "complement(s)" of a particular sequence, typically comprising a molecule. As used herein, a single stranded nucleic acid may be denoted by the prefix "ss" and a double stranded nucleic acid by the prefix "ds".

### 2. Nucleobases

As used herein a "nucleobase" refers to a heterocyclic base, such as for example a naturally occurring nucleobase (*i.e*., an A, T, G, C or U) found in at least one naturally occurring nucleic acid (*i.e*., DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in manner that may substitute for naturally occurring nucleobase pairing (*e.g*., the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, caboxyalkyl, amino, hydroxyl, halogen (*i.e*., fluoro, chloro, bromo, or iodo), thiol or alkylthiol moeity. Preferred alkyl (*e.g*., alkyl, caboxyalkyl, etc.) moeities comprise of from about 1, about 2, about 3, about 4, about 5, to about 6 carbon atoms. Other non-limiting examples of a purine or pyrimidine include a deazapurine, a 2,6-diaminopurine, a 5-fluorouracil, a xanthine, a hypoxanthine, a 8-bromoguanine, a 8-chloroguanine, a bromothymine, a 8-aminoguanine, a 8-hydroxyguanine, a 8-methylguanine, a 8-thioguanine, an azaguanine, a 2-aminopurine, a 5-ethylcytosine, a 5-methylcyosine, a 5-bromouracil, a 5-ethyluracil, a 5-iodouracil, a 5-chlorouracil, a 5-propyluracil, a thiouracil, a 2-methyladenine, a methylthioadenine, a N,N-diemethyladenine, an azaadenines, a 8-bromoadenine, a 8-hydroxyadenine, a 6-hydroxyaminopurine, a 6-thiopurine, a 4-(6-aminohexyl/cytosine), and the like. A table non-limiting, purine and pyrimidine derivatives and analogs is also provided herein below.

| **Table 1-Purine and Pvrmidine Derivatives or Analogs** | | | |
|---|---|---|---|
| **Abbr.** | **Modified base description** | **Abbr.** | **Modified base description** |
| ac4c | 4-acetylcytidine | Mam5s2u | 5-methoxyaminomethyl-2-thiouridine |
| Chm5u | 5-(carboxyhydroxylmethyl) uridine | Man q | Beta,D-mannosylqueosine |
| Cm | 2'-O-methylcytidine | Mcm5s2u | 5-methoxycarbonylmethyl-2-thiouridine |
| Cmnm5s2u | 5-carboxymethylamino-methyl-2-thioridine | Mcm5u | 5-methoxycarbonylmethyluridine |
| Cmnm5u | 5-carboxymethylaminomethyluridine | Mo5u | 5-methoxyuridine |
| D | Dihydrouridine | Ms2i6a | 2-methylthio-N6-isopentenyladenosine |
| Fm | 2'-O-methylpseudouridine | Ms2t6a | N-((9-beta-D-ribofuranosyl-2-methylthiopurine-6-yl)carbamoyl)threonine |
| Gal q | Beta,D-galactosylqueosine | Mt6a | N-((9-beta-D-ribofuranosylpurine-6-yl)N-methyl-carbamoyl)threonine |
| Gm | 2'-O-methylguanosine | Mv | Uridine-5-oxyacetic acid methylester |
| I | Inosine | o5u | Uridine-5-oxyacetic acid (v) |
| I6a | N6-isopentenyladenosine | Osyw | Wybutoxosine |
| m1a | 1-methyladenosine | P | Pseudouridine |
| mlf | 1-methylpseudouridine | Q | Queosine |
| m1g | 1-methylguanosine | s2c | 2-thiocytidine |
| m1I | 1-methylinosine | s2t | 5-methyl-2-thiouridine |
| m22g | 2,2-dimethylguanosine | s2u | 2-thiouridine |
| m2a | 2-methyladenosine | s4u | 4-thiouridine |
| m2g | 2-methylguanosine | T | 5-methyluridine |
| m3c | 3-methylcytidine | t6a | N-((9-beta-D-ribofuranosylpurine-6-yl)carbamoyl)threonine |
| m5c | 5-methylcytidine | Tm | 2'-O-methyl-5-methyluridine |
| m6a | N6-methyladenosine | Um | 2'-O-methyluridine |
| m7g | 7-methylguanosine | Yw | Wybutosine |
| Mam5u | 5-methylaminomethyluridine | X | 3-(3-amino-3-carboxypropyl)uridine, (acp3)u |

A nucleobase may be comprised in a nucleside or nucleotide, using any chemical or natural synthesis method described herein or known to one of ordinary skill in the art.

### 3. Nucleosides

As used herein, a "nucleoside" refers to an individual chemical unit comprising a nucleobase covalently attached to a nucleobase linker moiety. A non-limiting example of a "nucleobase linker moiety" is a sugar comprising 5-carbon atoms (*i.e*., a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring.

Different types of covalent attachment(s) of a nucleobase to a nucleobase linker moiety are known in the art. By way of non-limiting example, a nucleoside comprising a purine (*i.e*., A or G) or a 7-deazapurine nucleobase typically covalently attaches the 9 position of a purine or a 7-deazapurine to the 1'-position of a 5-carbon sugar. In another non-limiting example, a nucleoside comprising a pyrimidine nucleobase (*i.e*., C, T or U) typically covalently attaches a 1 position of a pyrimidine to a 1'-position of a 5-carbon sugar (Kornberg and Baker, 1992).

### 4. Nucleotides

As used herein, a "nucleotide" refers to a nucleoside further comprising a "backbone moiety". A backbone moiety generally covalently attaches a nucleotide to another molecule comprising a nucleotide, or to another nucleotide to form a nucleic acid. The "backbone moiety" in naturally occurring nucleotides typically comprises a phosphorus moiety, which is covalently attached to a 5-carbon sugar. The attachment of the backbone moiety typically occurs at either the 3'- or 5'-position of the 5-carbon sugar. However, other types of attachments are known in the art, particularly when a nucleotide comprises derivatives or analogs of a naturally occurring 5-carbon sugar or phosphorus moiety.

### 5. Nucleic Acid Analogs

A nucleic acid may comprise, or be composed entirely of, a derivative or analog of a nucleobase, a nucleobase linker moiety and/or backbone moiety that may be present in a naturally occurring nucleic acid. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980).

Additional non-limiting examples of nucleosides, nucleotides or nucleic acids comprising 5-carbon sugar and/or backbone moiety derivatives or analogs, include those in U.S. Patent 5,681,947 which describes oligonucleotides comprising purine derivatives that form triple helixes with and/or prevent expression of dsDNA; U.S. Patents 5,652,099 and 5,763,167 which describe nucleic acids incorporating fluorescent analogs of nucleosides found in DNA or RNA, particularly for use as flourescent nucleic acids probes; U.S. Patent 5,614,617 which describes oligonucleotide analogs with substitutions on pyrimidine rings that possess enhanced nuclease stability; U.S. Patents 5,670,663, 5,872,232 and 5,859,221 which describe oligonucleotide analogs with modified 5-carbon sugars (*i.e*., modified 2'-deoxyfuranosyl moieties) used in nucleic acid detection; U.S. Patent 5,446,137 which describes oligonucleotides comprising at least one 5-carbon sugar moiety substituted at the 4' position with a substituent other than hydrogen that can be used in hybridization assays; U.S. Patent 5,886,165 which describes oligonucleotides with both deoxyribonucleotides with 3'-5' internucleotide linkages and ribonucleotides with 2'-5' internucleotide linkages; U.S. Patent 5,714,606 which describes a modified internucleotide linkage wherein a 3'-position oxygen of the internucleotide linkage is replaced by a carbon to enhance the nuclease resistance of nucleic acids; U.S. Patent 5,672,697 which describes oligonucleotides containing one or more 5' methylene phosphonate internucleotide linkages that enhance nuclease resistance; U.S. Patents 5,466,786 and 5,792,847 which describe the linkage of a substituent moeity which may comprise a drug or label to the 2' carbon of an oligonucleotide to provide enhanced nuclease stability and ability to deliver drugs or detection moieties; U.S. Patent 5,223,618 which describes oligonucleotide analogs with a 2 or 3 carbon backbone linkage attaching the 4' position and 3' position of adjacent 5-carbon sugar moiety to enhanced cellular uptake, resistance to nucleases and hybridization to target RNA; U.S. Patent 5,470,967 which describes oligonucleotides comprising at least one sulfamate or sulfamide internucleotide linkage that are useful as nucleic acid hybridization probe; U.S. Patents 5,378,825, 5,777,092, 5,623,070, 5,610,289 and 5,602,240 which describe oligonucleotides with three or four atom linker moeity replacing phosphodiester backbone moeity used for improved nuclease resistance, cellular uptake and regulating RNA expression; U.S. Patent 5,858,988 which describes hydrophobic carrier agent attached to the 2'-O position of oligonuceotides to enhanced their membrane permeability and stability; U.S. Patent 5,214,136 which describes olignucleotides conjugaged to anthraquinone at the 5' terminus that possess enhanced hybridization to DNA or RNA; enhanced stability to nucleases; U.S. Patent 5,700,922 which describes PNA-DNA-PNA chimeras wherein the DNA comprises 2'-deoxy-erythro-pentofuranosyl nucleotides for enhanced nuclease resistance, binding affinity, and ability to activate RNase H; and U.S. Patent 5,708,154 which describes RNA linked to a DNA to form a DNA-RNA hybrid.

### 6. Polyether and Peptide Nucleic Acids

In certain embodiments, it is contemplated that a nucleic acid comprising a derivative or analog of a nucleoside or nucleotide may be used in the methods and compositions as disclosed herein. A non-limiting example is a "polyether nucleic acid", described in U.S. Patent 5,908,845. In a polyether nucleic acid, one or more nuclcobascs arc linked to chiral carbon atoms in a polyether backbonc.

Another non-limiting example is a "peptide nucleic acid", also known as a "PNA", "peptide-based nucleic acid analog" or "PENAM", described in U.S. Patent 5,786,461, 5891,625, 5,773,571, 5,766,855, 5,736,336, 5,719,262, 5,714,331, 5,539,082, and WO 92/20702. Peptide nucleic acids generally have enhanced sequence specificity, binding properties, and resistance to enzymatic degradation in comparison to molecules such as DNA and RNA *(*Egholm et al., 1993; PCT/EP/01219). A peptide nucleic acid generally comprises one or more nucleotides or nucleosides that comprise a nucleobase moiety, a nucleobase linker moeity that is not a 5-carbon sugar, and/or a backbone moiety that is not a phosphate backbone moiety. Examples of nucleobase linker moieties described for PNAs include aza nitrogen atoms, amido and/or ureido tethers (see for example, U.S. Patent 5,539,082). Examples of backbone moieties described for PNAs include an aminoethylglycine, polyamide, polyethyl, polythioamide, polysulfinamide or polysulfonamide backbone moiety.

As disclosed herein, a nucleic acid analogue such as a peptide nucleic acid may be used to inhibit nucleic acid amplification, such as in PCR™, to reduce false positives and discriminate between single base mutants, as described in U.S. Patent 5,891,625. Other modifications and uses of nucleic acid analogs are known in the art, and it is anticipated that these techniques and types of nucleic acid analogs may be used with the present invention. In a non-limiting example, U.S. Patent 5,786,461 describes PNAs with amino acid side chains attached to the PNA backbone to enhance solubility of the molecule. In another example, the cellular uptake property of PNAs is increased by attachment of a lipophilic group. U.S. Application Ser. No. 117,363 describes several alkylamino moeities used to enhance cellular uptake of a PNA. Another example is described in U.S. Patents 5,766,855, 5,719,262, 5,714,331 and 5,736,336, which describe PNAs comprising naturally and non-naturally occurring nucleobases and alkylamine side chains that provide improvements in sequence specificity, solubility and/or binding affinity relative to a naturally occurring nucleic acid.

### 7. Preparation of Nucleic Acids

A nucleic acid may be made by any technique known to one of ordinary skill in the art, such as for example, chemical synthesis, enzymatic production or biological production. Non-limiting examples of a synthetic nucleic acid (*e.g*., a synthetic oligonucleotide), include a nucleic acid made by *in vitro* chemically synthesis using phosphotriester, phosphite or phosphoramidite chemistry and solid phase techniques such as described in EP 266,032, or via deoxynucleoside H-phosphonate intermediates as described by Froehler *et al*., 1986 and U.S. Patent 5,705,629.

In the methods of the present invention, one or more oligonucleotide may be used. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR™ (see for example, U.S. Patent 4,683,202 and U.S. Patent 4,682, 195), or the synthesis of an oligonucleotide described in U.S. Patent 5,645,897. A non-limiting example of a biologically produced nucleic acid includes a recombinant nucleic acid produced (*i.e*., replicated) in a living cell, such as a recombinant DNA vector replicated in bacteria (see for example, Sambrook *et al.* 2001).

### 8. Purification of Nucleic Acids

A nucleic acid may be purified on polyacrylamide gels, cesium chloride centrifugation gradients, or by any other means known to one of ordinary skill in the art (see for example, Sambrook *et al*., 2001).

In certain embodiments, the present invention concerns a nucleic acid that is an isolated nucleic acid. As used herein, the term "isolated nucleic acid" refers to a nucleic acid molecule (*e.g*., an RNA or DNA molecule) that has been isolated free of, or is otherwise free of, the bulk of the total genomic and transcribed nucleic acids of one or more cells. In certain embodiments, "isolated nucleic acid" refers to a nucleic acid that has been isolated free of, or is otherwise free of, bulk of cellular components or in vitro reaction components such as for example, macromolecules such as lipids or proteins, small biological molecules, and the like.

### 9. Hybridization

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but precludes hybridization of random sequences. Stringent conditions tolerate little, if any, mismatch between a nucleic acid and a target strand. Such conditions are well known to those of ordinary skill in the art, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.15 M NaCl at temperatures of about 50°C to about 70°C. It is understood that the temperature and ionic strength of a desired stringency are determined in part by the length of the particular nucleic acid(s), the length and nucleobase content of the target sequence(s), the charge composition of the nucleic acid(s), and to the presence or concentration of formamide, tetramethylammonium chloride or other solvent(s) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is preferred to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions", and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suite a particular application.

### D. Aptamers

Aptamers may be used to inhibit Cripto/GRP78 binding and/or signaling. Aptamers are single stranded nucleic acids which selectively bind a molecular target, such as a protein. Aptamers may comprise DNA, RNA, and/or modified nucleotides, although in certain embodiments it may be desirable to use DNA aptamers or aptamers comprising modified nucleotides which resist enzymatic degradation in order to increase half-life when administered to a subject *in vivo.*

The idea of using single stranded nucleic acids (aptamers) as affinity molecules for proteins has shown modest progress. See Tuerk and Gold, (1990); Ellington and Szostak (1990); and Ellington and Szostak (1992). The concept is based on the ability of short oligomer (20-80 mer) sequences to fold, in the presence of a target, into unique 3-dimensional structures that bind the target with high affinity and specificity. Aptamers are generated by a process that combines combinatorial chemistry with in vitro evolution, commonly known as SELEX (Systematic Evolution of Ligands by Exponential Enrichment). Following the incubation of a protein with a library of DNA or RNA sequences (typically about 10¹⁴ molecules in complexity) protein-DNA complexes are isolated, the DNA is amplified, and the process is repeated until the sample is enriched with sequences that display high affinity for the protein of interest. Since the selection pressure is high affinity for the target, aptamers with low nanomolar affinities may be obtained. Aptamers offer advantages over protein-based affinity reagents because nucleic acids possess increased stability, ease of regeneration (PCR or oligonucleotide synthesis), and simple modification for detection and immobilization. High-throughput methods for aptamer production which utilize robotics may also be used with the present invention (Cox *et al*., 2002).

Several variations in aptamer production protocols (e.g., varying target partitioning) may be used with the present invention. Unbound DNA molecules may be removed from target proteins via: 1) filtration on a membrane (Ellington and Szostak, 1992); 2) column chromatography, in which the targets are bound to a matrix, such as sepharose, using a covalent linkage or an affinity tag (Ylera *et al*., 2002); and 3) binding of the protein to the wells of a microtiter plate (Drolet *et al*., 1999). Methods for aptamer production which may be used with the present invention are also described, *e*.*g*., in US patent 6,423,493; US patent 6,515,120; US patent 6,180,348; US patent 5,756,291, and US patent 7,329,742.

### E. Small molecules

Small molecules may also be used to inhibit Cripto/GRP78 binding and/or signaling. In various embodiments, one or more small molecule chemical libraries may be screened to identify small molecules which may selectively affect the Cripto/GRP78 interaction. For example, a high-throughput screen may be automated via robotics to evaluate and/or identify a small molecule which can inhibit Cripto/GRP78 binding and/or signaling. In certain embodiments, computer modeling of Cripto/GRP78 binding may be used to select candidate small molecules for testing.

Without wishing to be bound by any theory, the inventors envision that GRP78 could be binding Cripto at the cell surface in its capacity as a chaperone. In this case, GRP78 might stabilize the Cripto molecule or promote a Cripto conformation that is required for signaling; thus, GRP78 may resemble HSP90 which which has chaperone function that is required for the activity of several oncogenes. HSP90 antagonists that block its ATP binding site are in clinical trials. In the instance that the classical chaperone function of GRP78 is required for its role as a mediator of Cripto signaling, small molecules that target the GRP78 ATP binding domain might be utilized clinically. For example, (-)-Epigallocatechin Gallate (EGCG) is a major component of green tea that has several anticancer properties. EGCG has been shown to specifically bind GRP78 and inhibit its chaperone function by inhibiting its ability to bind ATP (Svetlana P et al Cancer Res. 2006 66(18) 9260-69). Derivatives of EGCG could thus be screened to determine if these compounds can sidrupt Cripto/GRP78 complex formation.

### V. PHARMACEUTICAL PREPARATIONS

Pharmaceutical compositions as disclosed herein comprise an effective amount of one or more Cripto- and/or GRP78-targeting agent or additional agent dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of an pharmaceutical composition that contains at least one Cripto- and/or GRP78-targeting agent or additional active ingredient will be known to those of skill in the art in light of the present disclosure, as exemplified by Remington: The Science and Practice of Pharmacy, 21st edition, by University of the Sciences in Philadelphia. Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329).

Except insofar as any conventional carrier is incompatible with the active ingredient, its use in the therapeutic or pharmaceutical compositions is contemplated.

The Cripto- and/or GRP78-targeting agent may comprise different types of carriers depending on whether it is to be administered in solid, liquid or aerosol form, and whether it need to be sterile for such routes of administration as injection. The present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (*e*.*g*.. aerosol inhalation), injection, infusion, continuous infusion, localized perfusion bathing target cells directly, *via* a catheter, via a lavage, in cremes, in lipid compositions (*e*.*g*., liposomes), or by other method or any combination of the forgoing as would be known to one of ordinary skill in the art (see, for example, Remington's Pharmaceutical Sciences, 19th Ed. Mack Printing Company, 1995).

The actual dosage amount of a composition of the present invention administered to an animal patient can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, the an active compound may comprise between about 2% to about 75% of the weight of the unit, or between about 25% to about 60%, for example, and any range derivable therein. In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, *etc*., can be administered, based on the numbers described above.

In certain embodiments, a monoclonal antibody may be administered to a subject (*e*.*g*., a human patient) at a dose of about 1 - 25 mg/kg every 1 to 3 weeks. In certain embodiments, a siNA *(e.g.,* a siRNA) may be administered at a dose of about 1 - 10 mg/kg at an interval of about daily to about weekly.

In any case, the composition may comprise various antioxidants to retard oxidation of one or more component. Additionally, the prevention of the action of microorganisms can be brought about by preservatives such as various antibacterial and antifungal agents, including but not limited to parabens (*e*.*g*., methylparabens, propylparabens), chlorobutanol, phenol, sorbic acid, thimerosal or combinations thereof.

The Cripto- and/or GRP78-targeting agent may be formulated into a composition in a free base, neutral or salt form. Pharmaceutically acceptable salts, include the acid addition salts, *e.g.,* those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine.

Where the composition is in a liquid form, a carrier can be a solvent or dispersion medium comprising but not limited to, water, ethanol, polyol (*e*.*g*., glycerol, propylene glycol, liquid polyethylene glycol, etc), lipids (*e*.*g*., triglycerides, vegetable oils, liposomes) and combinations thereof. The proper fluidity can be maintained, for example, by the use of a coating, such as lecithin; by the maintenance of the required particle size by dispersion in carriers such as, for example liquid polyol or lipids; by the use of surfactants such as, for example hydroxypropylcellulose; or combinations thereof such methods. In many cases, it will be preferable to include isotonic agents, such as, for example, sugars, sodium chloride or combinations thereof.

As also disclosed herein, one may use eye drops, nasal solutions or sprays, aerosols or inhalants in the present invention. Such compositions are generally designed to be compatible with the target tissue type. In a non-limiting example, nasal solutions are usually aqueous solutions designed to be administered to the nasal passages in drops or sprays. Nasal solutions are prepared so that they are similar in many respects to nasal secretions, so that normal ciliary action is maintained. Thus, as disclosed herein, the aqueous nasal solutions usually are isotonic or slightly buffered to maintain a pH of about 5.5 to about 6.5. In addition, antimicrobial preservatives, similar to those used in ophthalmic preparations, drugs, or appropriate drug stabilizers, if required, may be included in the formulation. For example, various commercial nasal preparations are known and include drugs such as antibiotics or antihistamines.

As also disclosed herein, the Cripto- and/or GRP78-targeting agent is prepared for administration by such routes as oral ingestion. The solid
composition may comprise, for example, solutions, suspensions, emulsions, tablets, pills, capsules (*e*.*g*., hard or soft shelled gelatin capsules), sustained release formulations, buccal compositions, troches, elixirs, suspensions, syrups, wafers, or combinations thereof. Oral compositions may be incorporated directly with the food of the diet. Preferred carriers for oral administration comprise inert diluents, assimilable edible carriers or combinations thereof. In other aspects of the invention, the oral composition may be prepared as a syrup or elixir. A syrup or elixir, and may comprise, for example, at least one active agent, a sweetening agent, a preservative, a flavoring agent, a dye, a preservative, or combinations thereof.

As disclosed herein, an oral composition may comprise one or more binders, excipients, disintegration agents, lubricants, flavoring agents, and combinations thereof. As disclosed herein, a composition may comprise one or more of the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, *etc*.; or combinations thereof the foregoing. When the dosage unit form is a capsule, it may contain, in addition to materials of the above type, carriers such as a liquid carrier. Various other materials may be present as coatings or to otherwise modify the physical form of the dosage unit. For instance, tablets, pills, or capsules may be coated with shellac, sugar or both.

Additional formulations which are suitable for other modes of administration include suppositories. Suppositories are solid dosage forms of various weights and shapes, usually medicated, for insertion into the rectum, vagina or urethra. After insertion, suppositories soften, melt or dissolve in the cavity fluids. In general, for suppositories, traditional carriers may include, for example, polyalkylene glycols, triglycerides or combinations thereof.

Suppositories may be formed from mixtures containing, for example, the active ingredient in the range of about 0.5% to about 10%, and preferably about 1% to about 2%.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

As disclosed herein, prolonged absorption of an injectable composition can be brought about by the use in the compositions of agents delaying absorption, such as, for example, aluminum monostearate, gelatin or combinations thereof.

### VI. HYPERPROLIFERATIVE DISEASES

Compounds which disrupt the Cripto/GRP78 interaction may be used to treat a hyperproliferative disease, such as cancer. In addition to cancers, compounds which disrupt the Cripto/GRP78 interaction may be used to treat other hyperproliferative diseases including psoriasis, fibrosis, tumor angiogenesis, dermal hypoproliferation/scarring, atheroma, atherosclerosis, rheumatoid arthritis, inflammation and autoimmune disorders.. "Hyperproliferative disease," as used herein, refers to a disease which results in or is characterized by the abnormal growth or multiplication of cells. Hyperproliferative diseases may manifest lesions in a subject, such as, *e*.*g*., pre-malignant lesions, benign tumors, and cancers.

Various cancers may be treated via the disruption of Cripto/GRP78 binding and/or interactions in cancerous cells, *e.g.,* via contacting at least some of the cancerous cells with a Cripto-targeting and/or a GRP78-targeting compound. Cancers which may be treated with compounds of or identified via methods of the present invention include solid tumors, metastatic cancers, and/or non-metastatic cancers. The cancer may originate in the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In various embodiments, the cancer may be histologically classified as a: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary or follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphoma; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; or hairy cell leukemia.

Cripto and GRP78 are broadly overexpressed in many human tumors. It is nonetheless anticipated that cancers of the breast and prostate may be particularly sensitive to inhibition of cellular proliferation by inhibition of Cripto/GRP78 complex formation.

### A. Combination Therapies

In order to increase the effectiveness of a Cripto- and/or GRP78-targeting agent, it may be desirable to combine these compositions and methods as disclosed herein with an agent effective in the treatment of hyperproliferative disease, such as, for example, an anti-cancer agent. An "anti-cancer" agent is capable of negatively affecting cancer in a subject, for example, by killing one or more cancer cells, inducing apoptosis and/or nccrosis in one or more cancer cells, reducing the growth rate of one or more cancer cells, reducing the incidence or number of metastases, reducing a tumor's size, inhibiting a tumor's growth, reducing the blood supply to a tumor or one or more cancer cells, altering a tumor stroma micro-environment, promoting an immune response against one or more cancer cells or a tumor, preventing or inhibiting the progression of a cancer, or increasing the lifespan of a subject with a cancer. Anti-cancer agents include, for example, chemotherapy agents (chemotherapy), radiotherapy agents (radiotherapy), a surgical procedure (surgery), immune therapy agents (immunotherapy), genetic therapy agents (gene therapy), hormonal therapy, other biological agents (biotherapy) and/or alternative therapies.

More generally, such an agent would be provided in a combined amount with an Cripto- and/or GRP78-targeting agent effective to kill or inhibit proliferation of a cancer cell. This process may involve contacting the cell(s) with an agent(s) and the Cripto- and/or GRP78-targeting agent at the same time or within a period of time wherein separate administration of the Cripto- and/or GRP78-targeting agent and an agent to a cell, tissue or organism produces a desired therapeutic benefit. This may be achieved by contacting the cell, tissue or organism with a single composition or pharmacological formulation that includes both a Cripto- and/or GRP78-targeting agent and one or more agents, or by contacting the cell with two or more distinct compositions or formulations, wherein one composition includes a Cripto- and/or GRP78-targeting agent and the other includes one or more agents.

The terms "contacted" and "exposed," when applied to a cell, tissue or organism, are used herein to describe the process by which a therapeutic construct of the Cripto- and/or GRP78-targeting agent and/or another agent, such as for example a chemotherapeutic or radiotherapeutic agent, are delivered to a target cell, tissue or organism or are placed in direct juxtaposition with the target cell, tissue or organism. To achieve cell killing or stasis, the Cripto- and/or GRP78-targeting agent and/or additional agent(s) are delivered to one or more cells in a combined amount effective to kill the cell(s) or prevent them from dividing.

The Cripto- and/or GRP78-targeting agent may precede, be co-current with and/or follow the other agent(s) by intervals ranging from minutes to weeks. In embodiments where the Cripto- and/or GRP78-targeting agent, and other agent(s) are applied separately to a cell, tissue or organism, one would generally ensure that a significant period of time did not expire between the time of each delivery, such that the Cripto- and/or GRP78-targeting agent and agent(s) would still be able to exert an advantageously combined effect on the cell, tissue or organism. For example, in such instances, it is contemplated that one may contact the cell, tissue or organism with two, three, four or more modalities substantially simultaneously (*i*.*e*., within less than about a minute) as the Cripto- and/or GRP78-targeting agent. In other aspects, one or more agents may be administered within of from substantially simultaneously, about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, about 48 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 1, about 2, about 3, about 4, about 5, about 6, about 7 or about 8 weeks or more, and any range derivable therein, prior to and/or after administering the Cripto- and/or GRP78-targeting agent.

Various combination regimens of the Cripto- and/or GRP78-targeting agent and one or more agents may be employed. Non-limiting examples of such combinations are shown below, wherein a composition comprising a Cripto- and/or GRP78-targeting agent is "A" and an agent is "B":

| | | | | | | | |
|---|---|---|---|---|---|---|---|
| A/B/A | B/A/B | B/B/A | A/A/B | A/B/B | B/A/A | A/B/B/B | B/A/B/B |
| B/B/B/A | B/B/A/B | | A/A/B/B | A/B/A/B | | A/B/B/A | B/B/A/A |
| B/A/B/A | B/A/A/B | | A/A/A/B | B/A/A/A | | A/B/A/A | A/A/B/A |

Administration of the Cripto- and/or GRP78-targeting agent to a cell, tissue or organism may follow general protocols for the administration of chemotherapeutics, taking into account the toxicity, if any. It is expected that the treatment cycles would be repeated as necessary. In particular embodiments, it is contemplated that various additional agents may be applied in any combination with the present invention.

### 1. Chemotherapeutic Agents

The term "chemotherapy" refers to the use of drugs to treat cancer. A "chemotherapeutic agent" is used to connote a compound or composition that is administered in the treatment of cancer. One subtype of chemotherapy known as biochemotherapy involves the combination of a chemotherapy with a biological therapy.

Chemotherapeutic agents include, but are not limited to, 5-fluorouracil, bleomycin, busulfan, camptothecin, carboplatin, chlorambucil, cisplatin (CDDP), cyclophosphamide, dactinomycin, daunorubicin, doxorubicin, estrogen receptor binding agents, etoposide (VP16), farnesyl-protein transferase inhibitors, gemcitabine, ifosfamide, mechlorethamine, melphalan, mitomycin, navelbine, nitrosurea, plicomycin, procarbazine, raloxifene, tamoxifen, taxol, temazolomide (an aqueous form of DTIC), transplatinum, vinblastine and methotrexate, vincristine, or any analog or derivative variant of the foregoing. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis. Most chemotherapeutic agents fall into the following categories: alkylating agents, antimetabolites, antitumor antibiotics, corticosteroid hormones, mitotic inhibitors, and nitrosoureas, hormone agents, miscellaneous agents, and any analog or derivative variant thereof.

Chemotherapeutic agents and methods of administration, dosages, etc. are well known to those of skill in the art (see for example, the "Physicians Desk Reference," Goodman & Gilman's "The Pharmacological Basis of Therapeutics," "Remington's Pharmaceutical Sciences," and "The Merck Index, Eleventh Edition,"),
and may be combined with the invention in light of the disclosures herein. Some variation in dosage will necessarily occur depending on the condition of the subject being treated. The person responsible for administration will, in any event, determine the appropriate dose for the individual subject. Examples of specific chemotherapeutic agents and dose regimes are also described herein. Of course, all of these dosages and agents described herein are exemplary rather than limiting, and other doses or agents may be used by a skilled artisan for a specific patient or application. Any dosage in-between these points, or range derivable therein is also expected to be of use in the invention.

### 2. Radiotherapeutic Agents

Radiotherapeutic agents include radiation and waves that induce DNA damage for example, γ-irradiation, X-rays, proton beam therapies (U.S. Patents 5,760,395 and 4,870,287), UV-irradiation, microwaves, electronic emissions, radioisotopes, and the like. Therapy may be achieved by irradiating the localized tumor site with the above described forms of radiations. It is most likely that all of these agents effect a broad range of damage DNA, on the precursors of DNA, the replication and repair of DNA, and the assembly and maintenance of chromosomes.

Radiotherapeutic agents and methods of administration, dosages, etc. are well known to those of skill in the art, and may be combined with the invention in light of the disclosures herein. For example, dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

### 3. Surgery

Approximately 60% of persons with cancer will undergo surgery of some type, which includes, for example, preventative, diagnostic or staging, curative and palliative surgery. Surgery, and in particular a curative surgery, may be used in conjunction with other therapies, such as the present invention and one or more other agents.

Curative surgery includes resection in which all or part of cancerous tissue is physically removed, excised and/or destroyed. It is further contemplated that surgery may remove, excise or destroy superficial cancers, precancers, or incidental amounts of normal tissue. Treatment by surgery includes for example, tumor resection, laser surgery, cryosurgery, electrosurgery, and miscopically controlled surgery (Mohs' surgery). Tumor resection refers to physical removal of at least part of a tumor. Upon excision of part of all of cancerous cells, tissue, or tumor, a cavity may be formed in the body.

Further treatment of the tumor or area of surgery may be accomplished by perfusion, direct injection or local application of the area with an additional anti-cancer agent. Such treatment may be repeated, for example, about every 1, about every 2, about every 3, about every 4, about every 5, about every 6, or about every 7 days, or about every 1, about every 2, about every 3, about every 4, or about every 5 weeks or about every 1, about every 2, about every 3, about every 4, about every 5, about every 6, about every 7, about every 8, about every 9, about every 10, about every 11, or about every 12 months. These treatments may be of varying dosages as well.

### 4. Immunotherapeutic Agents

An immunotherapeutic agent generally relies on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (*e*.*g*., a chemotherapeutic, a radionuclide, a ricin A chain, a cholera toxin, a pertussis toxin, *etc*.) and serve merely as a targeting agent. Such antibody conjugates are called immunotoxins, and are well known in the art (see U.S. Patent 5,686,072, U.S. Patent 5,578,706, U.S. Patent 4,792,447, U.S. Patent 5,045,451, U.S. Patent 4,664,911, and U.S. Patent 5,767,072).

Alternatively, the effector may be a lymphocyte carrying a surface molecule that interacts, either directly or indirectly, with a tumor cell target. Various effector cells include cytotoxic T cells and NK cells.

In one aspect of immunotherapy, the tumor cell must bear some marker that is amenable to targeting, *i*.*e*., is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155.

### 5. Genetic Therapy Agents

A tumor cell resistance to agents, such as chemotherapeutic and radiotherapeutic agents, represents a major problem in clinical oncology. One goal of current cancer research is to find ways to improve the efficacy of one or more anti-cancer agents by combining such an agent with gene therapy. For example, the herpes simplex-thymidine kinase (HS-tK) gene, when delivered to brain tumors by a retroviral vector system, successfully induced susceptibility to the antiviral agent ganciclovir (Culver, *et al*., 1992). In the context of the present invention, it is contemplated that gene therapy could be used similarly in conjunction with the Cripto- and/or GRP78-targeting agent and/or other agents.

### 6. Molecular Targeting Therapy

The term of "targeting agent" means any agent (e.g., small molecules and polypeptides including antibodies) used to treat a disease through targeting specific molecules or signaling pathways. The combination of an anti-Cripto- and/or anti-GRP78 agent with one or more other targeting agents may improve treatment of cancer through targeting multiple pathways critical to the cancer cells.

### VII. PROMOTING NEURONAL DIFFERENTIATION BY INHIBITING CRIPTO/GRP78 COMPLEX FORMATION

Inhibition of Cripto/GRP78 complex formation and/or function according to the present invention may also be utilized to promote neuronal differentiation of stem cells. It is anticipated that the differentiation of virtually any pluripotent stem cell or cell line, *e.g.,* human embryonic stem cells or induced pluripotent stem cells (iPS cells), may be influenced by disruption of Cripto/GRP78 complex formation and/or signaling. For example, human embryonic stem cell line H1, H9, hES2, hES3, hES4, hES5, hES6, BG01, BG02, BG03, HSF1, HSF6, H1, H7, H9, H13B, and/or H14 *etc.* may be used in the context of the present disclosure. It is further anticipated that stem cell lines which subsequently become available may also be used with the present invention. Other embryonic stem cells, such as mammal, mouse, primate, *etc*. may also be used in the context of the present disclosure.

As would be appreciated by one of skill, induced pluripotent stem cells, commonly abbreviated as iPS cells or iPSCs, are a type of pluripotent stem cell artificially derived from a non-pluripotent cell, typically an adult somatic cell, by inserting certain genes. Induced pluripotent stem cells are believed to be identical to natural pluripotent stem cells, such as embryonic stem cells, in many respects including the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed. IPS cells have been described previously (see , *e.g.,* Takahashi *et al*., 2006; Takahashi *et al.,* 2007; Yu *et al,* 2007).

### VIII. SCREENING FOR MODULATORS OF CRIPTO/GRP78 COMPLEX FORMATION AND FUNCTION

As disclosed herein, methods for identifying modulators of the function of the Cripto/GRP78 interaction, *e*.*g*., the ability of Cripto and GRP78 to bind and result in downstream signaling. These assays may comprise random screening of large libraries of candidate substances; alternatively, the assays may be used to focus on particular classes of compounds selected with an eye towards structural attributes that are believed to make them more likely to modulate the function of the formation of Cripto/GRP78 complexes.

By function, it is meant that one may assay for binding of Cripto to GRP78 and/or the evaluation of one or more downstream signaling pathways resulting from the formation of Cripto/GRP78 complexes (*e*.*g*., activin/Nodal/TGF-β signaling). For example, one may assay for Cripto/GRP binding in the presence of absence of a candidate modulator.

To identify a Cripto/GRP78 complex modulator, one generally will determine the function of Cripto and GRP78 in the presence and absence of the candidate substance, a modulator defined as any substance that alters the function or formation of Cripto/GRP78 complexes. For example, a method generally comprises:
(a) providing a candidate modulator;
(b) admixing the candidate modulator with an isolated compound or cell, or a suitable experimental animal;
(c) measuring whether or not the candidate modulator can alter or disrupt Cripto/GRP78 binding and/or downstream signaling in the cell or animal in step (c); and
(d) comparing the characteristic measured in step (c) with the characteristic of the compound, cell or animal in the absence of said candidate modulator,
wherein a difference between the measured characteristics indicates that said candidate modulator is, indeed, a modulator of the compound, cell or animal.

In certain embodiments, candidate modulators which selectively disrupt Cripto/GRP78 binding and/or signaling may be used to treat a hyperproliferative disease. Assays may be conducted in cell free systems, in isolated cells, or in organisms including transgenic animals.

It will, of course, be understood that all the screening methods as disclosed herein are useful in themselves notwithstanding the fact that effective candidates may not be found. There are disclosed methods for screening for such candidates, not solely methods of finding them.

### 1. Modulators

As used herein the term "candidate substance" refers to any molecule that may potentially inhibit or enhance Cripto/GRP78 complex formation or activity. The candidate substance may be a protein or fragment thereof, a small molecule, or even a nucleic acid molecule. It may prove to be the case that the most useful pharmacological compounds will be compounds that are structurally related to the N-20 antibody, a shRNA which targets GRP78 *(e.g.,* SEQ ID NO:5) or Cripto *(e.g.,* SEQ ID NO:4), peptides derived from Cripto or GRP78, the sythetic CFC domain of Cripto, soluble ALK4 ECD or mutants thereof (170A, L75A, P77A), ECGC or compounds structurally related to Cripto antibodies targeting GRP78 binding site on the CFC domain. Using lead compounds to help develop improved compounds is know as "rational drug design" and includes not only comparisons with know inhibitors and activators, but predictions relating to the structure of target molecules.

The goal of rational drug design is to produce structural analogs of biologically active polypeptides or target compounds. By creating such analogs, it is possible to fashion drugs, which are more active or stable than the natural molecules, which have different susceptibility to alteration or which may affect the function of various other molecules. In one approach, one would generate a three-dimensional structure for a target molecule, or a fragment thereof. This could be accomplished by x-ray crystallography, computer modeling or by a combination of both approaches.

It also is possible to use antibodies to ascertain the structure of a target compound activator or inhibitor. In principle, this approach yields a pharmacore upon which subsequent drug design can be based. It is possible to bypass protein crystallography altogether by generating anti-idiotypic antibodies to a functional, pharmacologically active antibody. As a mirror image of a mirror image, the binding site of anti-idiotype would be expected to be an analog of the original antigen. The anti-idiotype could then be used to identify and isolate peptides from banks of chemically- or biologically-produced peptides. Selected peptides would then serve as the pharmacore. Anti-idiotypes may be generated using the methods described herein for producing antibodies, using an antibody as the antigen.

On the other hand, one may simply acquire, from various commercial sources, small molecule libraries that are believed to meet the basic criteria for useful drugs in an effort to "brute force" the identification of useful compounds. Screening of such libraries, including combinatorially generated libraries (*e*.*g*., peptide libraries), is a rapid and efficient way to screen large number of related (and unrelated) compounds for activity. Combinatorial approaches also lend themselves to rapid evolution of potential drugs by the creation of second, third and fourth generation compounds modeled of active, but otherwise undesirable compounds.

Candidate compounds may include fragments or parts of naturally-occurring compounds, or may be found as active combinations of known compounds, which are otherwise inactive. It is proposed that compounds isolated from natural sources, such as animals, bacteria, fungi, plant sources, including leaves and bark, and marine samples may be assayed as candidates for the presence of potentially useful pharmaceutical agents. It will be understood that the pharmaceutical agents to be screened could also be derived or synthesized from chemical compositions or man-made compounds. Thus, it is understood that the candidate substance identified by the present invention may be peptide, polypeptide, polynucleotide, small molecule inhibitors or any other compounds that may be designed through rational drug design starting from known inhibitors or stimulators.

Other suitable modulators include antisense molecules, ribozymes, and antibodies (including single chain antibodies), each of which would be specific for the target molecule. Such compounds are described in greater detail elsewhere in this document. For example, an antisense molecule that bound to a translational or transcriptional start site, or splice junctions, would be ideal candidate inhibitors.

In addition to the modulating compounds initially identified, the inventors also contemplate that other sterically similar compounds may be formulated to mimic the key portions of the structure of the modulators. Such compounds, which may include peptidomimetics of peptide modulators, may be used in the same manner as the initial modulators.

An inhibitor as disclosed herein may be one which exerts its inhibitory or activating effect upstream, downstream or directly on Cripto/GRP78 complexes. Regardless of the type of inhibitor or activator identified by the present screening methods, the effect of the inhibition or activator by such a compound results in decreased or inhibited Cripto/GRP78 complex formation or activity as compared to that observed in the absence of the added candidate substance.

### 2. In vitro Assays

A quick, inexpensive and easy assay to run is an *in vitro* assay. Such assays generally use isolated molecules, can be run quickly and in large numbers, thereby increasing the amount of information obtainable in a short period of time. A variety of vessels may be used to run the assays, including test tubes, plates, dishes and other surfaces such as dipsticks or beads.

One example of a cell free assay is a binding assay. While not directly addressing function, the ability of a modulator to bind to a target molecule in a specific fashion is strong evidence of a related biological effect. For example, binding of a molecule to a target may, in and of itself, be inhibitory, due to steric, allosteric or charge-charge interactions. The target may be either free in solution, fixed to a support, expressed in or on the surface of a cell. Either the target or the compound may be labeled, thereby permitting determining of binding. Usually, the target will be the labeled species, decreasing the chance that the labeling will interfere with or enhance binding. Competitive binding formats can be performed in which one of the agents is labeled, and one may measure the amount of free label versus bound label to determine the effect on binding.

A technique for high throughput screening of compounds is described in WO 84/03564. Large numbers of small peptide test compounds are synthesized on a solid substrate, such as plastic pins or some other surface. Bound polypeptide is detected by various methods.

### 3. In cyto Assays

There is also disclosed the screening of compounds for their ability to modulate Cripto/GRP78 complex formation and/or function in cells. Various cell lines can be utilized for such screening assays, including cells specifically engineered for this purpose. For example, based on the observations described herein, cancer cells and/or stem cells may be contacted with a candidate Cripto/GRP78 complex modulator. As disclosed herein, a cell line may be engineered to over-express Cripto and GRP78 to facilitate screening of putative Cripto/GRP78 complex modulators. GRP78 protein is commercially available and may be immobilized on the surface of multi-well plates to allow the development of an ELISA-based assay to measure soluble Cripto binding and effects of Cripto/GRP78 complex modulators. Alternatively, the inventors have shown that soluble ¹²⁵I-Cripto binding to intact cells expressing GRP78 at their surface can be measured (*e.g*., see Kelber *et al*.)*.* Cripto/GRP78 complex modulators could also be screened in this assay to measure their ability to affect Cripto/GRP78 binding and signaling.

Depending on the assay, culture may be required. The cell is examined using any of a number of different physiologic assays. Alternatively, molecular analysis may be performed, for example, looking at protein expression, mRNA expression (including differential display of whole cell or polyA RNA) and others.

### 4. In vivo Assays

*In vivo* assays involve the use of various animal models, including transgenic animals that have been engineered to have specific defects, or carry markers that can be used to measure the ability of a candidate substance to reach and effect different cells within the organism. Due to their size, ease of handling, and information on their physiology and genetic make-up, mice arc a preferred embodiment, especially for transgenics. However, other animals are suitable as well, including rats, rabbits, hamsters, guinea pigs, gerbils, woodchucks, cats, dogs, sheep, goats, pigs, cows, horses and monkeys (including chimps, gibbons and baboons). Assays for modulators may be conducted using an animal model derived from any of these species.

In such assays, one or more candidate substances are administered to an animal, and the ability of the candidate substance(s) to alter one or more characteristics, as compared to a similar animal not treated with the candidate substance(s), identifies a modulator. The characteristics may be any of those discussed above with regard to the function of a particular compound (e.g., enzyme, receptor, hormone) or cell *(e.g.,* growth, tumorigenicity, survival), or instead a broader indication such as behavior, anemia, immune response, *etc.*

There are disclosed methods of screening for a candidate substance that affects Cripto/GRP78 complex formation and/or function. The present disclosure is directed to a method for determining the ability of a candidate substance to inhibit Cripto/GRP78 complex formation and/or function, generally including the steps of: administering a candidate substance to the animal; and determining the ability of the candidate substance to reduce one or more characteristics of cell proliferation, development or inhibition of a hyperproliferative disease, Cripto/GRP78 complex formation and/or signaling.

Treatment of these animals with test compounds will involve the administration of the compound, in an appropriate form, to the animal. Administration will be by any route that could be utilized for clinical or non-clinical purposes, including but not limited to oral, nasal, buccal, or even topical. Alternatively, administration may be by intratracheal instillation, bronchial instillation, intradermal, subcutaneous, intramuscular, intraperitoneal or intravenous injection. Specifically contemplated routes are systemic intravenous injection, regional administration via blood or lymph supply, or directly to an affected site.

Determining the effectiveness of a compound *in vivo* may involve a variety of different criteria. Also, measuring toxicity and dose response can be performed in animals in a more meaningful fashion than in *in vitro* or *in cyto* assays.

### IX. EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1

### Materials and Methods

**Materials.** NuPAGE gels, molecular weight standards and Cyquant cell proliferation assay kit were from Invitrogen (San Diego, CA). Sulfo-NHS-LC-Biotin was purchase from Pierce (Rockford, IL). TGF-β1 was purchased from R&D systems (Minneapolis, MN). Anti-Flag (M2), anti-HA (HA-7), anti-His (His-1) and anti-pan cadherin antibodies as well as anti-Flag M2 gel beads, Flag peptide and thapsigargin were purchased from Sigma-Aldrich (St Louis, MO). Anti-GRP78 (N-20 and 76-E6), anti-TβRII (C16) and protein G-PLUS-agarose beads were from Santa Cruz (Santa Cruz, CA). Anti GRP78 (KDEL) was from Stressgen bioreagents (Ann Arbor, MI). Anti-phospho-Smad2, anti-TβRI and anti-pan actin were purchased from Cell Signaling (Danvers, MA). The p26-Flag expression construct was a generous gift from Kuo Fen Lee (Peptide Biology Laboratories, Salk Institute). Antibodies directed against Cripto (6900) were raised against a peptide spanning mouse Cripto amino acids 81-97 and cyclizcd between Cys 81 and Cys 90. Smad2 antisera were raised against a peptide conserved between Smad2 and Smad3 spanning amino acids 159-175 of human Smad3. Polyclonal antisera targeting the Flag epitope (6643) were raised against a 2X Flag peptide. Rabbit polyclonal anti-Cripto, anti-Smad2/3 and anti-Flag antisera were produced by Joan Vaughan (Peptide Biology Laboratories, Salk Institute).

Recombinant human activin-A was generated using a stable activin-A-expressing cell line generously provided by Dr. J. Mather (Genentech, Inc.) and was purified by Wolfgang Fischer (Peptide Biology Laboratory, Salk institute). Recombinant mouse Nodal, human TGF-β1, human activin-B and mouse Cripto were purchased from R&D Systems (Minneapolis, MN). Protein A- and G-agarose and the phosphoinositide 3-kinase (LY294002) and MAPK or Erk kinase (PD98059) inhibitors were purchased from Calbiochem (San Diego, CA). ¹²⁵I-Cripto was prepared using the chloramine T method as described previously (Vaughan, 1993). A polyclonal anti-Cripto antibody (6900) was produced in rabbits immunized with a peptide from the epidermal growth factor-like domain of Cripto (⁸²CPPSFYGRNCEHDVRKE⁹⁸ (SEQ ID NO:1)). Goat IgG, anti-GRP78 (N-20) and anti-phospho-tyrosine (PY99) were purchased from Santa Cruz Biotechnology, Inc. (Santa Cruz, CA). Anti-phospho-Smad2, anti-Smad2/3, anti-pan-actin, anti-phospho-Akt, anti-Akt, anti-phospho-GSK3b, anti-phospho-Erk1/2, anti-Erk1/2, anti-phospho-Src (Y416), and anti-Src were purchased from Cell Signaling Technologies, Inc. (Danvers, MA). Anti-HA, anti-Flag (M2) and anti-Flag (M2) agarose were purchased from Sigma (St. Louis, MO). Horseradish peroxidase-linked anti-mouse, anti-goat, anti-rabbit IgG, 3,3',5'5-tetramethlbenzidine substrate, chemiluminescent substrate (Supersignal™), and the BCA protein assay kit were obtained from Pierce (Rockford, IL).

**Expression constructs, cell lines and transient transfection.** The wild type and mutant mouse Cripto-Flag expression constructs have been previously described *(Gray et al.,* 2006). Cripto constructs were also generated in the lentiviral vector pCSC (Miyoshi *et al*., 1998) for production of lentivirus. Lentiviral vectors used in this study were a generous gift from Inder Verma (Salk Institute). The TβRI-HA and TβRII-His expression constructs were a gift from Joan Massagué (Memorial Sloan-Kettering Cancer Center, New York). Standard PCR techniques were used to generate the human GRP78 construct with a hemagluttinin (HA) epitope at its C terminus. 293T cells, P19 cells and HeLa cells were grown in DMEM and PC3 cells were grown in Φ-K12 media. Media were supplemented with 10% fetal calf serum (293T, HeLa and PC3) or 7.5% fetal calf serum (P19) together with penicillin, streptomycin and L-glutamine. For transient transfection, 293T cells were plated on polylysine-coated 15 cm plates (∼10⁷ cells/ plate) and then transfected the following day at ∼40-60% confluence using the PEI transfection reagent as previously described (Harrison *et al*., 2004)*.*

The following culture protocols were used in Example 3. HEK 293T cells were grown in Dulbecco's modified Eagle's medium, and NCCIT cells were grown in RPMI 1640. Both media were supplemented with 10% fetal bovine serum, penicillin, streptomycin, and L-glutamine. MCF10A cells were grown in Dulbecco's modified Eagle's medium - F-12 (50:50) supplemented with 5% donor horse serum, 20 ng/mL EGF, 10 µg/mL insulin, 0.5 µg/mL hydrocortisone, and 100 ng/mL cholera toxin.

For transient transfection, cells were plated at densities between 40 and 60% confluence and Lipofectamine 2000 (Invitrogen) was used for NCCIT cells and Perfectin (Gene Therapy Systems) was used for 293T cells according to manufacturer's instructions. For viral transduction, lentivirus was produced as previously described (Miyoshi *et al.,* 1998). An appropriate dilution of virus-containing media to obtain a multiplicity of infection of 3 to 5 was used to generate pools of cells containing the delivery vector, and the efficiency of infection was determined by monitoring green fluorescent protein expression in infected cells by use of fluorescence microscopy.

**Mass spectrometric analysis.** Mass-specific bands were excised from a Coomassie Blue stained gel. Gel slices were further de-stained by treatment with 40% aqueous n-propanol and 50% aqueous acetonitrile. To the de-stained gel slice, 100 ng trypsin was added in 10 µl ammonium bicarbonate solution (20 mM). Digestion was allowed to proceed at 37 °C for 16 h. One microliter of the supernatant was spotted onto a MALDI target and mixed with 1 µl of a saturated solution of alpha-cyano-hydroxycinnamic acid. After drying, the sample was analyzed on a Bruker Ultraflex TOF/TOF (Bruker Daltonics, Billerica, MA) in positive reflected TOF mode. Mass fingerprint data were analyzed using the Mascot algorithm (Matrix Science, London, UK).

**Fluorescence imaging.** 293T cells (35,000 per well) and P19 cells (500,000 per well were plated in 12 well plates and grown overnight on cover slips pretreated with polylysine. Cells were washed with KPBS, fixed in 4% paraformaldehyde and then permeabilized in buffer A (KPBS supplemented with 2% donkey serum and 0.2% Triton X-100). 293T cells were treated with rabbit anti-Cripto (6900; 1:600) and goat anti-GRP78 (N-20 sc-1050; 1:400) while P19 cells were treated with the same anti-Cripto (6900; 1:600) and anti-GRP78 (N-20 sc-1050 1:125) antibodies together with mouse anti-pan-cadherin (C1821, Sigma, 1/125) in buffer A for 48 hours at 4°C. Cells were washed with KPBS and then treated with anti-rabbit, anti-goat and anti-mouse respectively for one hour at room temperature in buffer A. After further washing in KPBS, the cover slips were mounted in the presence of DAPI and then subjected to fluorescence visualization. For confocal images, a Leica TCS SP2 AOBS confocal system (Leica, Wetzlar, Germany) was used. Images were collected using sequential scanning of each excited wavelength to avoid any bleed through between flourophores.

**Design of lentiviral shRNA vectors and infection of cell lines.** Target sequences within the human GRP78 gene were identified and selected using the S-fold program (http://sfold.wadsworth.org/sirna.pl). The design of short hairpin RNA (shRNA) and production of lentiviral shRNA vectors was carried out as previously described (Singer *et al*., 2005). The 83-mer used to generate the GRP78 1 (G1) shRNA was as follows: 5'-CTGTCTAGACAAAAAACCATACATTCAAGTTGATTCTCTTGAA ATCAACTTGAATGTATGGTCGGGGATCTGTGGTCTCATACA -3' (SEQ ID NO:2). For viral transduction, lentivirus was produced as previously described (Miyoshi *et al*., 1998).

The wild-type Cripto-Flag expression constructs have previously been described (Gray et *al*., 2006). Standard PCR techniques were used to generate the human GRP78 construct with an HA epitope at its C-terminus. The Δ19-68 GRP78-HA construct was generated using PCR techniques, as previously described (Harrison, CA 2003 JBC). The Cripto construct was also generated in the lentiviral vector pCSC (Miyoshi HU 1998 J Virol) for the production of lintivirus and infection of cell lines. Target sequences within the human Cripto or GRP78 genes were identified and selected using the Sfold program (http://sfoldwadsworth.org/sirna.pl). The design of short hairpin RNA (shRNA) and production of lentiviral shRNA vectors were carried out essentially as previously described (Singer O 2005 Nat Neuro). Briefly, an 83-mer oligonucleotide containing the human Cripto or GRP78 shRNA sequence and a T3 oligonucleotide (5'-CTCGAAATTAACCCTCACTAAAGGG-3' (SEQ ID NO:3)) were used to PCR amplify a fragment which was then subcloned into the lentiviral vector in which shRNA expression is driven by an H1 promoter (Singer O 2005 Nat Neuro). The 83-mers used to generate the Cripto shRNA and GRP78 shRNA vectors were 5'-CTGTCTAGACAAAAACAATGACTCTGAATTAAAGTCTCTTGAACTTTAATTCAGA GTCATTGCGGGGATCTGTGGTCTCATACA-3' (SEQ ID NO:4) and 5'-CTGTCTAGACAAAAAACCATACATTCAAGTTGATTCTCTTGAAATCAACTTG A ATGTATGGTCGGGGATCTGTGGTCTCATACA-3' (SEQ ID NO:5), respectively, and have been previously validated (Gray *et al*., 2006; Shani *et al*., 2008).

**Cell lysates and immunoprecipitations.** Cell lysates were prepared in RIPA buffer as previously described *(Gray et al.,* 2006). For immunoprecipitation experiments, 1-5 mg protein extract was pre-cleared by protein G-PLUS-Agarose beads for 2 hours at 4°C. The pre-cleared extracts were incubated as indicated with 40 µl anti-FLAG M2 gel beads or 20 µl G-PLUS-Agarose pre-incubated with 15 µl anti-GRP78 (KDEL), 10 µl anti-HA, or 25 µl anti-His for 2 hours at 4°C. Immunoprecipitates were subsequently washed 4 times with RIPA buffer and 2 times with 54K buffer (50mM tris pH 7.9, 150 mM NaCl, 0.5% Triton X-100). The proteins were then eluted either by heating the beads at 95°C in sample buffer or by the addition of 50 µl of Flag peptide (1 µg/µl) followed by removal of any remaining associated proteins by heating in sample buffer. Western blotting

**Cell Surface Biotinylation.** Sulfo-NHS-LC-Biotin was prepared fresh at 0.5 mg/ml in HDB and then stored on ice until used. Adherent, intact cells were rinsed twice with ice cold HDB and then incubated with biotin solution 30 min on ice using sufficient volume to completely cover the cells *(e.g.* 1 ml/well for 6-well plates). The biotinylation reaction was then quenched following the addition of 1 M Tris pH 7.5 to bring the biotin/HDB solution to a concentration of 50 mM Tris final. The resulting solution was removed and the cells were rinsed one time in HDB containing 50 mM Tris. Cells were then solubilized in RIPA buffer (50 mM TrisHCl, pH 7.4/150 mM NaCl/1% NP40/0.5% deoxycholate/0.1% SDS) supplemented with standard protease inhibitors. Biotinylated proteins were separated by SDS PAGE, blotted to nitrocellulose and then visualized following treatment with avidin-HRP and ECL.

**Cell death assays.** For each cell population, three fields, each consisting of at least 100 GFP-positive cells, were scored for apoptotic cells according to their morphology. The number of cells determined to be apoptotic was divided by the total number of GFP-positive cells in the field resulting in % apoptotic cells.

**Protein Phosphorylation.** Cells were grown to confluence in 24-well plates, rinsed with serum-free media and serum-starved for 4 hours. Appropriate inhibitors or blocking antibodies were added as indicated for 1 hour. Cells were stimulated with the indicated doses of TGF-β ligands for 60 minutes or soluble Cripto for 10 minutes. Cells were harvested by adding 50 µL of ice-cold radioimmune precipitation (RIPA) buffer (50 mM Tris-HCl, pH 7.4, 150 mM NaCl, 1% Nonidet P-40, 0.5% deoxycholate, and 0.1% SDS) supplemented with 20 mM NaF, 500 µM NaPyrophosphate, 1 mM NaOrthothanitate, and standard protease inhibitors. 15 mL of 4X SDS-PAGE loading buffer (with dithiothreitol) were then added to each sample, and the proteins were separated by SDS-PAGE and blotted on nitrocellulose. Blots were treated with anti-phospho-Smad2 (1:500), anti-Smad2/3 (1:1000), anti-phospho-Akt (1:500), anti-Akt (1:500), anti-phospho-GSK3β (1:500), anti-pan-Actin (1:500), anti-phospho-Erk1/2 (1:500), or anti-Erk1/2 (1:500) antibodies, followed by anti-rabbit or mouse IgG conjugated to horseradish peroxidase, and bands were detected using enhanced chemiluminescence.

Smad2 phosphorylation and Western blotting was performed as follows. HeLa cells or PC3 cells stably infected with lentivirus were plated on 6 well plates at a density of 200,000 cells per well. 48 h after plating, cells were washed once with HDB, starved for 4 h in additive-free medium and then left untreated or treated with TGF-β1 for 30 min. Cells were lysed and Smad2 phosphorylation assays were carried out by Western blotting as previously described (Gray *et al.,* 2006).

**Cell proliferation assays and colony formation in soft agar.** PC3 cells were stably infected with pCSC lentivirus constructs as described above. Cells were plated on 96 well plates at a density of 500 cells/well and 24 h later cells were either treated with 10 pM TGF-β1 or left untreated. 8 days after treatment, cell proliferation was measured using the Cyquant Cell Proliferation Kit according to the manufacturer's instructions. To measure colony formation in soft agar, 96 well plates were prepared with 50 µl/well surface layers consisting of 0.6% agar (Nobel) resuspended in PC3 growth medium. An additional 75µl/well of 0.33% agar/PC3 growth medium containing 1,000 stably infected PC3 cells was then added to each well followed by addition of TGF-β1 which was included in varying amounts to yield the specified final concentrations before the agar solidified. Wells were re-fed with 100 µl of PC3 growth medium with or without TGF-β1 for 15 days and then colonies were visualized microscopically and counted. Photographs of specified wells were taken using Canon EOS 400D camera mounted on an inverted Olympus CK40 Microscope set to its lowest magnification.

**Cell Surface Protein Detection.** Intact cells plated in triplicate in 24-well plates were washed with Hepes Dissociation Buffer (HDB), blocked with 3% bovine serum albumin/HDB, and incubated with anti-Cripto (6900; 1:200), anti-GRP78 (N-20; 1:200) or the appropriate negative control primary antibodies for 2 hours at room temperature in 3% bovine serum albumin/HDB. Cells were washed with HDB and incubated with the appropriate peroxidase-conjugated secondary antibody. Specific antibody staining was measured using the 3'3',5'5-tetramethylbenzidine peroxidase substrate, as previously described (Gray *et al*., 2000; Kelber JA et al 2008 J. Biol. Chem. 283(8) 4490-500).

**Smad2-Dependent Luciferase Activity.** Luciferase assays were carried out using the A3-luciferase reporter as previously described (Gray *et al*., 2003). The A3-luciferase reporter construct contains three copies of the activin response element from the *Xenopus laevis* Mix.2 promoter linked to a basic TATA box and a luciferase reporter gene. NCCIT cells were plated on poly-D-lysine-coated 24-well plates at 1x10⁵ cells/well and transfected (Lipofectamine 2000) in triplicate ∼24 hours later with 1.2 µg of DNA/well using 200 ng of A3-luciferase, 400 ng of FAST2 (FoxH1), 400 ng of CMV-β-galactosidase, and 200 ng of empty vector (pcDNA 3.0). Cells were treated -24 hours following trasfection and then harvested ∼16 hours following treatment. Cells were incubated in solubilization buffer (1% Triton-X-100, 25 mM glycylglycine (pH 7.8), 15 mM MgSO₄, 4 mM EGTA, and 1 mM dithiothreitol) for 30 minutes on ice, and luciferase reporter activity was measured and normalize relative to CMV-β-galactosidase.

**Co-immunoprecipitation.** 1x10⁶ HEK 293T cells were plated in 10-cm plates. 24 hours later, cells were transfected with 12 µg of DNA/plate (6 µg of Vector and 6µg of Cripto-Flag, WT GRP78-HA, Δ19-68 GRP78-HA or 6 µg Cripto-Flag and 6 µg WT GRP78-HA or Δ19-68 GRP78-HA) using Perfectin and cells were incubated another 48 hours before harvesting. Cells were lysed and scraped on ice in 0.8 mL of cold RIPA buffer containing standard protease inhibitors. Cellular lysates were precleared by centrifugation, and 75 µL of the total lysates were heated and frozen in 4X SDS-PAGE loading buffer (with dithiothreitol), whereas the remainder of the lysates were incubated overnight at 4 °C with HA- or Flag(M2)-agarose. Precipitated complexes were washed three times with cold RIPA buffer for 1 hour each, eluted from beads, and then analyzed by SDS-PAGE and electrotransfer to nitrocellulose, followed by Western blotting using anti-Flag, HA or GRP78 (N-20) antibodies.

**Cell Proliferation.** Cells were plated on 96-well plates at a density of 500 (NCCIT) or 200 (MCF10A) cells/well. 24 hours later, cells were either treated with indicated combinations of blocking agents (goat IgG or anti-GRP78 (N-20)) and growth factors or left untreated in quadruplicate. Eight days after treatment, cell proliferation was measured using the CyQUANT cell proliferation kit according to the manufacturer's instructions.

**Cell Surface Cripto Binding.** Cells were plated at 4x10⁵ cells/well in 24-well plates coated with poly-D-lysine. 16 hours after plating the cells were binding was carried out in the wells at room temperature on intact cells. Cells were washed in Hepes Dissociation Buffer (HDB) (12.5 mM Hepes (pH 7.4), 140 mM NaCl and 5 mM KCl) and then 200 µl was added to each well: 200 µl of binding buffer (HDB with 0.1% bovine serum albumin, 5 mM MgSO₄, 1.5 mM CaCl2), 10 µl of unlabeled competitor (25.0 µg/mL soluble Cripto) at various dilutions in binding buffer and 40 µl of ¹²⁵I-Cripto (1x10⁶ cpm/well). Plates were incubated for 2 h at room temperature and then wells were rinsed in HDB, and cells were solubilized in 1% SDS and ¹²⁵I-Cripto from each well was counted using a γ counter.

**E-Cadherin Expression and Cell Adhesion.** MCF10A cells were plated at 4x10⁵ cells/well in 6-well plates. 24 hours later cells were pretreated with goat IgG or anti-GRP78 (N-20) for 1 hour and then treated with 400 ng/mL soluble Cripto or left untreated. 48 hours after treatment, cells were either lysed in cold RIPA buffer containing standard protease inhibitors or analyzed for cell adhesion properties. Cell lysates were analyzed by SDS-PAGE and electrotransfer to nitrocellulose, followed by Western blotting using anti-E-Cadherin or pan-Actin antibodies. To quantitate cell adhesion, ∼2x10⁵ cells/well were plated in quadruplicate onto 96-well plates and incubated for 1 hour at 37 °C. To analyze the total number of cells plated, media was removed from each well and the relative cell number was measured using the CyQUANT cell proliferation kit according to the manufacturer's instructions. Percent adhesion was calculated using the relative cell number measured (CyQUANT) from plates that had been rinsed.

### EXAMPLE 2

### GRP78 and Cripto form a Complex at the Cell Surface and Collaborate to Inhibit TGF-β Signaling and Enhance Cell Growth

Cripto and GRP78 play essential roles during embryogenesis and promote the tumor phenotype. The importance of these proteins in tumor progression is highlighted by the fact that they have each been independently validated as cell surface tumor-specific therapeutic targets *in vivo.* In order to identify novel Cripto interacting proteins, a protein interaction screen using full-length, membrane-anchored Cripto as bait was conducted. This screen led to the identification of GRP78, a multifunctional regulator of ER homeostasis that has also been heavily implicated in cancer. Interestingly, although generally localized to the ER, GRP78 is also selectively expressed at the plasma membrane in tumor cells and data presented herein indicates that Cripto binds GRP78 at the cell surface. The data indicates that they interact in a cell free system in a manner that does not require their association within the ER. Finally, the data indicates that GRP78 and Cripto cooperate to attenuate TGF-β-dependent growth inhibitory effects and increase colony growth of prostate cancer cells in soft agar. Together, the results indicate that these two proteins form a complex at the cell surface and thereby confer a growth advantage to tumor cells via inhibition of TGF-β signaling.

A screen aimed at identifying novel Cripto binding proteins that led to the identification of Glucose Regulated Protein-78 (GRP78), an ER chaperone that promotes protein folding and assembly and co-ordinates the unfolded protein response (UPR). GRP78 is strongly induced under conditions of ER stress such as glucose deprivation and hypoxia and is highly expressed in the tumor microenvironment where these conditions prevail (Lee, 2001; Lee, 2007; Li and Lee, 2006). It was shown that delivery of a HSVTK suicide transgene driven by the GRP78 promoter into breast tumor cells caused complete eradication of sizable tumors in mice (Dong *et al*., 2004). GRP78 has also been heavily implicated in promoting tumor cell survival, chemoresistance and malignancy (Lee, 2007; Li and Lee, 2006). Inhibition of GRP78 induction in fibrosarcoma B/C10ME cells using antisense prevented these cells from forming tumors in nude mice (Jamora *et al*., 1996). Also, while GRP78 heterozygous mice develop normally, they were shown to be resistant to transgene-induced mammary tumor growth due to reduced GRP78 levels (Dong *et al*., 2008). Although it is generally restricted to the lumen of the ER, GRP78 is localized to the plasma membrane of tumor cells where it has receptor function associated with increased cellular proliferation, motility and survival (Pizzo ref) (Lee, 2007; Li and Lee, 2006).

**Identification of GRP78 as a novel Cripto binding protein.** In order to identify new Cripto-associated proteins, the inventors have employed a strategy in which Cripto was used as bait to "pull down" its binding partners at the cellular membrane. The inventors subjected lysates from 293T cells transfected with empty vector or Cripto-Flag to anti-Flag immunoprecipitation followed by specific elution of bound proteins with Flag peptide. Since it was carried out under mild conditions, this elution allowed for an additional purification step. As visualized following silver staining, two proteins migrating at ∼72 kDa and ∼50 kDa specifically co-precipitated with Cripto-Flag (FIG. 1A, labeled a and b, respectively).

The inventors next sought to identify these Cripto-associated proteins using mass spectrometry. The ∼72 kDa and ∼50 kDa bands were excised from the gel, further de-stained and subjected to in-gel trypsin digestion. Samples were then analyzed by MALDI/TOF and Mass fingerprint data were characterized using the Mascot algorithm (Matrix Science, London, UK). The top hit score of 55 for the band at approximately 72 kDA was for GRP78, also known as BiP, and a total of 7 peptides could be assigned in the mass fingerprint with a peptide mass error tolerance of <0.1 Da (FIG. 1B). The band at ∼50 kDa (Protein b) has not yet been conclusively identified.

GRP78 has multiple functions including a prominent role in mediating protein folding and the stress response in the ER (Lee, 2001). GRP78 has also been heavily implicated in tumorigenesis (Lee, 2007) and in the present study the inventors focused on its potential role in binding Cripto and modulating its function. To unequivocally validate the identity of GRP78 as a specific Cripto binding partner, the inventors repeated the co-immunoprecipitation procedure described above and subjected precipitated proteins to Western blotting using specific anti-GRP78 or anti-Cripto antibodies. As shown in FIG. 1C, GRP78 is present in the Flag peptide elute indicating it specifically co-immunoprecipitates with Cripto.

**GRP78 binds Cripto at the cell surface.** Although GRP78 is thought to function primarily as an ER-associated protein (Lee, 2001), several recent studies have demonstrated that GRP78 is also expressed at the plasma membrane of cancer cells under certain conditions (Lee, 2007). In order to test whether Cripto and GRP78 interact at the cell surface, the inventors labeled intact cells with a cell impermeable biotin reagent and subjected resulting cell lysates to anti-Flag immunoprecipitation followed by elution with Flag peptide and detection of biotinylated proteins with avidin. As a negative control, the inventors used a ∼26 kDa transmembrane fragment of the p75 neurotrophin receptor referred to as p26-Flag. This irrelevant protein is similar in size to Cripto and was subjected to the same procedure, side by side with Cripto-Flag. As shown in FIG. 2A, biotinylated forms of GRP78 and protein b co-immunoprecipitated with Cripto but not with p26 suggesting the association of Cripto with GRP78 and protein b is specific. The fact that GRP78 and Cripto were biotinylated indicates that they interact at the cell surface and diminishes the likelihood that their association depends on the chaperone activity of GRP78.

To further characterize the ability of Cripto to bind cell surface GRP78, the inventors tested whether GRP78 from one population of cells could bind mature Cripto isolated from a separate cell population in a cell free system. 293T cells were infected with vector or Cripto-Flag and then subjected to anti-Flag immunoprecipitation followed by extensive washing of the beads. In parallel, a separate population of 293T cells infected with GRP78 was subjected to cell surface biotinylation and lysates from these cells were incubated with the beads previously incubated with vector or Cripto-Flag lysates. The beads were then washed again and bound proteins were eluted with Flag peptide and subjected to Western blotting using avidin-HRP, anti-GRP78 or anti-Cripto antibodies. As shown in FIG. 2B, Flag peptide specifically eluted Cripto-Flag together with the majority of bound GRP78. Moreover, biotinylated (*i*.*e*. cell surface-labeled) GRP78 was specifically eluted from beads previously exposed to Cripto-Flag lysates but not from beads exposed to vector lysates. These results demonstrate that the interaction between cell surface-derived GRP78 and Cripto occurs *in vitro* in a manner that is independent of cellular or membranal contexts. In addition, since biotinylated GRP78 and Cripto originated from separate cell populations, their interaction does not depend on prior association in the ER, on the translational machinery or any chaperone function of GRP78.

Cripto possesses two modular domains that mediate protein-protein interactions, an EGF-like domain and a cysteine-rich CFC domain *(Strizzi et al.,* 2005). In order to explore the interaction between Cripto and GRP78 further, the inventors used the method described above (FIG. 2B) to test whether cell surface labeled GRP78 binds Cripto mutants lacking either the EGF-like domain (ΔEGF) or CFC domain (ΔCFC). In this experiment, the inventors assessed the ability of biotinylated, HA-tagged GRP78 from one cell population to bind wild type and mutant forms of Cripto originating from separate cell populations. As shown in FIG. 2C, cell surface GRP78 bound wild type Cripto and the Cripto ΔEGF mutant to similar extents but did not bind the Cripto ΔCFC mutant. Therefore, this result indicates that cell surface-derived GRP78 binds the CFC domain of Cripto.

Having shown that overexpressed Cripto and GRP78 bind at the cell surface in a specific manner that depends on the CFC domain of Cripto, the inventors next tested whether these two proteins are associated in an endogenous setting. Embryonal carcinoma cell lines were reported to express high levels of Cripto protein and the inventors tested whether endogenous Cripto and endogenous GRP78 interact in mouse embryonal carcinoma P19 cells. The inventors treated these cells with membrane-impermeable biotin as described above and subjected lysates to immunoprecipitation with anti-Cripto antibody or rabbit IgG as a negative control. As a positive control for the immunoprecipitation, the inventors used 293T cells infected with empty vector or Cripto-Flag. As shown in FIG. 2D, anti-Cripto immunoprecipitation from P19 cells followed by anti-GRP78 Western blotting led to the detection of a band corresponding to GRP78 while precipitation with non-immune IgG failed to do so. Furthermore, the precipitated Cripto and GRP78 proteins were biotinylated as indicated by their detection with avidin-HRP indicating they originated from the cell surface. A similar result was obtained with 293T cells overexpressing Cripto-Flag but not with empty vector cells (FIG. 2D, right panel) validating the specificity of the anti-Cripto antibody. Therefore, endogenous Cripto and endogenous GRP78 specifically interact at the cell surface of mouse embryonal carcinoma P19 cells and their interaction does not require the overexpression of either protein.

**Cripto and GRP78 co-localize at the cell surface.** The cellular localization of these proteins was assessed by immunofluorescence and confocal microscopy. Initially, 293T cells infected with empty vector or co-infected with Cripto and GRP78 were stained with anti-Cripto and anti-GRP78 antibodies. In these studies, vector cells displayed minimal, background level Cripto staining and weak GRP78 staining resulting from the presence of the endogenous protein. By contrast, 293T cells overexpressing Cripto and GRP78 gave rise to prominent, punctate staining of both proteins with striking co-localization at the cell surface. Although the significance of the punctate structures remains to be determined, this result clearly demonstrates the association of overexpressed Cripto and GRP78 at the plasma membrane of intact 293T cells.

Next, the inventors tested whether Cripto and GRP78 are similarly associated at the cell surface when expressed at endogenous levels in P19 cells. Here, both Cripto and GRP78 were readily detected in native P19 cells. These cells were also stained with an anti-pan-cadherin antibody that was used as a marker of the plasma membrane. Overall, the staining for Cripto and GRP78 appeared to be predominantly punctate/vesicular in nature with partial but substantial co-localization. Importantly, several of the punctate structures containing both Cripto and GRP78 displayed overlapping staining with pan-cadherin placing them at the plasma membrane of these cells. The co-localization of Cripto and GRP78 both at the membrane and within vesicular structures suggests that they associate not only at the plasma membrane but also during the endosomal/lysosomal trafficking and recycling commonly associated with cell surface signaling proteins.

**Cripto-associated GRP78 can be targeted using shRNA.** Having demonstrated that Cripto and GRP78 are associated co-factors at the cell surface, the inventors next aimed to determine whether GRP78 modulates known Cripto functions. To this end, the inventors developed short hairpin RNAs (shRNAs) capable of reducing endogenous GRP78 expression. GRP78 is induced by thapsigargin, a compound that raises cytosolic calcium levels, causes ER stress and triggers apoptosis. Following thapsigargin treatment, GRP78 induction alleviates ER stress and delays the cellular apoptotic response (Jamora *et al*., 1996). Therefore, the inventors initially tested the ability of an shRNA targeting GRP78 (G1) to prevent the induction and function of GRP78 following thapsigargin treatment. As shown by Western blot using anti-GRP78 antibody, thapsigargin clearly induced GRP78 expression in HeLa cells and this induction was blocked by the G1 shRNA (FIG. 3A). Furthermore, as shown in FIG. 3B, G1 infected HeLa cells showed a marked increase in thapsigargin-induced apoptosis in comparison to vector cells demonstrating the functional consequences of GRP78 knockdown by this shRNA.

The inventors next aimed to examine whether the G1 shRNA could similarly target the cell surface pool of GRP78 associated with Cripto. HeLa cells infected with empty vector or G1 shRNA were subsequently infected with Cripto-Flag. These cells were then subjected to cell surface biotinylation followed by anti-Flag immunoprecipitation and specific elution with Flag peptide as previously described. Eluted proteins were subsequently analyzed by Western blot using avidin-HRP, anti-GRP78 or anti-Cripto antibodies. As shown in FIG. 3C, the amount of cell surface biotinylated GRP78 that co-immunoprecipitated with Cripto was substantially reduced in the presence of the G1 shRNA construct. Importantly, this result indicates that the G1 shRNA can disrupt functions of GRP78-Cripto complexes at the cell surface.

**Targeted reduction of GRP78 expression enhances TGF-β dependent Smad2 phosphorylation.** The inventors have previously demonstrated that shRNA knockdown of endogenous Cripto in HeLa cells causes an increase in TGF-β-induced Smad2 phosphorylation *(Gray et al.,* 2006). Here the inventors have shown that GRP78 and Cripto interact at the cell surface, raising the possibility that Cripto and GRP78 work in concert to inhibit TGF-β signaling. Having demonstrated that the G1 shRNA effectively targets the Cripto-associated pool of GRP78 at the plasma membrane, the inventors next tested whether it, similar to the Cripto shRNA, could enhance TGF-β signaling. Once again, the same HeLa cells infected with empty vector or G1 were tested in the absence (FIG. 4A) or presence (FIG. 4B) of 5 µM thapsigargin. In each case, cells were treated with a range of TGF-β1 doses and resulting levels of phospho-Smad2 and total Smad2 were monitored. As shown in FIG. 4A, G1 shRNA cells were more responsive to TGF-β than vector infected cells at lower doses *(e.g.,* 1 pM TGF-β1). Following thapsigargin treatment, the TGF-β dose response relationship was shifted substantially to the right (FIG. 4B). Again, cells infected with G1 had a greater sensitivity to TGF-β with a prominent phospho-Smad2 band detected at 10 pM TGF-β1. Interestingly, the G1 shRNA effect of sensitizing cells to TGF-β was more pronounced in the presence of thapsigargin than in its absence. This suggests that induction of cell surface GRP78 by thapsigargin causes inhibition of TGF-β signaling that can be blocked by the G1 shRNA construct.

To test whether thapsigargin causes induction of cell surface GRP78 that is targeted by G1, the same HeLa cells infected with vector or G1 were treated with vehicle or thapsigargin and then subjected to cell surface biotinylation. To visualize biotinylated GRP78, cell lysates were subjected to immunoprecipitation with anti-GRP78 antibody followed by Western blotting with avidin-HRP. As shown in FIG. 4C, thapsigargin treatment induced GRP78 at the cell surface and this induction was blocked by the G1 shRNA construct. Finally, as an additional control, the inventors tested whether GRP78 knockdown or induction results in altered levels of type I and/or type II TGF-β signaling receptors. As shown in FIG. 4D, neither the presence of G1 shRNA nor thapsigargin treatment significantly affected receptor levels with one exception being that TβRI levels were slightly higher in vector cells than in G1 cells in the absence of thapsigargin. This discrepancy did not correlate with TGF-β signaling, however, since phospho-Smad2 levels were higher in G1 cells than in vector cells (FIG. 4A). Thus, GRP78 does not appear to affect TGF-β signaling by altering the levels of these receptors. In summary, these data indicate for the first time that, similar to endogenous Cripto, endogenous GRP78 inhibits TGF-β signaling. Furthermore, these findings are consistent with a novel role for cell surface GRP78-Cripto complexes in blocking TGF-β signaling.

**GRP78 does not directly bind TGF-β signaling receptors.** The finding that GRP78 inhibits TGF-β signaling raised the possibility that it does so by binding directly to type I and/or type II TGF-β signaling receptors. The inventors have shown above that endogenous cell surface GRP78 co-immunoprecipitates with Cripto both when Cripto is overexpressed in 293T cells and with endogenous Cripto in P19 cells. Here the inventors have further tested whether GRP78 similarly co-immunoprecipitates with TβRI and TβRII. 293T cells were transfected with p26-Flag, Cripto-Flag, TβRI-HA or TβRII-His and surface proteins were biotinylated as before. Each of these proteins was immunoprecipitated as bait and then immune complexes were assessed for the presence of GRP78. As shown in FIG. 5, the avidin-HRP panel reflects the fact that similar amounts of these different cell surface bait proteins were precipitated in these pull downs. However, only Cripto pulled down endogenous GRP78 as detected both by avidin-HRP and anti-GRP78 antibody (FIG. 5). Therefore, under these conditions, cell surface GRP78 does not bind TβRI or TβRII but rather appears to associate exclusively with Cripto. This result suggests that the effect of GRP78 on TGF-β signaling is not likely to occur via its direct, independent binding to either signaling receptor.

**Cripto and GRP78 cooperate to inhibit TGF-β signaling.** TGF-β has been shown to inhibit both anchorage dependent and anchorage independent growth of human prostate carcinoma PC3 cells (Wilding *et al*., 1989). Therefore, the inventors tested whether Cripto and GRP78 work together to modify TGF-β effects in these cells. First, the inventors tested the effects of Cripto and GRP78 on TGF-β-dependent Smad2 phosphorylation. As shown in FIG. 6A, treatment of vector infected cells with 10 pM TGF-β1 resulted in Smad2 phosphorylation and this effect was moderately attenuated when GRP78 or Cripto were overexpressed separately. When cells were infected with both Cripto and GRP78, however, the TGF-β effect was inhibited to a much greater extent. The intensities of the phospho-Smad2 bands presented in FIG. 6A were then quantitated and normalized to corresponding total Smad2 levels (FIG. 6B). This quantitation shows that TGF-β signaling was inhibited in cells expressing GRP78 or Cripto by ∼40% and ∼43%, respectively. By contrast, cells co-expressing GRP78 and Cripto together showed a reduction of ∼74% in TGF-β-induced Smad2 phosphorylation. Next, the inventors tested whether overexpression of Cripto and/or GRP78 affected the levels of TGF-β signaling receptors in these cells. As shown in FIG. 6C, the levels of these receptors were not significantly altered (FIG. 6C). Together, these data further support a novel role for GRP78 as a TGF-β antagonist and indicate that Cripto and GRP78 function cooperatively to inhibit TGF-β signaling.

Next, the inventors measured the relative proliferation rates of infected PC3 cell populations in the absence or presence of 10 pM TGF-β1. As shown in FIG. 6D, TGF-β1 treatment of vector infected cells reduced proliferation by ∼58% while treatment of cells expressing GRP78 or Cripto alone reduced proliferation by ∼42% and ∼19%, respectively. Again, when GRP78 and Cripto were expressed together in these cells, they had a stronger effect. Interestingly, TGF-β1 treatment in this case actually resulted in an increase in cellular proliferation of∼31% (FIG. 6C). Importantly, the data presented here demonstrate that while GRP78 and Cripto each attenuate the antiproliferative effects of TGF-β, their co-expression, which allows for their physical interaction, creates conditions that cause TGF-β to enhance cellular growth.

**GRP78 and Cripto collaborate to block the antiproliferative effects of TGF-β.** Finally, the inventors tested the effects of GRP78 and Cripto on anchorage independent growth in the presence or absence of TGF-β1. The same PC3 cells infected with empty vector, GRP78, Cripto or both were seeded in soft agar in the presence of escalating doses of TGF-β1 and colonies were allowed to grow for fifteen days. As shown in FIG. 7A, TGF-β1 inhibited colony formation in a dose-dependent manner. In order to highlight the relative effects of TGF-β on each cell population, the same data are also presented as the number of colonies in the presence of TGF-β1 normalized to the number of colonies in its absence (FIG. 7B). Two major conclusions can be drawn from these data. First, in the absence of TGF-β treatment, cells expressing either GRP78 or Cripto formed more colonies than vector cells and this increase was largely enhanced in cells expressing both GRP78 and Cripto (FIG. 7A). Second, while GRP78 and Cripto each have some ability to block the growth inhibitory effects of TGF-β individually, they have a much greater ability to do so when expressed together (FIG. 7B). The extent to which co-expression of GRP78 and Cripto attenuated the growth inhibitory effect of TGF-β is further illustrated by photographs of these colonies. As shown in FIG. 7C, 100 pM TGF-β1 was sufficient to dramatically reduce colony formation of vector infected cells and had a similar but weaker inhibitory effect on cells expressing either Cripto or GRP78 individually. By contrast, cells expressing both GRP78 and Cripto together appeared to be more refractory to the cytostatic effects of TGF-β as illustrated by both the number and size of the colonies. Once again, these data support a cooperative function for GRP78 and Cripto in blocking TGF-β inhibition of anchorage independent growth.

FIG. 7D depicts a model in which GRP78 and Cripto function as cell surface binding partners to restrict TGF-β-dependent growth inhibition and promote cell proliferation. The data indicate that Cripto and GRP78 carry out these functions in a cooperative manner, presumably as a complex, since they physically interact and since their effects were cooperatively enhanced. However, the data do not completely rule out the possibility that these proteins can partly inhibit TGF-β signaling and cause enhanced proliferation on their own. Finally, in addition to its ability to activate cytostatic signaling, TGF-β itself has been reported to activate survival pathways under certain conditions. This coincides with the observation that TGF-β causes enhanced proliferation of PC3 cells only when they co-express both Cripto and GRP78 (FIG. 7D, dashed arrow).

### EXAMPLE 3

### Cell Surface GRP78 mediates Cripto signaling via activin/Nodal/TGF-β and MAPK/PI3K pathways in stem cells and tumor cells

### Cripto and GRP78 cooperatively regulate activin/Nodal/TGF-β signaling

Cripto and GRP78 each play essential roles during embryonic development and both proteins also promote tumor cell proliferation, survival and metastasis. The above identification of cell surface GRP78 as a Cripto binding partner suggested that these proteins function cooperatively during normal embryonic development and tumor progression. The above example shows that Cripto and GRP78 form a cell surface complex in P19 cells (Shani *et al.,* 2008). Here the inventors have tested whether the interaction between Cripto and GRP78 is required for Cripto modulation of activin/Nodal/TGF-β signaling in NCCIT cells. NCCIT populations were generated infected with empty vector or stably expressing shRNAs targeting Cripto and/or GRP78. These shRNAs substantially reduced levels of Cripto and GRP78 protein in NCCIT cells as measured by Western blot (FIG. 8A) or intact cell surface ELISA (FIG. 8B) which measures protein levels at the cell surface. Importantly, knockdown of Cripto does not affect cell surface levels of GRP78 and vice versa (FIG. 8B).

The inventors used these cells to test whether knockdown of Cripto and/or GRP78 would affect activin-A- and Nodal-induced Smad2 phosphorylation in these cells. As shown in FIG. 1C, activin-A-induced Smad2 phosphorylation was enhanced by Cripto knockdown. This is consistent with the previous demonstration that Cripto inhibits activin-A signaling *(Gray et al.,* 2003; Kelber *et al*., 2008) and provides the first demonstration that endogenous Cripto functions as an activin antagonist. Interestingly, activin-A-dependent Smad2 phosphorylation was similarly enhanced in GRP78 knockdown cells and in cells in which both Cripto and GRP78 were knocked down, consistent with a role for both of these proteins in modulating activin-A signaling. In contrast to what was observed with activin-A, Cripto knockdown dramatically reduced Nodal-induced Smad2 phosphorylation while GRP78 knockdown modestly reduced Nodal signaling (FIG. 8D). Knockdown of Cripto and GRP78 together resulted in Nodal-induced Smad2 phosphorylation that was less than that of Cripto knockdown alone (FIG. 8D). Together, these results suggest that GRP78 facilitates the opposing effects of endogenous Cripto on activin-A and Nodal signaling.

Next, the inventors used the same cells to test whether Cripto and/or GRP78 knockdown would affect activin/Nodal/TGF-β induction of a Smad2-responsive luciferase reporter construct. As shown in FIG. 8E, treatment of empty vector-infected NCCIT cells with activin-A, activin-B, TGF-β1 or Nodal resulted in similar low levels of luciferase induction. Cripto knockdown resulted in enhanced activin-A, activin-B and TGF-β1 signaling and reduced Nodal signaling. A similar result was observed in cells stably expressing GRP78 shRNA consistent with a role for GRP78 in Cripto-dependent modulation of signaling by these ligands. By contrast, knockdown of both Cripto and GRP78 resulted in a dramatically increased response to activin-A, activin-B and TGF-β1 together with a loss of detectable Nodal signaling (FIG. 8E). The inventors further explored the requirement of GRP78 for Cripto-dependent Nodal signaling using the same Smad2-dependent luciferase reporter in 293T cells. As shown in FIG. 8F, Cripto overepxression facilitates Nodal signaling in these cells and this signaling is enhanced in the presence of overexpressed GRP78. Furthermore, as shown in FIG. 8G, Cripto-dependent Nodal signaling is attenuated in these cells when endogenous GRP78 is knocked down. Together with the Smad2 phosphorylation data above, these results strongly support a collaborative role for Cripto and GRP78 in regulating activin/Nodal/TGF-β signaling.

### GRP78 co-localizes with Cripto and mediates Cripto signaling in human ES cells.

Cripto is expressed in human embryonic stem (hES) cells where it has been shown to have key roles in regulating proliferation, differentiation and pluripotency. Here, the inventors have tested whether GRP78 co-localizes with Cripto and regulates Cripto function in hES cells. As shown in FIG. 9A, GRP78 and Cripto are both expressed at the surface of H9 hES cells as measured by cell surface ELISA. The inventors subjected H9 cells to immunostaining with Cripto and GRP78 antibodies to test whether these proteins co-localize at the plasma membrane and, as shown in FIG. 9B, Cripto and GRP78 each displayed punctate staining near the periphery of the cell. Importantly, the staining for these proteins displayed a high degree of overlap indicating they are co-localized at the cell surface (FIG. 9B). Next, the inventors tested whether an anti-GRP78 antibody could block Cripto-dependent effects on activin-A and Nodal signaling. This antibody (N-20, Santa Cruz) binds cell surface GRP78 in H9 cells (FIG. 9A) and has been reported to block GRP78 receptor function (Davidson *et al*., 2005; Philippova *et al*., 2008). As shown in FIG. 9C, treatment of H9 cells with the N-20 antibody increased activin-A-induced Smad2 phosphorylation and decreases Nodal-induced Smad2 phosphorylation suggesting it blocked the ability of Cripto to affect signaling by these ligands. Consistent with this, treatment of empty vector-infected NCCIT cells with the N-20 antibody increased activin-A, activin-B and TGF-β1 signaling and decreased Nodal signaling (FIG. 9D), while the antibody treatment of Cripto knockdown cells had no effect on the signaling of these ligands (FIG. 9E). Together, these results demonstrate that cell surface GRP78 mediates Cripto signaling in hES cells and that, similar to GRP78 knockdown, targeting GRP78 with the N-20 antibody blocks Cripto-dependent regulation of activin/Nodal/TGF-β signaling.

The results raise the possibility that Cripto and the N-20 antibody compete for binding to GRP78. Since the N-20 antibody targets an epitope within the first 50 amino acids of GRP78, the inventors generated a GRP78 mutant (Δ19-68 GRP78) lacking this region and tested its ability to bind Cripto. FIG. 9F illustrates the position of the N-20 epitope and the Δ19-68 GRP78 mutant in which it is deleted. When lysates from 293T cells overexpressing these proteins were subjected to Western blot using N-20 or HA antibody, the N-20 antibody detected wild type GRP78 but not the Δ19-68 GRP78 mutant in which the N-20 epitope was deleted (FIG. 9F). Importantly, as shown in FIG. 9G, while wild type GRP78 co-immunoprecipitated with Cripto the Δ19-68 GRP78 mutant does not indicating the N-20 antibody and Cripto share a binding site on GRP78 and suggesting they compete for GRP78 binding.

### Targeting GRP78 receptor function blocks Cripto-dependent MAPK/PI3K signaling and mitogenesis in NCCIT cells

Next, the inventors tested whether cell surface GRP78 mediates soluble Cripto-dependent activation of MAPK/PI3K pathways. First, the inventors tested the effects of Cripto and/or GRP78 knockdown on soluble Cripto-dependent phosphorylation of Akt, GSK3β and ERK1/2 in NCCIT cells. As shown in FIG. 10A, empty vector-infected cells had high basal phospho-Akt levels that were unaffected by increasing soluble Cripto doses. By contrast, basal phospho-Akt levels were undetectable in cells expressing Cripto shRNA and soluble Cripto treatment increased Akt phosphorylation in these cells in a dose-dependent manner (FIG. 10A). By contrast, soluble Cripto-dependent Akt phosphorylation was blocked when GRP78 was knocked and especially when Cripto and GRP78 were knocked down together. Soluble Cripto-dependent GSK3β phosphorylation followed the same pattern as that observed for Akt phosphorylation and was also sharply reduced by GRP78 knockdown (FIG. 10A). Finally, Cripto-induced phosphorylation of Akt and GSK3β was blocked by LY 2940002 and therefore dependent on PI3K activation (FIG. 10A).

The inventors further tested the effects of Cripto and/or GRP78 knockdown on soluble Cripto-dependent phosphorylation of ERK1/2. As shown in FIG. 10B, basal levels of phospho-ERK1/2 were relatively high in empty vector-infected NCCIT cells and soluble Cripto treatment did not increase ERK phosphorylation in these cells. By contrast, phosphorylated ERK1/2 was undetectable in untreated Cripto shRNA cells but treatment of these cells with soluble Cripto caused pronounced phosphorylation of ERK2 (p42). This is consistent with previous results demonstrating that soluble Cripto triggers ERK2 phosphorylation (Kannan *et al*., 1997). Similar to Cripto shRNA cells, basal phospho-ERK levels were low or undetectable in GRP78 knockdown cells and also in cells with both Cripto and GRP78 knocked down (FIG. 10B). However, Cripto treatment of these cells was unable to stimulate ERK phosphorylation, suggesting that GRP78 is required for Cripto-dependent activation of the MAPK pathway and ERK2 phosphorylation. These results are similar to those observed with soluble Cripto-dependent phosphorylation of Akt and GSK3β indicating that GRP78 plays similar roles in mediating soluble Cripto-dependent activtioin of PI3K and MAPK pathways.

Cripto tumor growth factor activity is associated with increased cellular proliferation *(Strizzi et al.,* 2005) and the inventors tested whether soluble Cripto promotes proliferation of NCCIT cells in a GRP78-dependent manner. As shown in FIG. 10C, empty vector-infected NCCIT cells had a high basal proliferation rate that was unaffected by soluble Cripto treatment. By contrast, Cripto knockdown, either alone or in combination with GRP78 knockdown, substantially reduced the proliferation rate of NCCIT cells. Importantly, soluble Cripto treatment substantially increased proliferation of Cripto knockdown cells but had no effect on cells in which Cripto and GRP78 were both knocked down. This result indicates that, similar to Cripto-induced MAPK and PI3K signaling, the pro-proliferative effect of Cripto is GRP78-dependent.

The inventors used the N-20 antibody to directly assess the role of cell surface GRP78 in mediating Cripto growth factor activity. Initially, the inventors tested the ability of this antibody to compete with soluble Cripto for binding to NCCIT cells stably expressing Cripto shRNA. As shown in FIG. 10D, ^{l25}I-Cripto bound to these cells specifically and was displaced in a dose-dependent manner by N-20 antibody but not control IgG. Together with the results presented in FIG. 9G, these data indicate that Cripto and the N-20 antibody directly compete for binding to the same N-terminal site on GRP78. The inventors went on to test the ability of the N-20 antibody to block Cripto-dependent Akt phosphorylation in NCCIT cells expressing Cripto shRNA. As shown in FIG. 10E, soluble Cripto-dependent Akt phosphorylation in these cells was almost completely blocked by the N-20 antibody. The inventors further asked whether Cripto-dependent proliferation of Cripto knockdown cells could be blocked with the N-20 antibody. Indeed, as shown in FIG. 10F, the N-20 antibody reduced the basal proliferation rate of these cells and, strikingly, it completely blocked the pro-proliferative effect of soluble Cripto treatment. Together, these data indicate that Cripto binding to cell surface GRP78 is required for soluble Cripto-dependent MAPK/PI3K signaling and mitogenic effects in NCCIT cells.

### Cell surface GRP78 mediates Cripto tumor growth factor activity in mammary epithelial cells

Cripto is overexpressed in ∼80% of human breast cancers and promotes the tumor phenotype in mammary epithelial cells *(Strizzi et al.,* 2005). Here the inventors have tested the role of GRP78 in mediating oncogenic Cripto signaling in MCF10A cells, human mammary epithelial cell line that lack endogenous Cripto expression. To conduct these studies, the inventors generated MCF10A cell clones stably infected with empty vector or Cripto. As shown in FIG. 11A, GRP78 is expressed at the surface of empty vector-infected MCF10A cells as measured by cell surface ELISA. In addition, as shown in FIG. 11B, ¹²⁵I-Cripto bound empty vector-infected MCF10A cells specifically and was displaced in a dose-dependent manner by N-20 antibody. Soluble Cripto-dependent activation of ERK/MAPK and PI3K/Akt pathways requires upstream activation of c-Src (Bianco *et al*., 2003) and the inventors tested if the N-20 antibody blocks Cripto-dependent c-Src activation. As shown in FIG. 11C, soluble Cripto caused phosphorylation of c-Src on Y416 in vector-infected MCF10A cells and this was blocked by pre-incubation of the cells with N-20 antibody. Soluble Cripto treatment of these cells also caused Akt phosphorylation (FIG. 11D). Together these data indicate that Cripto binding to cell surface GRP78 on MCF10A cells is required for its ability to activate c-Src/MAPK/PI3K pathways.

Next the inventors tested whether the ability of Cripto to promote the tumor phenotype in MCF10A cells depends on its ability to bind cell surface GRP78. As mentioned above, Cripto is not expressed at detectable levels in vector-infected MCF10A cells but is highly expressed in Cripto-infected cells (FIG. 11E). As shown in FIG. 11F, Cripto overexpression in MCF10A cells caused a dramatic increase in cellular proliferation that was substantially inhibited by treatment with the N-20 GRP78 antibody. Furthermore, as shown in FIG. 11G, treatment of empty vector-infected MCF10A cells with soluble Cripto increased their proliferation in a manner that was completely blocked by co-treatment with the N-20 GRP78 antibody. These data indicate that the pro-proliferative effects of Cripto on MCF10A cells require Cripto binding to cell surface GRP78.

Cripto overexpression causes migration and invasion of mammary epithelial cells and promotes EMT *(Strizzi et al.,* 2004). Loss of E-Cadherin expression is a hallmark of EMT and the inventors have tested whether cell surface GRP78 mediates Cripto-dependent downregulation of E-Cadherin in human mammary epithelial MCF10A cells. As shown in FIG. 11H, E-Cadherin expression was reduced following treatment of empty vector-infected MCF10A cells with soluble Cripto and was undetectable in Cripto overexpressing cells. However, treatment with N-20 GRP78 antibody reversed the soluble Cripto effect on E-Cadherin levels in vector cells and dramatically rescued E-Cadherin expression in Cripto overexpressing cells (FIG. 11H). Importantly, the rescue of E-Cadherin expression in Cripto-overexpressing cells by the N-20 GRP78 antibody was almost completely blocked by soluble Cripto indicating Cripto and the N-20 antibody compete functionally for binding to cell surface GRP78. E-cadherin mediates cell-cell adhesion in epithelial cells and its loss is associated with invasive, metastatic cancer. Therefore the inventors measured cell adhesion in MCF10A cells stably infected with empty vector or Cripto and tested for effects of the N-20 antibody. As shown in FIG. 11I, cell adhesion was reduced by ∼50% in Cripto overexpressing cells relative to vector cells, consistent with the ability of Cripto to cause down regulation of E-Cadherin. While the N-20 antibody had no effect on adhesion of vector-infected cells, it completely blocked the Cripto-dependent decrease in cell adhesion implicating GRP78 in this effect (FIG. 11I). Together, these data demonstrate that Cripto binding to cell surface GRP78 mediates Cripto tumor growth factor activity including its ability to promote cell proliferation, decrease E-Cadherin expression and decrease cell adhesion.

### The cell surface interaction between Cripto and GRP78 mediates pro-proliferative effects of activin and Nodal

The inventors have provided evidence that cell surface Cripto/GRP78 complexes regulate activin/Nodal/TGF-β signaling, and here the inventors have tested how Cripto and GRP78 coordinately affect activin-A- and Nodal-induced effects on cellular proliferation. As shown in FIG. 12A, activin-A and Nodal both increase proliferation of empty vector-infected NCCIT cells. Knockdown of Cripto and/or GRP78 blocked the pro-proliferative effects of these ligands and, interestingly, caused activin-A but not Nodal to inhibit cellular proliferation. The inventors tested whether the N-20 antibody would have effects similar to those of Cripto and/or GRP78 knockdown. Indeed, as shown in FIG. 12B, the pro-proliferative effects of activin-A and Nodal were blocked in the presence of the N-20 antibody and activin-A again switched from having pro-proliferative effects to having cytostatic effects. Next, the inventors tested whether Cripto and GRP78 similarly affect the proliferative effects of activin-A and Nodal on MCF10A cells. As shown in FIG. 12C, activin-A substantially inhibited proliferation of MCF10A cells stably infected with empty vector while Nodal had no effect. MCF10A cells overexpressing Cripto had an increased rate of proliferation relative to empty vector cells and were no longer growth-inhibited by activin-A. Rather, activin-A and Nodal each increased proliferation of these cells (FIG. 12C). As shown in FIG. 12D, the ability of Cripto overexpression to cause a pro-proliferative response to activin-A and Nodal was completely blocked by treatment of cells with the N-20 antibody and this treatment also caused activin-A to inhibit proliferation of MCF10A cells. Thus, the cell surface interaction between Cripto and GRP78 converts activin-A and Nodal into pro-proliferative cytokines and reverses the cytostatic effects of activin-A.

### A GRP78 mutant lacking Cripto binding inhibits Cripto signaling via EGF receptors and PI3K

The inventors hypothesized that cell surface GRP78 facilitates Cripto signaling via EGF receptors and tested the roles of GRP78, ErbB4 and ErbB2 in mediating Cripto-dependent activation of PI3K. To investigate this hypothesis, the inventors used a PI3K-repressible reporter construct consisting of the glucose-6-phosphatase promoter coupled to a luciferase gene (G6Pase-Lux). Cripto treatment had little or no effect on the activity of this reporter construct in cells transfected with either ErbB2 or ErbB4 but reduced luciferase expression by ∼50% in cells transfected with ErbB2 and ErbB4 together (FIG. 13A). By contrast, in the presence of transfected GRP78, Cripto caused a ∼30% reduction in luciferase expression in cells transfected with ErbB4 and almost completely blocked luciferase expression in cells transfected with both ErbB4 and ErbB2 (FIG. 13B). This result indicates that GRP78 facilitates Cripto activation of the PI3K pathway downstream of ErbB2 and ErbB4 and is consistent with previous data demonstrating that cell surface GRP78 mediates Cripto-dependent Akt/PI3K signaling. Complex formation between Cripto and GRP78 appears to be necessary for this effect since the GRP78 D19-68 mutant deficient in Cripto binding did not promote signaling via ErbB2 and ErbB4 (FIG. 13C). Rather, the GRP78 D19-68 mutant completely blocked Cripto signaling in the presence of transfected ErbB2 and ErbB4 (compare FIGs. 13A and 13C). This result suggests that the GRP78 D19-68 mutant acts in a dominant negative fashion to prevent endogenous GRP78 from facilitating the Cripto response and points to a possible role for this GRP78 mutant as a Cripto antagonist with therapeutic potential. Finally, as predicted, the PI3K inhibitor LY294002 caused basal luciferase levels to increase (∼2-fold) and completely blocked Cripto-induced decreases in luciferase levels (FIG. 13A-C).

### Cell surface GRP78 mediates Cripto signaling

Overall, the data support a model (FIG. 14) in which the cell surface Cripto/GRP78 complex acts as a growth-control node that inhibits tumor suppressor function and activates proliferation/survival pathways. On the one hand, Cripto and GRP78 cooperatively inhibit cytostatic Smad2/3 signaling in response to activin and TGF-β and cause these ligands to adopt pro-proliferative effects. Cripto binding to cell surface GRP78 is also necessary for Cripto-dependent Nodal signaling which has been linked to tumor cell plasticity and tumorigenicity. On the other hand, Cripto binding to cell surface GRP78 is required for Cripto tumor growth factor activity including its ability to cause Src, ERK and Akt phosphorylation as well as to promote proliferation, EMT and migration. This model highlights the dual role of the Cripto/GRP78 complex in promoting the tumor phenotype as well as the potential therapeutic benefit of targeting the interaction between Cripto and cell surface GRP78. This model also illustrates the inventors understanding that Cripto binding to cell surface GRP78 is necessary for subsequent Cripto interactions with either ErbB2/ErbB4 or activin/Nodal/TGF-β/receptor complexes (FIG. 14A). The data indicate that Cripto/GRP78 binding occurs upstream of each of these Cripto signaling "arms" and, therefore, reagents that disrupt Cripto/GRP78 complcx formation will inhibit oncogcnic Cripto cffccts on both Smad2/3 and MAPK/PI3K signaling (FIG. 14B).

All of the compositions and methods disclosed and claimed herein can be made and executed without undue experimentation in light of the present disclosure.

### REFERENCES

U.S. Patent 3,817,837
U.S. Patent 3,850,752
U.S. Patent 3,939,350
U.S. Patent 3,996,345
U.S. Patent 4,196,265
U.S. Patent 4,275,149
U.S. Patent 4,277,437
U.S. Patent 4,366,241
U.S. Patent 4,472,509
U.S. Patent 4,659,774
U.S. Patent 4,664,911
U.S. Patent 4,682,195
U.S. Patent 4,683,202
U.S. Patent 4,792,447
U.S. Patent 4,816,571
U.S. Patent 4,870,287
U.S. Patent 4,938,948
U.S. Patent 4,959,463
U.S. Patent 5,021,236
U.S. Patent 5,045,451
U.S. Patent 5,141,813
U.S. Patent 5,196,066
U.S. Patent 5,206,347
U.S. Patent 5,214,136
U.S. Patent 5,223,618
U.S. Patent 5,264,566
U.S. Patent 5,378,825
U.S. Patent 5,428,148
U.S. Patent 5,446,137
U.S. Patent 5,466,786
U.S. Patent 5,470,967
U.S. Patent 5,539,082
U.S. Patent 5,539,082
U.S. Patent 5,554,744
U.S. Patent 5,574,146
U.S. Patent 5,578,706
U.S. Patent 5,602,240
U.S. Patent 5,602,244
U.S. Patent 5,610,289
U.S. Patent 5,614,617
U.S. Patent 5,623,070
U.S. Patent 5,645,897
U.S. Patent 5,652,099
U.S. Patent 5,670,663
U.S. Patent 5,672,697
U.S. Patent 5,681,947
U.S. Patent 5,686,072
U.S. Patent 5,705,629
U.S. Patent 5,708,154
U.S. Patent 5,714,331
U.S. Patent 5,714,606
U.S. Patent 5,719,262
U.S. Patent 5,736,336
U.S. Patent 5,756,291
U.S. Patent 5,760,395
U.S. Patent 5,763,167
U.S. Patent 5,766,855
U.S. Patent 5,767,072
U.S. Patent 5,773,571
U.S. Patent 5,777,092
U.S. Patent 5,786,461
U.S. Patent 5,792,847
U.S. Patent 5,858,988
U.S. Patent 5,859,221
U.S. Patent 5,872,232
U.S. Patent 5,886,165
U.S. Patent 5,891,625
U.S. Patent 5,908,845
U.S. Patent 6,180,348
U.S. Patent 6,423,493
U.S. Patent 6,506,559
U.S. Patent 6,515,120
U.S. Patent 6,573,099
U.S. Patent 7,329,742
U.S. Patent Serial 117,363
U.S. Patent Appln. 2002/0168707
U.S. Patent Appln. 2003/0051263
U.S. Patent Appln. 2003/0055020
U.S. Patent Appln. 2003/0159161
U.S. Patent Appln. 2004/0064842
U.S. Patent Appln. 2004/0265839

Adewumi et al., Nat. Biotechnol., 25:803-816, 2007.
Adkins et al., J. Clin. Invest., 112:575-87, 2003.
Arap et al., Cancer Cell, 6:275-84, 2004.
Atwell et al., Mol. Immunol., 33(17-18):1301-1312, 1996.
Bernales et al., Annu. Rev. Cell Dev. Biol., 22:487-508, 2006.
Bianco et al., Cancer Res., 63:1192-1197, 2003.
Bianco et al., J. Biol. Chem., 274:8624-9, 1999.
Bianco et al., J. Cell Physiol., 190:74-82, 2002b.
Bianco et al., Mol. Cell Biol., 22:2586-2597, 2002a.
Chen et al., Breast Cancer Res. Treat., 59:81-90, 2000.
Chen et al., Development, 133:319-329, 2006.
Cheng et al., Genes Dev., 17:31-36, 2003.
Cox et al., Comb. Chem. High Throughput Screen, 5(4):289-99, 2002.
Culver et al., Science, 256(5063):1550-1552, 1992.
Davidson et al., Cancer Res., 65:4663-4672, 2005.
De Santis et al., Cell Growth Differ., 8:1257-1266, 1997.
Delpino and Castelli, Biosci. Rep., 22:407-20, 2002.
Delpino et al., Mol. Membr. Biol., 15:21-6, 1998.
Dhillon et al., Oncogene, 26:3279-3290, 2007.
Ding et al., Nature, 395:702-707, 1998.
Dong et al., Cancer Res., 68:498-505, 2008.
Dong et al., Hum. Gene Ther., 15:553-561, 2004.
Drolet et al., Comb. Chem. High Throughput Screen, 2(5):271-278, 1999.
Dutertre and Teillaud, J. Soc. Biol., 200(4):377-386, 2006.
Ebert et al., Cancer Res., 59:4502-4505, 1999.
Egholm et al., Nature, 365(6446):566-568, 1993.
Ellington and Szostak, Nature, 346(6287):818-822, 1990.
Ellington and Szostak, Nature, 355(6363):850-852, 1992.
EP 266,032
Froehler et al., Nucleic Acids Res., 14(13):5399-5407, 1986.
Gazit et al., Cancer Res., 59:3100-6, 1999.
Gonzalez-Gronow et al., Cancer Res., 66:11424-11431, 2006.
Gray et al., Mol. Cell Biol., 26:9268-9278, 2006.
Gray et al., Proc. Natl. Acad. Sci. USA, 100:5193-5198, 2003.
Harrison et al., J. Biol. Chem., 279:28036-44, 2004.
Hendrix et al., Nat. Rev. Cancer, 7:246-255, 2007.
Hudson et al,. J. Immunol. Methods, 231(1-2):177-89, 1999.
Jakobsen et al., Cancer Res., 67:9507-9517, 2007.
Jamora et al., Proc. Natl. Acad. Sci. USA, 93:7690-7694, 1996.
Kannan et al., J. Biol. Chem., 272:3330-3335, 1997.
Kelber et al., J. Biol. Chem., 283:4490-4500, 2008.
Kinoshita et al., Cell, 83:621-630, 1995.
Kortt et al., Biomol. Eng., 18(3):95-108, 2001.
Lee, Cancer Res., 67:3496-3499, 2007.
Lee, Cancer Res., 67:3496-9, 2007.
Lee, Methods, 35:373-81, 2005.
Lee, Trends Biochem. Sci., 26:504-510, 2001.
Li and Lee, Curr. Mol. Med., 6:45-54, 2006.
Liu et al., Mol. Pharm., 4(3):435-47, 2007.
Luo et al., Mol. Cell Biol., 26:5688-5697, 2006.
Mao et al., J. Biol. Chem., 281:8877-8887, 2006.
Minchiotti, Oncogene, 24:5668-5675, 2005.
Mintz et al., Nat. Biotechnol., 21:57-63, 2003.
Misra et al., Cell Signal, 16:929-938, 2004.
Misra et al., J. Biol. Chem., 277:42082-7, 2002.
Misra et al., J. Biol. Chem., 280:26278-86, 2005.
Misra et al., J. Biol. Chem., 281:13694-13707, 2006.
Mitra and Schlaepfer, Curr. Opin. Cell Biol., 18:516-23, 2006.
Miyoshi et al., J. Virol., 72:8150-7, 1998.
Pardali and Moustakas, Biochim. Biophys. Acta, 1775:21-62, 2007.
PCT Appln. PCT/EP/01219
PCT Appln. WO 92/20702
Philippova et al., Mol. Cell Biol., 28(12):4004-17, 2008.
Plückthun et al. Immunotechnology, 3(2):83-105, 1997.
Postovit et al., Proc. Natl. Acad. Sci. USA, 105:4329-4334, 2008.
Reddy et al.., J. Biol. Chem., 278:20915-20924, 2003.
Remington's Pharmaceutical Sciences, 18th Ed., Mack Printing Company, 1289-1329, 1990.
Robinson et al., Br J Cancer. 99(9):1415-25, 2008.
Salomon, Nat. Med., 12:1231, 2006.
Sambrook et al., In: Molecular cloning, Cold Spring Harbor Laboratory Press, Cold Spring Harbor, NY, 2001.
Schier, Annu. Rev. Cell Dev. Biol., 19:589-621, 2003.
Shani et al., Mol. Cell Biol., 28:666-677, 2008.
Shaw and Cantley, Nature, 441:424-430, 2006.
Shen and Schier, Trends Genet., 16:303-309, 2000.
Shen, Development, 134:1023-1034, 2007.
Shen, J. Clin. Invest., 112:500-502, 2003.
Shin et al., J. Biol. Chem., 278:7607-16, 2003.
Shukla et al., Mol. Cancer Res., 6:509-516, 2008.
Siegel and Massague, Nat. Rev. Cancer, 3:807-21, 2003.
Singer et al., Nat. Neurosci., 8:1343-9, 2005.
Strizzi et al., J. Cell Physiol., 201:266-276, 2004.
Strizzi et al., Oncogene, 24:5731-5741, 2005.
Sun et al., Am. J. Pathol., 167:585-597, 2005.
Takahashi et al., Cell, 126(4):663-676, 2006.
Takahashi et al., Cell, 126(4):663-76, 2007.
Todorovska et al., J. Immunol. Methods, 248(1-2):47-66, 2001.
Topczewska et al., Nat. Med., 12:925-932, 2006.
Triantafilou and Triantafilou, Virology, 317:128-35, 2003.
Triantafilou et al., J. Virol., 76:633-43, 2002.
Tuerk and Gold, Science, 249(4968):505-510, 1990
Vaughan, Endocrinol., 132(5):2038-50, 1993.
Watanabe et al., J. Biol. Chem., 282(43):31643-55, 2007.
Wechselberger et al., Oncogene, 24(25):4094-105, 2005..
Wilding et al., Mol. Cell Endocrinol., 62:79-87, 1989.
Xing et al., Cancer Res., 64:4018-4023, 2004.
Xu et al., Dev. Biol., 196:237-247, 1998.
Xu et al., Development, 126:483-494, 1999.
Yeo and Whitman, Mol. Cell, 7:949-57, 2001.
Ylera et al., Biochem. Biophys. Res. Commun., 290(5):1583, 2002.
Yu et al., Science, 318:1917-1920, 2007.
Mintz PJ et al Nat Biotechnol 2003 21(1) 57-63.
Kim Y et al Biochemistry 45(31) 9434-44.
Jakobsen CG et al Cancer Res 2007 67(19) 9507-17.
Gonzalez-Gronow M et al Cancer Res 2006 66(23) 11424-31.

## Claims

1. A selective GRP78/Cripto targeting compound for use in treating a hyperproliferative disease, wherein said use comprises inhibiting Cripto signaling in a cancerous, pre-cancerous, or malignant cell to reduce the cell's proliferation by contacting the cell with an amount of said selective GRP78/Cripto targeting compound,
wherein said targeting compound is effective to inhibit the formation of complexes between Cripto and GRP78, thereby reducing the cell's proliferation,
wherein said targeting compound is an anti-GRP78 antibody that binds an N-20 epitope of the GRP78, wherein said N-20 epitope consists of 20 amino acid residues within the first 50 residues downstream from the GRP78 signal peptide.

2. Use of a selective GRP78/Cripto targeting compound for the preparation of a pharmaceutical composition for use in treating a hyperproliferative disease, wherein said use comprises inhibiting Cripto signaling in a cancerous, pre-cancerous, or malignant cell to reduce the cell's proliferation by contacting the cell with an amount of said selective GRP78/Cripto targeting compound,
wherein said targeting compound is effective to inhibit the formation of complexes between Cripto and GRP78, thereby reducing the cell's proliferation,
wherein said targeting compound is an anti-GRP78 antibody that binds an N-20 epitope of the GRP78, wherein said N-20 epitope consists of 20 amino acid residues within the first 50 residues downstream from the GRP78 signal peptide.

3. The selective GRP78/Cripto targeting compound for the use according to claim 1 or the use according to claim 2, wherein the antibody is a human or humanized monoclonal antibody, is a bispecific antibody, or is conjugated to a reporter molecule.

4. The selective GRP78/Cripto targeting compound for the use according to claim 3 or the use according to claim 3, wherein the reporter molecule is a radioligand or a fluorescent label.

5. The selective GRP78/Cripto targeting compound for the use according to any one of claims 1, 3 and 4 or the use according to any one of claims 2 to 4, wherein antibody is an anti-GRP78 scFv, F(ab) or F(ab)2.

6. The selective GRP78/Cripto targeting compound for the use according to claim 1 or the use according to claim 2, wherein the hyperproliferative disease is cancer.

7. The selective GRP78/Cripto targeting compound for the use according to any one of claims 1 and 2 to 6 or the use according to any one of claims 2 to 6, wherein the cancer is selected from the group consisting of breast cancer, colon cancer, stomach cancer, pancreatic cancer, lung cancer, ovarian cancer, endometrial cancer, testicular cancer, bladder cancer, prostate cancer, head and neck cancer, cervical cancer, gall bladder cancer, or adrenocortical carcinoma.

8. A humanized monoclonal anti-GRP78 antibody, wherein the antibody binds an N-20 epitope in GRP78, wherein said N-20 epitope consists of 20 amino acid residues within the first 50 residues downstream from the GRP78 signal peptide and wherein said binding inhibits the formation of Cripto/GRP78 complexes.

9. The antibody of claim 8, wherein the antibody is comprised in a pharmaceutical composition.

## Patentansprüche

1. Selektive GRP78/Cripto-Zielverbindung zur Verwendung bei der Behandlung einer hyperproliferativen Erkrankung, wobei die Verwendung das Hemmen der Cripto-Signalgebung in einer kanzerösen, präkanzerösen oder malignen Zelle umfasst, um die Proliferation der Zelle zu verringern, durch Inkontaktbringen der Zelle mit einer Menge der selektiven GRP78/Cripto-Zielverbindung,
wobei die Zielverbindung wirksam ist, um die Bildung von Komplexen zwischen Cripto und GRP78 zu hemmen und dabei die Proliferation der Zelle zu verringern,
wobei die Zielverbindung ein anti-GRP78-Antikörper ist, der ein N-20-Epitop von GRP78 bindet, wobei das N-20-Epitop aus 20 Aminosäureresten innerhalb der ersten 50 Reste stromabwärts des GRP78 Signalpeptids besteht.

2. Verwendung einer selektiven GRP78/Cripto-Zielverbindung zur Herstellung eines Arzneimittels zur Verwendung bei der Behandlung einer hyperproliferativen Erkrankung, wobei die Verwendung das Hemmen der Cripto-Signalgebung in einer kanzerösen, präkanzerösen oder malignen Zelle umfasst, um die Proliferation der Zelle zu verringern, durch Inkontaktbringen der Zelle mit einer Menge der selektiven GRP78/Cripto-Zielverbindung,
wobei die Zielverbindung wirksam ist, um die Bildung von Komplexen zwischen Cripto und GRP78 zu hemmen und dabei die Proliferation der Zelle zu verringern,
wobei die Zielverbindung ein anti-GRP78-Antikörper ist, der ein N-20-Epitop von GRP78 bindet, wobei das N-20-Epitop aus 20 Aminosäureresten innerhalb der ersten 50 Reste stromabwärts des GRP78 Signalpeptids besteht.

3. Selektive GRP78/Cripto-Zielverbindung zur Verwendung nach Anspruch 1 oder Verwendung nach Anspruch 2, wobei der Antikörper ein humaner oder humanisierter monoclonaler Antikörper ist, ein bispezifischer Antikörper ist oder an ein Reportermolekül gebunden ist.

4. Selektive GRP78/Cripto-Zielverbindung zur Verwendung nach Anspruch 3 oder Verwendung nach Anspruch 3, wobei das Reportermolekül ein Radioligand oder ein Fluoreszenzmarker ist.

5. Selektive GRP78/Cripto-Zielverbindung zur Verwendung nach einem der Ansprüche 1, 3 und 4 oder Verwendung nach einem der Ansprüche 2 bis 4, wobei der Antikörper ein anti-GRP78-scFv, -F(ab) oder -F(ab)2 ist.

6. Selektive GRP78/Cripto-Zielverbindung zur Verwendung nach Anspruch 1 oder Verwendung nach Anspruch 2, wobei die hyperproliferative Erkrankung Krebs ist.

7. Selektive GRP78/Cripto-Zielverbindung zur Verwendung nach einem der Ansprüche 1 und 2 bis 6 oder Verwendung nach einem der Ansprüche 2 bis 6, wobei der Krebs ausgewählt ist aus der Gruppe bestehend aus Brustkrebs, Darmkrebs, Magenkrebs, Pankreaskrebs, Lungenkrebs, Eierstockkrebs, Endometriumkrebs, Hodenkrebs, Blasenkrebs, Prostatakrebs, Kopf- und Halskrebs, Gebärmutterhalskrebs, Gallenblasenkrebs oder adrenokortikalem Karzinom.

8. Humanisierter monoclonaler anti-GRP78-Antikörper, wobei der Antikörper ein N-20-Eptiop im GRP78 bindet, wobei das N-20-Epitop aus 20 Aminosäureresten innerhalb der ersten 50 Reste stromabwärts des GRP78 Signalpeptids besteht und wobei die Bindung die Bildung von Cripto/GRP78-Komplexen hemmt.

9. Antikörper nach Anspruch 8, wobei der Antikörper in einem Arzneimittel umfasst ist.

## Revendications

1. Composé de ciblage sélectif de GRP78/Cripto destiné à une utilisation dans le traitement d'une maladie hyperproliférative, où ladite utilisation comprend l'inhibition de la signalisation de Cripto dans une cellule cancéreuse, précancéreuse, ou maligne afin de réduire la prolifération de la cellule par la mise en contact de la cellule avec une quantité dudit composé de ciblage sélectif de GRP78/Cripto,
où ledit composé de ciblage est efficace pour inhiber la formation de complexes entre Cripto et la GRP78, en réduisant ainsi la prolifération de la cellule,
où ledit composé de ciblage est un anticorps anti-GRP78 qui se lie à un épitope N-20 de la GRP78, où ledit épitope N-20 consiste en 20 résidus d'acides aminés au sein des 50 premiers résidus en aval du peptide signal de la GRP78.

2. Utilisation d'un composé de ciblage sélectif de GRP78/Cripto pour la préparation d'une composition pharmaceutique destiné à une utilisation dans le traitement d'une maladie hyperproliférative, où ladite utilisation comprend l'inhibition de la signalisation de Cripto dans une cellule cancéreuse, précancéreuse, ou maligne afin de réduire la prolifération de la cellule par la mise en contact de la cellule avec une quantité dudit composé de ciblage sélectif de GRP78/Cripto,
où ledit composé de ciblage est efficace pour inhiber la formation de complexes entre Cripto et la GRP78, en réduisant ainsi la prolifération de la cellule,
où ledit composé de ciblage est un anticorps anti-GRP78 qui se lie à un épitope N-20 de la GRP78, où ledit épitope N-20 consiste en 20 résidus d'acides aminés au sein des 50 premiers résidus en aval du peptide signal de la GRP78.

3. Composé de ciblage sélectif de GRP78/Cripto destiné à l'utilisation selon la revendication 1 ou l'utilisation selon la revendication 2, où l'anticorps est un anticorps monoclonal humain ou humanisé, est un anticorps bispécifique, ou est conjugué à une molécule rapporteur.

4. Composé de ciblage sélectif de GRP78/Cripto destiné à l'utilisation selon la revendication 3 ou l'utilisation selon la revendication 3, où la molécule rapporteur est un radioligand ou un marqueur fluorescent.

5. Composé de ciblage sélectif de GRP78/Cripto destiné à l'utilisation selon l'une quelconque des revendications 1, 3 et 4 ou l'utilisation selon l'une quelconque des revendications 2 à 4, où l'anticorps est un scFv, F(ab) ou F(ab)2 anti-GRP78.

6. Composé de ciblage sélectif de GRP78/Cripto destiné à l'utilisation selon la revendication 1 ou l'utilisation selon la revendication 2, où la maladie hyperproliférative est le cancer.

7. Composé de ciblage sélectif de GRP78/Cripto destiné à l'utilisation selon l'une quelconque des revendications 1 et 2 à 6 ou l'utilisation selon l'une quelconque des revendications 2 à 6 où le cancer est sélectionné dans le groupe consistant en le cancer du sein, le cancer du côlon, le cancer de l'estomac, le cancer du pancréas, le cancer du poumon, le cancer de l'ovaire, le cancer de l'endomètre, le cancer du testicule, le cancer de la vessie, le cancer de la prostate, le cancer de la tête et du cou, le cancer du col de l'utérus, le cancer de la vésicule biliaire, ou le carcinome corticosurrénalien.

8. Anticorps monoclonal humanisé anti-GRP78, où l'anticorps se lie à un épitope N-20 dans la GRP78, où ledit épitope N-20 consiste en 20 résidus d'acides aminés au sein des 50 premiers résidus en aval du peptide signal de la GRP78 et où ladite liaison inhibe la formation de complexes Cripto/GRP78.

9. Anticorps selon la revendication 8, où l'anticorps est compris dans une composition pharmaceutique.
